(19) Europäisches Patentamt
European Patent Office
Office européen des brevets

(11) Veröffentlichungsnummer: **0 604 800 A1**

(12) **EUROPÄISCHE PATENTANMELDUNG**

(21) Anmeldenummer: **93119786.7**

(22) Anmeldetag: **08.12.93**

(51) Int. Cl.5: **C07D 211/34**, C07D 295/14, C07D 211/70, C07D 207/08, C07D 401/04, A61K 31/445, A61K 31/40

(30) Priorität: **10.12.92 DE 4241632**

(43) Veröffentlichungstag der Anmeldung: **06.07.94 Patentblatt 94/27**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach(DE)**

(72) Erfinder: **Himmelsbach, Frank, Dr. Dipl.-Chem.**
**Ahornweg 12**
**D-88441 Mittelbiberach(DE)**
Erfinder: **Linz, Günter, Dr. Dipl.-Chem.**
**Erlenweg 8**
**D-88441 Mittelbiberach(DE)**
Erfinder: **Austel, Volkhard, Prof. Dr. Dipl.-Chem.**
**Kapellenweg 7**
**D-88400 Biberach(DE)**
Erfinder: **Pieper, Helmut, Dr. Dipl.-Chem.**
**Kapellenweg 5**
**D-88400 Biberach(DE)**
Erfinder: **Müller, Thomas, Dipl.-Chem.**
**Alter Postplatz 17**
**D-88400 Biberach(DE)**
Erfinder: **Weisenberger, Johannes, Dr. Dipl.-Chem.**
**Haydnweg 5**
**D-88400 Biberach(DE)**
Erfinder: **Guth, Brian, Dr. Phys.**
**Am Schlegelberg 24**
**D-88447 Warthausen(DE)**

(54) **4- 4- 4-(Carboxyalkyl)-phenyl aminocarbonyl -phenyl -Piperidine als Aggregationshemmer.**

(57) Die Erfindung betrifft Carbonsäurederivate der allgemeinen Formel

$$A - B - C - D - E - COOR_a \quad ,(I)$$

in der
A bis E und $R_a$ wie im Anspruch 1 definiert sind, insbesondere Verbindungen der Formel (II),

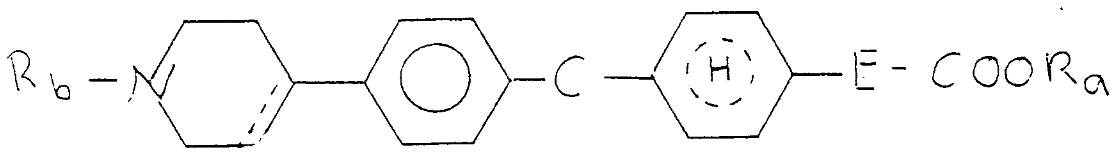

(II)

deren Tautomere, deren Stereoisomere einschließlich deren Gemische und deren Salze, insbesondere deren physiologisch verträgliche Salze mit anorganischen oder organischen Säuren oder Basen, welche wertvolle

EP 0 604 800 A1

pharmakologische Eigenschaften aufweisen, vorzugsweise aggregationshemmende Wirkungen, diese Verbindungen enthaltende Arzneimittel und Verfahren zu ihrer Herstellung.

In den europäischen Offenlegungsschriften 0,478,328 und 0,478,363 werden Phenylalanylderivate als Fibrinogen-Rezeptor-Antagonisten beschrieben.

Es wurde nun gefunden, daß die neuen Carbonsäurederivate der allgemeinen Formel

$$A - B - C - D - E - COOR_a \quad ,(I)$$

ebenfalls wertvolle pharmakologische Eigenschaften aufweisen, insbesondere aggregationshemmende Wirkungen, wobei sich die neuen Verbindungen von den in den obigen Offenlegungsschriften beschriebenen Verbindungen insbesondere durch den Rest B unterscheiden.

Gegenstand der vorliegenden Erfindung sind somit die neuen Carbonsäurederivate der obigen allgemeinen Formel I, deren Tautomere, deren Stereoisomere einschließlich deren Gemische und deren Salze, insbesondere deren physiologisch verträgliche Salze mit anorganischen oder organischen Säuren oder Basen, diese Verbindungen enthaltende Arzneimittel und deren Verwendung sowie Verfahren zu ihrer Herstellung.

In der obigen allgemeinen Formel I bedeutet

$R_a$ ein Wasserstoffatom, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkylgruppe mit 5 bis 7 Kohlenstoffatomen oder eine $R_1$-CO-O-($R_2$CH)-Gruppe, in der

$R_1$ eine Alkylgruppe mit 1 bis 5 Kohlenstoffatomen, eine Cycloalkylgruppe mit 5 bis 7 Kohlenstoffatomen, eine Phenylalkylgruppe mit 1 bis 3 Kohlenstoffatomen im Alkylteil, eine Alkoxygruppe mit 1 bis 5 Kohlenstoffatomen, eine Cycloalkoxygruppe mit 5 bis 7 Kohlenstoffatomen oder eine Phenylgruppe und

$R_2$ ein Wasserstoffatom, eine Alkylgruppe mit 1 bis 4 Kohlenstoffatomen, eine Cycloalkylgruppe mit 5 bis 7 Kohlenstoffatomen oder eine Phenylgruppe darstellen,

A eine über ein Kohlenstoffatom des Restes A mit dem Rest B verknüpfte Aminoalkylgruppe mit 1 bis 5 Kohlenstoffatomen oder eine 5-, 6- oder 7-gliedrige Azacycloalkylgruppe, wobei die Verknüpfung der Azacycloalkylgruppe mit dem Rest B über ein Kohlenstoffatom der Azacycloalkylgruppe erfolgt sowie ein Wasserstoffatom der Aminogruppe in den vorstehend erwähnten Aminoalkylgruppen und das Wasserstoffatom am Stickstoffatom in den vorstehend erwähnten Azacycloalkylgruppen durch den Rest $R_b$ ersetzt sein kann und zusätzlich die Kohlenstoffatome der Azacycloalkylgruppe durch ein bis drei Alkylgruppen mit jeweils 1 bis 3 Kohlenstoffatomen, durch eine Aminocarbonyl-, Cyano-, $R_3$O- oder $R_3$O-CO-Gruppe substituiert sein können, wobei

$R_b$ eine Alkylgruppe mit 1 bis 3 Kohlenstoffatomen, eine Phenylalkylgruppe mit 1 bis 3 Kohlenstoffatomen im Alkylteil, eine Alkanoylgruppe mit 1 bis 3 Kohlenstoffatomen im Alkylteil, eine Trifluoracetylgruppe, eine Alkoxycarbonylgruppe mit insgesamt 2 bis 6 Kohlenstoffatomen, eine Phenylalkoxycarbonylgruppe mit 1 bis 3 Kohlenstoffatomen im Alkoxyteil, eine Alkenyloxycarbonylgruppe mit insgesamt 4 bis 6 Kohlenstoffatomen oder eine $R_1$-CO-O-($R_2$CH)-O-CO-Gruppe, in der $R_1$ und $R_2$ wie vorstehend erwähnt definiert sind, und

$R_3$ ein Wasserstoffatom, eine Alkyl- oder Phenylalkylgruppe mit jeweils 1 bis 3 Kohlenstoffatomen im Alkylteil darstellen,

sowie in einer so gebildeten 6- oder 7-gliedrigen Azacycloalkylgruppe eine >CH- Einheit in 4-Stellung, bezogen auf das Ringstickstoffatom, durch ein Stickstoffatom oder in einer so gebildeten 5- bis 7-gliedrigen Azacycloalkylgruppe eine $-CH_2$-CH< Einheit durch eine -CH=C< Einheit und in einem so gebildeten Piperazinyl- oder Homopiperazinylring eine Methylengruppe, die benachbart zu dem Stickstoffatom in 4-Stellung steht, durch ein Carbonylgruppe ersetzt sein kann, eine Chinuclidinyl- oder Pyridylgruppe,

B eine gegebenenfalls durch ein Fluor-, Chlor- oder Bromatom, durch eine Trifluormethylgruppe, durch eine Alkyl-, Alkoxy-, Alkylsulfenyl-, Alkylsulfinyl- oder Alkylsulfonylgruppe mit jeweils 1 bis 3 Kohlenstoffatomen im Alkyl- und Alkoxyteil mono- oder disubstituierte Phenylengruppe, wobei die Substituenten gleich oder verschieden sein und in der zusätzlich 1 oder 2 unsubstituierte Methingruppen jeweils durch ein N-Atom ersetzt sein können, oder eine Piperidinylengruppe,

C eine Carbonyl-, $-CH_2CH_2$-, -CH=CH-, $-CH_2$-, $-CH_2O$-, $-OCH_2$-, $-CONR_4$-, $-CONR_4-CH_2$-, $-NR_4CO$-, $-NR_4CO-NR_4$-, $-CH_2NR_4$-, $-NR_4CH_2$-, $-SO_2NR_4$-, $-SO_2NR_4-CH_2$- oder $-NR_4SO_2$-Gruppe, in denen

$R_4$ ein Wasserstoffatom, eine Alkyl- oder Phenylalkylgruppe mit jeweils 1 bis 3 Kohlenstoffatomen im Alkylteil oder auch, wenn C eine über die Carbonylgruppe an den Rest B gebundene $-CONR_4$-Gruppe darstellt, $R_4$ eine Methylen- oder 1,2-Ethylengruppe darstellt, die jeweils an das zur Anknüpfungsstelle des $-CONR_4$-Restes orthoständige Kohlenstoffatom des Restes B gebunden sind,

D eine gegebenenfalls durch ein Fluor-, Chlor- oder Bromatom, durch eine Trifluormethylgruppe, durch eine Alkyl-, Alkoxy-, Alkylsulfenyl, Alkylsulfinyl- oder Alkylsulfonylgruppe mit jeweils 1 bis 3 Kohlenstoffatomen im Alkyl- und Alkoxyteil mono- oder disubstituierte Phenylengruppe, wobei die Substituenten gleich oder

verschieden sein und in der zusätzlich 1 oder 2 unsubstituierte Methingruppen jeweils durch ein N-Atom ersetzt sein können, eine Cycloalkylengruppe mit 5 bis 7 Kohlenstoffatomen, in der eine oder zwei >CH-Einheiten, durch ein N-Atom ersetzt sein können, wobei zusätzlich in einem so gebildeten Aza- oder Diazacyclohexylenring eine zu einem Stickstoffatom benachbarte Methylengruppe durch eine Carbonylgruppe ersetzt sein kann, und die Bindung an die Reste C und E auch über ein Stickstoffatom des Aza- oder Diazacyclohexylenringes erfolgen kann, und

E eine Bindung, eine Alkylenoxygruppe mit 1 bis 3 Kohlenstoffatomen im Alkylenteil, wobei das Sauerstoffatom an den Rest D gebunden ist, eine geradkettige Alkylengruppe mit 1 bis 5 Kohlenstoffatomen, die durch 1 oder 2 Alkylgruppen, durch eine Hydroxy-, Alkoxy-, $R_5$NH-, $R_5$N(Alkyl)- oder $R_5$N(Phenylalkyl)-Gruppe substituiert sein kann, oder eine Alkenylengruppe mit 2 bis 5 Kohlenstoffatomen, die durch ein oder zwei Alkylgruppen substituiert sein kann, wobei der Alkylteil der Phenylalkylgruppe 1 bis 3 Kohlenstoffatome und die übrigen Alkyl-, Alkylen- und Alkoxyteile jeweils 1 bis 8 Kohlenstoffatome enthalten können, wobei

$R_5$ ein Wasserstoffatom, eine Alkylgruppe mit 1 bis 8 Kohlenstoffatomen, eine Alkoxycarbonylgruppe mit insgesamt 2 bis 5 Kohlenstoffatomen, eine Phenylalkoxycarbonylgruppe oder eine durch eine Alkylgruppe mit 1 bis 8 Kohlenstoffatomen, durch eine Cycloalkyl-, Phenylalkyl- oder Phenylgruppe substituierte Carbonyl- oder Sulfonylgruppe darstellt, in denen der Alkyl- und Alkoxyteil in einer vorstehend erwähnten Phenylalkyl- und Phenylalkoxygruppe jeweils 1 bis 3 Kohlenstoffatome und der Cycloalkylteil 3 bis 7 Kohlenstoffatome enthalten kann, wobei die bei der Definition des Restes $R_5$ vorstehend erwähnten Phenylkerne jeweils zusätzlich durch ein Fluor-, Chlor- oder Bromatom, durch eine Trifluormethylgruppe, durch eine Alkyl-, Alkoxy-, Alkylsulfenyl-, Alkylsulfinyl- oder Alkylsulfonylgruppe mit jeweils 1 bis 3 Kohlenstoffatomen im Alkyl- und Alkoxyteil mono- oder disubstituiert und die Substituenten gleich oder verschieden sein können,

wobei, wenn

(i) die A-B-Gruppe eine in 4-Stellung durch eine $R_b$-NH-$CH_2$-Gruppe substituierte Phenylgruppe bedeutet, in der $R_b$ eine Benzyloxycarbonylgruppe darstellt, die $R_a$OOC-E-D-C-Gruppe keine 3-Carboxy-phenylaminocarbonylgruppe darstellen kann (siehe EP-A-0,372,486 und J. Med. Chem. 35, 4393-4407 (1992)),

oder, wenn

(ii) die A-B-Gruppe eine in 4-Stellung durch eine $R_b$-NH-$CH_2$-Gruppe substituierte Phenylgruppe bedeutet, in der $R_b$ ein Wasserstoffatom oder eine Acetylgruppe darstellt, die $R_a$OOC-E-D-C-Gruppe keine in 4-Stellung durch eine Carboxymethyl-, Methoxycarbonylmethyl-, 2-Carboxy-ethyl-, 2-Methoxycarbonyl-ethyl- oder 2-Ethoxycarbonylethylgruppe substituierte Phenylcarbonylgruppe darstellen kann (siehe EP-A-0,044,541, JP-A-5813553, JP-A-5939866, JP-A-5846051 und Chem. Pharm. Bull. 33, 5059-5061 (1985)),

oder, wenn

(iii) die A-B-Gruppe eine in 4-Stellung durch eine $NH_2$-$CH_2$-$CH_2$-Gruppe substituierte Phenylgruppe bedeutet, die $R_a$OOC-E-D-C-Gruppe keine 4-Ethoxycarbonyl-phenylcarbonylgruppe darstellen kann (siehe DE-A-3,718,638),

oder, wenn

(iv) die A-B-Gruppe eine 4-Aminomethyl-phenyl-, 3-Aminomethyl-phenyl-,4-Aminomethyl-3-methoxy-phenyl- oder 3-Aminomethyl-4-methoxyphenylgruppe bedeutet, die $R_a$OOC-E-D-C-Gruppe keine 4-Ethoxycarbonylmethoxy-2,3-dichlor-phenylcarbonylgruppe darstellt (siehe J. Med. Chem. 27, 1579-1587 (1984) und Diuretics: Chem. Pharmacol., Clin. Appl., Proc. Int. Conf. Diuretics, 1st, 21-29 (1984),

oder, wenn

(v) die A-B-Gruppe eine in 4-Stellung durch eine $NH_2$-$CH_2$- oder $(CH_3)_3$CO-CO-NH-$CH_2$-Gruppe substituierte Phenylgruppe darstellt, die $R_a$OOC-E-D-C-Gruppe keine 4-Carboxyphenylmethoxygruppe darstellen kann (siehe Int. J. Pept. Protein Res. 18, 451-458 (1981)),

oder, wenn

(vi) die A-B-Gruppe eine in 4-Stellung durch eine $NH_2$-$CH_2$-Gruppe substituierte Phenylgruppe darstellt, die $R_a$OOC-E-D-C-Gruppe keine 4-Carboxy-phenylaminosulfonylgruppe darstellen kann (siehe Chem. Pharm. Bull (Tokio) 7, 734-739 (1959) und J. Chem. Phys. 32, 691-697 (1960)),

oder, wenn

(vii) die AB-Gruppe eine 4-(2-Pyridyl)-phenyl- oder 4-(3-Pyridyl)-phenylgruppe darstellt, die $R_a$OOC-E-D-C-Gruppe keine 4-Carboxy-phenylcarbonylamino-, 4-Benzyloxycarbonyl-phenylcarbonylamino- oder 2-(4-Carboxy-phenyl)-ethylgruppe darstellen kann (siehe J. Med. Chem. 11, 295 (1968) und US-A-2,837,522),

oder, wenn

(viii) die A-B-Gruppe eine 3-(4-Pyridyl)-phenylgruppe darstellt, die $R_a$OOC-E-D-C-Gruppe keine 2-(Carboxymethyl)-phenylcarbonylaminogruppe darstellen kann (siehe Farmaco 44, 721-729 (1989)).

Bevorzugte Verbindungen der obigen allgemeinen Formel I sind jedoch diejenigen, in denen

$R_a$ ein Wasserstoffatom, eine Alkylgruppe mit 1 bis 5 Kohlenstoffatomen, eine Phenylalkylgruppe mit 1 bis 3 Kohlenstoffatomen im Alkylteil oder eine Cycloalkylgruppe mit 5 bis 7 Kohlenstoffatomen,

A eine über ein Kohlenstoffatom des Restes A mit dem Rest B verknüpfte Aminoalkylgruppe mit 2 bis 5 Kohlenstoffatomen oder eine Piperidinylgruppe, wobei die Verknüpfung der Piperidinylgruppe mit dem Rest B über ein Kohlenstoffatom der Piperidinylgruppe erfolgt sowie ein Wasserstoffatom der Aminogruppe in den vorstehend erwähnten Aminoalkylgruppen und das Wasserstoffatom am Stickstoffatom in den vorstehend erwähnten Piperidinylgruppen durch den Rest $R_b$ ersetzt sein kann und zusätzlich die Kohlenstoffatome der Piperidinylgruppe durch eine Methyl-, Cyano-, Carboxy-, Methoxycarbonyl- oder Aminocarbonylgruppe substituiert sein können, wobei

$R_b$ eine Alkylgruppe mit 1 bis 3 Kohlenstoffatomen, eine Benzylgruppe, eine Alkoxycarbonylgruppe mit insgesamt 2 bis 6 Kohlenstoffatomen, eine Benzyloxycarbonylgruppe oder eine $CH_3$-CO-O-($CH_2$)-O-CO-Gruppe darstellt,

sowie in einer so gebildeten Piperidinylgruppe eine >CH- Einheit in 4-Stellung durch ein Stickstoffatom oder in einer so gebildeten Piperidinylgruppe eine -$CH_2$-CH< Einheit durch eine -CH=C< Einheit ersetzt sein kann, eine Chinuclidinyl- oder Pyridylgruppe,

B eine gegebenenfalls durch ein Fluor-, Chlor- oder Bromatom, durch eine Alkyl-, Alkoxy-, Alkylsulfenyl-, Alkylsulfinyl- oder Alkylsulfonylgruppe mit jeweils 1 oder 2 Kohlenstoffatomen im Alkyl- und Alkoxyteil substituierte Phenylengruppe, eine Pyridinylen- oder Piperidinylengruppe,

C eine -CO-, -$CH_2CH_2$-, -CH=CH-, -$CH_2$-, -$CH_2$O-, -O$CH_2$-, -CON$R_4$-, -N$R_4$CO-, -N$R_4$CO-N$R_4$-, -$CH_2$N$R_4$-, -N$R_4$$CH_2$-, -$SO_2$N$R_4$- oder -N$R_4$$SO_2$-Gruppe, in denen

$R_4$ ein Wasserstoffatom, eine Alkyl- oder Phenylalkylgruppe mit jeweils 1 oder 2 Kohlenstoffatomen im Alkylteil oder auch, wenn C eine über die Carbonylgruppe an den Rest B gebundene -CON$R_4$-Gruppe darstellt, $R_4$ eine Methylen- oder 1,2-Ethylengruppe darstellt, die jeweils an das zur Anknüpfungsstelle des -CON$R_4$-Restes orthoständige Kohlenstoffatom des Restes B gebunden sind,

D eine gegebenenfalls durch ein Chlor- oder Bromatom, durch eine Alkyl- oder Alkoxygruppe mit 1 oder 2 Kohlenstoffatomen substituierte Phenylengruppe oder eine Cyclohexylengruppe, wobei in der Cyclohexylengruppe eine oder zwei >CH- Einheiten, durch ein N-Atom ersetzt sein können, wobei zusätzlich in einem so gebildeten Piperidinylen- oder Piperazinylenring eine zu einem Stickstoffatom benachbarte Methylengruppe durch eine Carbonylgruppe ersetzt sein kann, und die Bindung an die Reste C und E auch über ein Stickstoffatom des Piperidinylen- oder Piperazinylenringes erfolgen kann, und

E eine Bindung, eine Methylenoxygruppe, wobei das Sauerstoffatom an den Rest D gebunden ist, eine 1,2-Ethenylengruppe oder eine geradkettige Alkylengruppe mit I bis 5 Kohlenstoffatomen, die durch eine Alkylgruppe mit 1 bis 7 Kohlenstoffatomen oder durch eine $R_5$NH-Gruppe substituiert sein kann, wobei

$R_5$ ein Wasserstoffatom oder eine durch eine Alkylgruppe mit 1 bis 7 Kohlenstoffatomen oder durch eine Benzylgruppe substituierte Carbonylgruppe oder eine durch eine Alkylgruppe mit 1 bis 5 Kohlenstoffatomen oder eine Benzylgruppe substituierte Sulfonylgruppe darstellt,

bedeuten, wobei, wenn

(iii) die A-B-Gruppe eine in 4-Stellung durch eine $NH_2$-$CH_2$-$CH_2$-Gruppe substituierte Phenylgruppe bedeutet, die $R_a$OOC-E-D-C-Gruppe keine 4-Ethoxycarbonyl-phenylcarbonylgruppe darstellen kann,

oder, wenn

(vii) die A-B-Gruppe eine 4-(2-Pyridyl)-phenyl- oder 4-(3-Pyridyl)-phenylgruppe darstellt, die $R_a$OOC-E-D-C-Gruppe keine 4-Carboxy-phenylcarbonylamino-, 4-Benzyloxycarbonyl-phenylcarbonylamino- oder 2-(4-Carboxy-phenyl)-ethylgruppe darstellen kann, oder, wenn

(viii) die A-B-Gruppe eine 3-(4-Pyridyl)-phenylgruppe darstellt, die $R_a$OOC-E-D-C-Gruppe keine 2-(Carboxymethyl)-phenylcarbonylaminogruppe darstellen kann,

deren Tautomere, deren Stereoisomere einschließlich deren Gemische und deren Salze.

Besonders bevorzugte Verbindungen der obigen allgemeinen Formel I sind jedoch diejenigen, in denen

$R_a$ ein Wasserstoffatom, eine Alkylgruppe mit 1 bis 4 Kohlenstoffatomen, eine 2-Phenylethyl- oder Cyclohexylgruppe,

A eine über ein Kohlenstoffatom des Restes A mit dem Rest B verknüpfte Aminoalkylgruppe mit 3 bis 5 Kohlenstoffatomen oder eine Piperidinylgruppe, wobei die Verknüpfung der Piperidinylgruppe mit dem Rest B über ein Kohlenstoffatom der Piperidinylgruppe erfolgt sowie ein Wasserstoffatom der Aminogruppe in den vorstehend erwähnten Aminoalkylgruppen und das Wasserstoffatom am Stickstoffatom in den vorstehend erwähnten Piperidinylgruppen durch den Rest $R_b$ ersetzt sein kann und zusätzlich die Kohlenstoffatome der Piperidinylgruppe durch eine Methyl-, Cyano-, Carboxy-, Methoxycarbonyl- oder Aminocarbonylgruppe substituiert sein können, wobei

$R_b$ eine Alkylgruppe mit 1 bis 3 Kohlenstoffatomen, eine Benzylgruppe, eine Alkoxycarbonylgruppe mit insgesamt 2 bis 5 Kohlenstoffatomen oder eine $CH_3$-CO-O-($CH_2$)-O-CO-Gruppe darstellt,

sowie in einer so gebildeten Piperidinylgruppe eine >CH- Einheit in 4-Stellung durch ein Stickstoffatom oder in einer so gebildeten Piperidinylgruppe eine -$CH_2$-CH< Einheit durch eine -CH=C< Einheit ersetzt sein kann, eine Chinuclidinyl- oder 4-Pyridylgruppe,

B eine gegebenenfalls durch eine Methyl- oder Methoxygruppe substituierte Phenylengruppe, eine 2,5-Pyridinylen- oder 1,4-Piperidinylengruppe,

C eine -CO-, -$CH_2CH_2$-, -CH=CH-, -$CH_2$-, -$CH_2$O-, -O$CH_2$-, -CON$R_4$-, -N$R_4$CO-, -N$R_4$CO-N$R_4$- oder -$CH_2$N$R_4$-Gruppe oder eine -$SO_2$N$R_4$-Gruppe, wobei die $SO_2$-Gruppe mit dem Rest B verknüpft ist und in denen

$R_4$ ein Wasserstoffatom, eine Methyl- oder 2-Phenylethylgruppe oder auch, wenn C eine über die Carbonylgruppe an den Rest B gebundene -CON$R_4$-Gruppe darstellt, $R_4$ eine Methylen- oder 1,2-Ethylengruppe darstellt, die jeweils an das zur Anknüpfungsstelle des -CON$R_4$-Restes ortho-ständige Kohlenstoffatom des Restes B gebunden sind,

D eine Phenylen- oder Cyclohexylengruppe, wobei in der Cyclohexylengruppe eine oder zwei >CH-Einheiten, durch ein N-Atom ersetzt sein können, wobei die Bindung an die Reste C und E auch über ein Stickstoffatom des Piperidinylen- oder Piperazinylenringes erfolgen kann, und

E eine Bindung, eine Methylenoxygruppe, wobei das Sauerstoff an den Rest D gebunden ist, eine geradkettige Alkylengruppe mit 1 bis 5 Kohlenstoffatomen, die durch $R_5$NH-Gruppe substituiert sein kann, wobei

$R_5$ ein Wasserstoffatom, eine durch eine Alkylgruppe mit 1 bis 5 Kohlenstoffatomen substituierte Carbonylgruppe oder eine durch eine Alkylgruppe mit 1 bis 4 Kohlenstoffatomen oder eine Benzylgruppe substituierte Sulfonylgruppe darstellt,

bedeuten, deren Tautomere, deren Stereoisomere einschließlich deren Gemische und deren Salze.

Ganz besonders bevorzugte Verbindungen der obigen allgemeinen Formel I sind jedoch diejenigen, in denen

$R_a$ ein Wasserstoffatom, eine Alkylgruppe mit 1 bis 4 Kohlenstoffatomen oder eine Cyclohexylgruppe,

A eine über die 4-Stellung mit dem Rest B verknüpfte Piperidinyl- oder 3,4-Dehydro-piperidinylgruppe, wobei jeweils das Wasserstoffatom am Stickstoffatom durch den Rest $R_b$ ersetzt sein kann und

$R_b$ eine Alkoxycarbonylgruppe mit insgesamt 2 bis 5 Kohlenstoffatomen darstellt,

eine gegebenenfalls in 1-Stellung durch den Rest $R_b$ substituierte 4-Piperazinylgruppe, wobei $R_b$ wie vorstehend erwähnt definiert ist, oder eine Chinuclidinylgruppe,

B eine Phenylengruppe,

C eine -CON$R_4$-Gruppe, wobei

$R_4$ ein Wasserstoffatom oder eine Methylgruppe oder auch, wenn C eine über die Carbonylgruppe an den Rest B gebundene -CON$R_4$-Gruppe darstellt, $R_4$ eine Methylen- oder 1,2-Ethylengruppe darstellt, die jeweils an das zur Anknüpfungsstelle des -CON$R_4$-Restes ortho-ständige Kohlenstoffatom des Restes B gebunden sind,

D eine 1,4-Phenylen- oder 1,4-Cyclohexylengruppe, und

E eine Bindung, eine 1,2-Ethylengruppe, die durch eine $R_5$NH-Gruppe substituiert sein kann, wobei

$R_5$ eine durch eine Alkylgruppe mit 1 bis 5 Kohlenstoffatomen substituierte Carbonylgruppe oder eine durch eine Alkylgruppe mit 1 bis 4 Kohlenstoffatomen substituierte Sulfonylgruppe darstellt,

bedeuten, deren Tautomere, deren Stereoisomere einschließlich deren Gemische und deren Salze, insbesondere jedoch die Verbindungen

4-[4-[[trans-4-[2-(Methoxycarbonyl)-ethyl]-cyclohexyl]-aminocarbonyl]-phenyl]-piperidin,
4-[4-[[4-[2-(n-Butansulfonylamino)-2-(methoxycarbonyl)-ethyl]-phenyl]-aminocarbonyl]-phenyl]-piperidin,
4-[4-[[trans-4-(2-Carboxy-ethyl)-cyclohexyl]-aminocarbonyl]-phenyl]-piperidin,
4-[4-[[4-[2-(n-Butansulfonyl-amino)-2-carboxy-ethyl]-phenyl]-aminocarbonyl]-phenyl]-piperidin,
4-[3-[[4-[2-(n-Butansulfonylamino)-2-carboxy-ethyl]-phenyl]-aminocarbonyl]-phenyl]-piperidin und
4-[3-[[4-[2-(n-Butansulfonylamino)-2-(methoxycarbonyl)-ethyl]-phenyl]-aminocarbonyl]-phenyl]-piperidin,

deren Tautomere, deren Stereoisomere einschließlich deren Gemische und deren Salze.

Erfindungsgemäß erhält man die neuen Verbindungen der allgemeinen Formel I beispielsweise nach folgenden Verfahren:

a) Zur Herstellung von Verbindungen der allgemeinen Formel I, in der $R_a$ ein Wasserstoffatom darstellt: Hydrolyse, Pyrolyse oder Hydrogenolyse einer Verbindung der allgemeinen Formel

A - B - C - D - E - COOR$_a$'  ,(II)

in der
A bis E wie eingangs definiert sind und $R_a'$ mit Ausnahme des Wasserstoffatoms die eingangs für $R_a$ erwähnten Bedeutungen besitzt.

Die Hydrolyse wird zweckmäßigerweise entweder in Gegenwart einer Säure wie Salzsäure, Schwefelsäure, Phosphorsäure, Essigsäure, Essigsäure/Salzsäure, Trichloressigsäure oder Trifluoressigsäure oder in Gegenwart einer Base wie Lithiumhydroxid, Natriumhydroxid oder Kaliumhydroxid in einem geeigneten Lösungsmittel wie Wasser, Methanol, Wasser/Methanol, Äthanol, Wasser/Äthanol, Wasser/Isopropanol, Wasser/Tetrahydrofuran oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bei der sauren Hydrolyse können je nach den angewandten Bedingungen auch andere in einer Verbindung der Formel II gegebenenfalls vorhandene hydrolytisch abspaltbare Gruppen wie die Acetyl-, Trifluoracetyl-, Benzoyl-, tert.Butyloxycarbonyl- oder Benzyloxycarbonylgruppe gleichzeitig abgespalten werden.

Enthält beispielsweise eine Verbindung der Formel II die tert. Butyloxycarbonylgruppe, so kann die tert. Butylgruppe auch durch Behandlung mit einer Säure wie Trifluoressigsäure, Salzsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 °C und 120 °C, z.B. bei Temperaturen zwischen 0 und 60 °C, oder auch thermisch gegebenenfalls in einem inerten Lösungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Enthält beispielsweise eine Verbindung der Formel II die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe oder eine Benzyloxygruppe zur Hydroxygruppe und eine Benzylaminogruppe zu einer Aminogruppe mitreduziert werden. Außerdem können gleichzeitig C = C-Doppelbindung zu Einfachbindungen aufhydriert werden.

b) Zur Herstellung von Verbindungen der allgemeinen Formel I, in der A durch den Rest $R_b$ substituiert ist:
Umsetzung einer Verbindung der allgemeinen Formel

$A_1$ - B - C - D - E - $COOR_a$ ,(III)

in der
B bis E und $R_a$ wie eingangs definiert sind und
$A_1$ eine über ein Kohlenstoffatom mit dem Rest B verknüpfte Aminoalkylgruppe mit 1 bis 5 Kohlenstoffatomen oder eine über ein Kohlenstoffatom mit dem Rest B verknüpfte 5-, 6-, oder 7-gliedrige Azacycloalkylgruppe darstellt, wobei die Kohlenstoffatome der Azacycloalkylgruppe durch ein bis drei Alkylgruppen mit jeweils 1 bis 3 Kohlenstoffatomen, durch eine Aminocarbonyl-, Cyano-, $R_3O$- oder $R_3O$-CO-Gruppe substituiert sein können, wobei $R_3$ wie eingangs erwähnt definiert ist, mit einer Verbindung der allgemeinen Formel

$R_b$ - $Z_1$ ,(IV)

in der
$R_b$ wie eingangs definiert ist und
$Z_1$ eine nukleophile Austrittsgruppe wie ein Halogenatom, z.B. ein Chlor-, Brom- oder Jodatom, oder auch,
wenn $Z_1$ an eine Carbonylgruppe gebunden ist, eine Hydroxy-, Alkanoyloxy- oder Alkoxycarbonylosygruppe darstellt, oder auch

$Z_1$ zusammen mit einem benachbarten Wasserstoffatom des Restes $R_b$ ein Sauerstoffatom bedeuten.

Die Umsetzung wird vorzugsweise in einem geeigneten Lösungsmittel gegebenenfalls in Gegenwart einer Base oder gegebenenfalls in Gegenwart eines die Säure aktivierenden Mittels oder in Gegenwart eines Reduktionsmittels bei Temperaturen zwischen -30 und 150°C, durchgeführt.

Bedeutet $Z_1$ eine nukleophile Austrittsgruppe, so wird die Umsetzung zweckmäßigerweise in einem Lösungsmittel oder Lösungsmittelgemisch wie Wasser, Tetrahydrofuran, Tetrahydrofuran/Wasser, Dioxan, Dioxan/Wasser, Methylenchlorid, Chloroform, Essigester oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, gegebenenfalls in Gegenwart eines Reaktionsbeschleunigers wie Kaliumjodid bei Temperaturen zwischen -30 und 100°C, vorzugsweise jedoch bei Temperaturen zwischen -10 und 80°C, durchgeführt.

Bedeutet $Z_1$ eine Hydroxygruppe, so wird die Umsetzung zweckmäßigerweise in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid,2-(1H-Benztriazolyl)-1,1,3,3-tetramethyl-uronium-Salzen, N,N'-Carbonyldiimidazol, N,N'-Thionyldiimidazol oder Triphenylphosphin/Tetrachlorkohlenstoff, gebenenfalls in Gegenwart von Dimethylaminopyridin oder 1-Hydroxy-benzotriazol, zweckmäßigerweise bei Temperaturen zwischen 0 und 150°C, vorzugsweise bei Temperaturen zwischen 0 und 50°C, durchgeführt.

Bedeutet $Z_1$ zusammen mit einem benachbarten Wasserstoffatoms des Restes $R_b$ ein Sauerstoffatom so wird die reduktive Alkylierung zweckmäßigerweise in einem geeigneten Lösungsmittel wie Methanol, Ethanol, Tetrahydrofuran, Dioxan, Acetonitril oder deren Gemische mit Wasser in Gegenwart eines geeigneten Reduktionsmittels wie Ameisensäure oder eines geeigneten komplexen Metallhydrids, vorzugsweise jedoch in Gegenwart von Natriumcyanborhydrid, oder mit Wasserstoff in Gegenwart eines Hydrierungskatalysators wie Palladium/Kohle bei Temperaturen zwischen 0 und 50°C, vorzugsweise jedoch bei Raumtemperatur, durchgeführt.

c) Zur Herstellung von Verbindungen der allgemeinen Formel I, in der $R_a$ mit Ausnahme des Wasserstoffatoms wie eingangs definiert ist:

Umsetzung einer Verbindung der allgemeinen Formel

A - B - C - D - E - COOH ,(V)

in der

A bis E wie eingangs definiert sind, mit einer Verbindung der allgemeinen Formel

$Z_2$ - $R_a$' ,(VI)

in der

$R_a$' mit Ausnahme des Wasserstoffatoms die für $R_a$ eingangs erwähnten Bedeutungen besitzt und

$Z_2$ eine nukleophile Austrittsgruppe wie ein Halogenatom, z. B. ein Chlor- oder Bromatom, oder eine an der Sulfonylgruppe substituierte Sulfonyloxygruppe, z.B. eine Methansulfonyloxy-, Methoxysulfonyloxy- oder Toluolsulfonyloxygruppe oder eine Hydroxygruppe darstellt.

Die Umsetzung wird, falls $Z_2$ ein Halogenatom oder eine an der Sulfonylgruppe substituierte Sulfonyloxygruppe darstellt, zweckmäßigerweise in einem Lösungsmittel wie Tetrahydrofuran, Dioxan, Methylenchlorid, Chloroform, Essigester, Dimethylsulfoxid oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin oder N-Methyl-morpholin, welche gleichzeitig als Lösungsmittel dienen können, gegebenenfalls in Gegenwart eines Reaktionsbeschleunigers wie Kaliumjodid bei Temperaturen zwischen -30 und 100°C, vorzugsweise jedoch bei Temperaturen zwischen -10 und 80°C, durchgeführt.

Bedeutet $Z_2$ eine Hydroxygruppe, wird die Umsetzung vorzugsweise unter Verwendung der Verbindung VI als Lösungsmittel in Gegenwart von Thionylchlorid oder einer Säure wie Salzsäure oder Schwefelsäure bei Temperaturen zwischen -10°C und 100°C, vorzugsweise bei Temperaturen zwischen 0°C und 50°C, durchgeführt.

d) Zur Herstellung von Verbindungen der allgemeien Formel I, in der C eine $-CH_2-NH$-Gruppe darstellt: Reduktion einer Verbindung der allgemeinen Formel

A - B - CH = N - D - E - $COOR_a$ ,(VII)

in der
A, B, D, E und $R_a$ wie eingangs definiert sind.

Die Reduktion wird vorzugsweise in einem geeigneten Lösungsmittel wie Methanol, Methanol/Wasser, Methanol/Ammoniak, Methanol/Wasser/Ammoniak, Methanol/Salzsäure, Methanol/etherische Salzsäure, Ethanol, Essigsäureethylester, Ether, Tetrahydrofuran, Dioxan, Dimethylformamid oder Eisessig in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Natriumcyanborhydrid oder Lithiumborhydrid bei Temperaturen zwischen -20°C und 100°C, vorzugsweise bei Temperaturen zwischen 0°C und 60°C, durchgeführt.

e) Zur Herstellung von Verbindungen der allgemeinen Formel I, in der C eine $-CO-NR_4$-Gruppe darstellt: Umsetzung einer Verbindung der allgemeinen Formel

$A_2$ - B - COOH ,(VIII)

in der
B wie eingangs definiert ist und
$A_2$ eine am Stickstoffatom durch einen Alkyl-, Aralkyl-, Alkanoyl-, Trifluoracetyl- oder Alkoxycarbonylrest substituierte Gruppe A darstellt, mit einem Amin der allgemeinen Formel

$R_4$ - NH - D - E - $COOR_a'$ ,(IX)

in der
D, E und $R_4$ wie eingangs definiert sind und $R_a'$ mit Ausnahme des Wasserstoffatoms die für $R_a$ eingangs erwähnten Bedeutungen besitzt.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid,2-(1H-Benztriazolyl)-1,1,3,3-tetramethyluronium-Salzen, N,N'-Carbonyldiimidazol, N,N'-Thionyldiimidazol oder Triphenylphosphin/Tetrachlorkohlenstoff, gegebenenfalls in Gegenwart von Dimethylaminopyridin oder 1-Hydroxybenzotriazol und/oder einer Base wie Triethylamin, N-Ethyl-diisopropylamin oder N-Methyl-morpholin, zweckmäßigerweise bei Temperaturen zwischen -10 und 150°C, vorzugsweise bei Temperaturen zwischen 0 und 50°C, durchgeführt.

f) Zur Herstellung von Verbindungen der allgemeinen Formel I, in der C eine $-NR_4-CO$-Gruppe darstellt: Umsetzung einer Verbindung der allgemeinen Formel

$A_2$ - B - $NH-R_4$ ,(X)

in der
$R_4$ und B wie eingangs definiert sind und
$A_2$ eine am Stickstoffatom durch einen Alkyl-, Aralkyl-, Alkanoyl-, Trifluoracetyl- oder Alkoxycarbonylrest substituierte Gruppe A darstellt, mit einer Carbonsäure der allgemeinen Formel

HOOC - D - E - $COOR_a'$ ,(XI)

in der
D und E wie eingangs definiert sind und
$R_a'$ mit Ausnahme des Wasserstoffatoms die für $R_a$ eingangs erwähnten Bedeutungen besitzt.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart eines wasserentziehenden Mittels, z.B. in

Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid,2-(1H-Benztriazolyl)-1,1,3,3-tetramethyluronium-Salzen, N,N'-Carbonyldiimidazol, N,N'-Thionyldiimidazol oder Triphenylphosphin/Tetrachlorkohlenstoff, gegebenenfalls in Gegenwart von Dimethylaminopyridin oder 1-Hydroxybenzotriazol und/oder einer Base wie Triethylamin, N-Ethyl-diisopropylamin oder N-Methyl-morpholin, zweckmäßigerweise bei Temperaturen zwischen -10 und 150 °C, vorzugsweise bei Temperaturen zwischen 0 und 50 °C, durchgeführt.

g) Zur Herstellung von Verbindungen der allgemeinen Formel I, in der A eine über ein Kohlenstoffatom mit dem Rest B verknüpfte Aminoalkylgruppe mit 1 bis 5 Kohlenstoffatomen darstellt:
Reduktion einer Verbindung der allgemeinen Formel

$A_3$ - B - C - D - E - $COOR_a$ ,(XII)

in der
B bis E und $R_a$ wie eingangs definiert sind und
$A_3$ eine Cyanogruppe oder eine Cyanoalkylgruppe mit 1 bis 4 Kohlenstoffatomen im Alkylteil darstellt.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100 °C, vorzugsweise bei Temperaturen zwischen 20 und 80 °C, durchgeführt.

Erhält man erfindungsgemäß eine Verbindung der allgemeinen Formel I, in der $R_4$ ein Wasserstoffatom darstellt, so kann diese mittels Alkylierung in eine entsprechende Verbindung der allgemeinen Formel I, in der $R_4$ eine gegebenenfalls durch eine Phenylgruppe substituierte Alkylgruppe mit 1 oder 2 Kohlenstoffatomen darstellt, übergeführt werden.

Die nachträgliche Alkylierung wird zweckmäßigerweise mit einem entsprechenden Halogenid wie Methyljodid, Ethylbromid, Benzylchlorid oder Phenylethylbromid vorzugsweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden können.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 °C, vorzugsweise bei Temperaturen zwischen 10 und 50 °C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50 °C, vorzugsweise jedoch bei Raumtemperatur, und bei einem Wasserstoffdruck von 1 bis 7 bar, vorzugsweise jedoch von 3 bis 5 bar. Bei der hydrogenolytischen Abspaltung von Benzylgruppen können gleichzeitig gegebenenfalls in einer Verbindung der Formel II vorhandene C=C-Doppelbindungen aufhydriert werden. Beispielsweise kann auf diese Weise eine N-Benzyldehydro-piperidylgruppe in eine Piperidylgruppe umgewandelt werden.

Die Abspaltung einer Methoxybenzylgruppe kann auch in Gegenwart eines Oxidationsmittels wie Cer-(IV)ammoniumnitrat in einem Lösungsmittel wie Methylenchlorid, Acetonitril oder Acetonitril/Wasser bei Temperaturen zwischen 0 und 50 °C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Ether oder Dioxan/Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 °C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und in Gegenwart eines Überschusses eines Allylgruppenacceptors wie Morpholin oder 1,3-Dimedon bei Temperaturen zwischen 0 und 100 °C, vorzugsweise bei Raumtemperatur und unter Inertgas, oder durch Behandlung mit einer katalytischen Menge von Tris-(triphenylphosphin)-rhodium(I)-chlorid in einem Lösungsmittel wie wäßrigem Ethanol und gegebenenfalls in Gegenwart einer Base wie 1,4-Diazabicyclo-[2.2.2]octan bei Temperaturen zwischen 20 und 70 °C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomeren, und chirale Verbindungen in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Ethanolamin, Diethanolamin und Triethanolamin in Betracht.

Die als Ausgangsstoffe verwendeten Verbindungen sind teilweise literaturbekannt oder man erhält diese nach literaturbekannten Verfahren (siehe Beispiele I bis XXIV).

Wie bereits eingangs erwähnt, weisen die neuen Carbonsäurederivate der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I, in denen A eine gegebenenfalls am Stickstoff substituierte Aminoalkyl-, Aza- oder Diazacycloalkylgruppe oder eine Pyridyl- oder Chinuclidinylgruppe darstellt oder eine gegebenenfalls in vivo in eine Aminoalkyl-, Aza- oder Diazacycloalkylgruppe überführbare Gruppe, z.B. eine am Stickstoffatom durch eine Alkoxycarbonyl-, Alkenyloxycarbonyl-, Aralkoxycarbonyl-, Alkylcarbonyl-, Trifluormethylcarbonyl-, Alkanoyloxymethoxycarbonyl-, Cycloalkanoyloxymethoxycarbonyl- oder Aralkanoyloxymethoxycarbonylgruppe substituierte Amino-, Aza- oder Diazacycloalkylgruppe enthält und -$COOR_a$ eine Carboxylgruppe oder eine in vivo in eine Carboxylgruppe überführbare Gruppe, z.B. eine Alkoxy-, Alkenyloxy-, Phenylalkoxy-, Cycloalkyloxy-, Alkanoyloxyalkoxy-, Cycloalkanoyloxyalkoxy-, Phenylalkanoyloxyalkoxy-, Benzoyloxyalkoxy-, Alkoxycarbonyloxyalkoxy- oder Cycloalkyloxycarbonyloxyalkoxy-carbonylgruppe darstellt, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

1. Kompetitive Bindung $^3$H-BIBU 52/Testsubstanz an Humanthrombozyten:

Eine Suspension von Humanthrombozyten in Plasma wird mit $^3$H-BIBU 52 [= (3S,5S)-5-[(4'-Amidino-4-biphenylyl)oxymethyl]-3-[(carboxyl)methyl]-2-pyrrolidinon[3-$^3$H-4-biphenylyl]), das den literaturbekannten Liganden $^{125}$ I-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 $\mu$l werden mit 50 $\mu$l physiologischer Kochsalzlösung, 100 $\mu$l Testsubstanzlösung, 50 $\mu$l $^{14}$C-Sucrose (3.700 Bq) und 50 $\mu$l $^3$H-BIBU 52 (Endkonzentration: 5 nM) bei Raumtemperatur 20 Minuten inkubiert. Zur Messung der unspezifischen Bindung wird anstelle der Testsubstanz 5 $\mu$l BIBU 52 (Endkonzentration: 30 $\mu$M) eingesetzt. Die Proben werden 20 Sekunden bei 10000 x g zentrifugiert und der Überstand abgezogen. 100 $\mu$l hiervon werden zur Bestimmung des freien Liganden gemessen. Das Pellet wird in 500 $\mu$l 0,2N NaOH gelöst, 450 $\mu$l werden mit 2 ml Szintillator und 25 $\mu$l 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. Antithrombotische Wirkung

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchen-Suspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Konzentration wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37°C inkubiert.

Aus den Konzentrations-Wirkungskurven wird die $EC_{50}$ berechnet, die die Konzentration beschreibt, bei der die Änderung der "optical density" halbmaximal gehemmt ist.

Die nachfolgende Tabelle enthält die gefundenen Ergebnisse:

| Substanz (Beispiel Nr.) | Kompetitive Bindung $^3$H-BIBU 52/Textsubstanz an Humanthrombozyten $IC_{50}$ [nM] | Hemmung der Plättchenaggregation $EC_{50}$[nM] |
|---|---|---|
| 2 | 1 100 | 840 |
| 2.1 | >100 000 | 3 300 |
| 2.2 | 4 500 | 3 300 |
| 3 | 190 | 260 |
| 3.1 | 1 400 | 2 400 |
| 3.3 | 17 | 260 |
| 3.4 | 2 000 | 5 800 |
| 3.6 | 14 | 120 |
| 5 | 2 900 | 5 600 |

Die Hemmung der Thrombozytenaggregation nach oraler Gabe der Prüfsubstanz wird ex vivo an Rhesusaffen ermittelt.

Direkt vor der oralen Verabreichung der in Natrosol suspendierten Prüfsubstanz wird den Tieren eine Blutprobe aus der Kubitalvene als Referenzwert entnommen. Zu definierten Zeiten nach der Substanzgabe werden erneut Blutproben gewonnen und wie folgt untersucht.

Das mit 3,14% Natriumcitrat im Volumenverhältnis 1:10 versetzte Vollblut wird mit 200 g für 15 Minuten zentrifugiert. Der Überstand an plättchenreichem Plasma wird vorsichtig abgehoben. Aus dem erythrozytenreichen Sediment wird durch Zentrifugation mit 4000 g für 10 Minuten das plättchenarme Plasma als Überstand gewonnen.

Die mit Collagen (Hormonchemie, München; 2 µg/ml Endkonzentration im plättchenreichen Plasma) ausgelöste Thrombozytenaggregation in diesen ex vivo Proben wird nach der Methode von Born und Cross (J. Physiol. 170, 397 (1964)) photometrisch gemessen. Die nach Collagenstimulation gemessene größte Lichtdurchlässigkeit des plättchenreichen Plasmas wird mit dem Referenzwert verglichen, um die Hemmung der Aggregation zu den verschiedenen Zeitpunkten der Blutentnahme nach Substanzgabe relativ zum Referenzwert zu bestimmen.

Die Verbindungen der Beispiele 2 und 5(2) hemmen die Collagen-induzierte Thrombozytenaggregation ex vivo nach oraler Gabe von 1 mg/kg länger als 2 Stunden.

Die erfindungsgemäßen Verbindungen sind gut verträglich, da beispielsweise bei intravenöser Gabe von 30 mg/kg an jeweils 3 Mäusen bei den Verbindungen der Beispiele 2 und 3 keine Tieren gestorben waren.

Auf Grund ihrer Hemmwirkung auf Zell-Zell- bzw. Zell-Matrix-Wechselwirkungen eignen sich die neuen Carbonsäurederivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose, der Metastasierung von Tumoren und der Therapie genetisch bedingter oder auch erworbener Störungen der Interaktion von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmern oder deren Kombinationen, Serotonin-Antagonisten, $\alpha$-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen:

Beispiel I

4-(4-Carboxy-phenyl)-piperidin-hydrochlorid

Zu einer Lösung von 63,0 g 1-Acetyl-4-phenyl-piperidin in 1000 ml Methylenchlorid tropft man unter gutem Rühren bei -10 bis -20 °C 157,4 g Oxalylchlorid. Anschließend gibt man 46,7 g Aluminiumchlorid zu. Man rührt 1 Stunde bei -10 °C und gibt weitere 82,7 g Aluminiumchlorid zu. Nach weiteren 2 Stunden wird das Kühlbad entfernt und 24 Stunden bei Raumtemperatur gerührt. Die Reaktionslösung wird vorsichtig in ca. 4 l Eis/Wasser eingerührt und die wäßrige Phase zweimal mit Methylenchlorid extrahiert. Die vereinigten organischen Phasen werden mit Wasser gewaschen, über Natriumsulfat getrocknet, und das Lösungsmittel unter vermindertem Druck entfernt. Der verbleibende Rückstand wird unter kräftigem Rühren in 2,5 l 2N Natronlauge gelöst. Zur dunklen wäßrigen Lösung gibt man Eis und säuert mit konz. Salzsäure an. Der Niederschlag wird abgenutscht, mit Wasser gewaschen und in 2 l 6N Salzsäure 5 Stunden zum Rückfluß erhitzt. Das Lösungsmittel wird unter vermindertem Druck entfernt. Der verbleibende Feststoff wird mit wenig Wasser verrieben und abgenutscht.
Ausbeute: 40,5 g (54 % der Theorie),
Schmelzpunkt: > 300 °C
$R_f$-Wert: 0,07 (Kieselgel; Methylenchlorid/Methanol/konz. Ammoniak = 4:1:0,25)

Beispiel II

1-tert.Butyloxycarbonyl-4-(4-carboxy-phenyl)-piperidin

Zu 16,4 g Natriumhydroxid in 300 ml Wasser gibt man vorsichtig 47,5 g 4-(4-Carboxy-phenyl)-piperidin-hydrochlorid. Die Suspension wird mit 500 ml Dioxan und 250 ml Wasser verdünnt. Anschließend werden 54,6 g Pyrokohlensäure-di-tert.butylester portionsweise zugegeben. Man rührt 16 Stunde bei Raumtemperatur. Der Niederschlag wird abgenutscht und das Filtrat unter vermindertem Druck teilweise eingedampft. Der Niederschlag und das verbleibende wäßrige Filtrat werden vereinigt und mit 1 l Wasser verdünnt. Die wäßrige Phase wird mit gesättigter Kaliumhydrogensulfat-Lösung auf pH 2 gebracht und zweimal mit Essigester extrahiert. Die vereinigten Essigester-Phasen werden mit gesättigter Kochsalzlösung gewaschen, über Natriumsulfat getrocknet und das Lösungsmittel unter vermindertem Druck entfernt. Das kristalline Rohprodukt wird mit wenig Essigester verrieben, abgenutscht und getrocknet.
Ausbeute: 54,0 g (90 % der Theorie),
Schmelzpunkt: 172-174 °C
$R_f$-Wert: 0,73 (Kieselgel; Essigester/Cyclohexan = 4:1)

Beispiel III

3-(4-Amino-phenyl)-2-(n-butylsulfonylamino-propionsäure-methylester

19 g 2-(n-Butansulfonylamino)-3-(4-nitro-phenyl)-propionsäure-methylester werden in 200 ml Essigester in Gegenwart von 2 g 10%iger Palladiumkohle 1,5 Stunden bei Raumtemperatur mit Wasserstoff von 5 bar Druck behandelt. Man filtriert vom Katalysator ab, dampft ein und verwendet den Rückstand ohne weitere Reinigung.
Ausbeute: 17,8 g (100 % der Theorie),
$R_f$-Wert: 0,43 (Kieselgel; Cyclohexan/Essigester = 1:1)

Analog wird folgende Verbindung erhalten:

(1) 3-(4-Amino-phenyl-)propionsäure-methylester
Man geht von 4-Nitro-zimtsäure-methylester aus und hydriert bei 50 °C.
$R_f$-Wert: 0,76 (Kieselgel; Cyclohexan/Essigester = 1:3)

Beispiel IV

2-(n-Butansulfonylamino)-3-(4-nitro-phenyl)propionsäure-methylester

25,9 g 2-Amino-3-(4-nitro-phenyl)-propionsäure-methylester-hydrochlorid werden in 100 ml Methylenchlorid suspendiert und mit 32 g N-Ethyl-diisopropylamin versetzt, wobei sich der Niederschlag auflöst. Zu der Lösung werden bei 8 bis 15 °C 17,1 g n-Butansulfonylchlorid in 20 ml Methylenchlorid zugetropft. Nach 16-stündigem Rühren bei Raumtemperatur werden unter Eiskühlung 10 g N-Ethyl-diisopropylamin zugegeben und nochmals 8 g n-Butansulfonylchlorid zugetropft und weitere 2 Stunden bei Raumtemperatur gerührt. Man versetzt mit Eiswasser, wäscht die organische Phase nacheinander mit Wasser, 1N Salzsäure und Wasser und dampft ein. Der Rückstand wird über Kieselgel gereinigt (Elutionsmittel: Methylenchlorid).
Ausbeute: 19,4 g (57 % der Theorie),
Schmelzpunkt: 100-102 °C
$R_f$-Wert: 0,38 (Kieselgel; Cyclohexan/Essigester = 6:4)

Beispiel V

2-Amino-3-(4-nitro-phenyl)-propionsäure-methylester-hydrochlorid

21 g 4-Nitro-phenylalanin werden in 250 ml Methanol suspendiert und mit 10 ml methanolischer Salzsäure versetzt, wobei sich das Festprodukt auflöst. Man läßt 60 Stunden bei Raumtemperatur stehen und dampft im Vakuum ein. Das Produkt wird ohne weitere Reinigung verwendet.
Ausbeute: 25,9 g (99 % der Theorie),
Schmelzpunkt: 206-208 °C (Zers.)
$R_f$-Wert: 0,67 (Reversed Phase Platte RP8; Methanol/5%ige Natriumchloridlösung = 6:4)

Analog wird folgende Verbindung erhalten:

(1) 3-(trans-4-Amino-cyclohexyl)-propionsäure-methylester-hydrochlorid
Schmelzpunkt: über 200 °C

Beispiel VI

3-(trans-4-Amino-cyclohexyl)-propionsäure-hydrochlorid

26 g 3-(trans-4-Acetamino-cyclohexyl)-propionsäure werden in 200 ml 6N Salzsäure 16 Stunden zum Rückfluß erhitzt. Man dampft im Vakuum zur Trockene ein und dampft mehrmals nach Zugabe von Toluol und Methanol ein. Der Rückstand wird direkt weiter verwendet.
Ausbeute: 26 g (100 % der Theorie)

Beispiel VII

3-(trans-4-Acetamino-cyclohexyl)-propionsäure

112,7 g 3-(4-Acetamino-phenyl)-propionsäure und 10 g Platindioxid werden in 350 ml Eisessig bei 60 °C mit Wasserstoff von 5 bar Druck behandelt. Nach 1,5 und 7 Stunden wird das Platindioxid jeweils gegen neues ausgetauscht. Die gesamte Reaktionszeit beträgt 10 Stunden. Man dampft im Vakuum zur Trockne ein und kristallisiert den Rückstand aus 1800 ml Aceton um. Das Primärkristallisat wird noch aus 50 ml 80%iger Essigsäure umkristallisiert.
Ausbeute: 26 g (22,4 % der Theorie),
Schmelzpunkt: 200-201 °C
$R_f$-Wert: 0,30 (Kieselgel; Methylenchlorid/Essigester/ Eisessig = 4:1:0,4)
Aus den Aceton-Mutterlaugen gewinnt man nach Eindampfen und Umkristallisieren aus 100 ml Wasser 53,6 g (46 % der Theorie) cis-3-(4-Acetamino-cyclohexyl)-propionsäure.

Beispiel VIII

1-Benzyl-4-(3-carboxy-phenyl)-3,4-dehydro-piperidin-hydrochlorid

25 g 1-Benzyl-4-(3-brom-phenyl)-3,4-dehydro-piperidin werden in 300 ml Tetrahydrofuran gelöst. Man kühlt auf unter -65°C ab und tropft bei dieser Temperatur innerhalb von 30 Minuten 47,6 ml einer 1,6-molaren Lösung von n-Butyllithium in Hexan zu. Man rührt noch eine Stunde weiter und leitet dann einen langsamen Strom von über konz. Schwefelsäure getrocknetem Kohlendioxid über die Reaktionslösung. Dabei hält man die Temperatur zunächst 1 Stunde unter -65°C, läßt dann langsam auf Raumtemperatur kommen und läßt das Reaktionsgemisch bei dieser Temperatur 60 Stunden stehen. Man dampft zur Trockne ein, nimmt den Rückstand in 500 ml Essigester auf und extrahiert mit Wasser. Die wäßrigen Phasen werden im Vakuum eingeengt, im Eisbad abgekühlt und mit 2N Salzsäure auf pH 1 gebracht. Die ausgefallenen Kristalle werden abfiltriert und mit Wasser gewaschen.
Ausbeute: 9 g (40 % der Theorie),
Schmelzpunkt: ab 185°C (Zers.)
$R_f$-Wert: 0,50 (Reversed Phase Platte RP8; Methanol/5%ige Natriumchloridlösung = 6:4)

Beispiel IX

1-Benzyl-4-(3-brom-phenyl)-3,4-dehydro-piperidin

29,6 g 1-Benzyl-4-(3-brom-phenyl)-4-hydroxy-piperidin, 32,5 g p-Toluolsulfonsäure-hydrat und 300 ml Toluol werden 2 Stunden am Wasserabscheider erhitzt. Nach dem Abkühlen verdünnt man mit Methylenchlorid, gibt Eiswasser zu und stellt mit 30%iger Natronlauge alkalisch. Die Wasserphase wird nochmals mit Methylenchlorid extrahiert und die vereinigten organischen Phasen im Vakuum eingedampft.
Ausbeute: 25,2 g (90 % der Theorie),
$R_f$-Wert: 0,69 (Kieselgel; Methylenchlorid/Methanol = 100:2)

Beispiel X

1-Benzyl-4-(3-brom-phenyl)-4-hydroxy-piperidin

Zu einer Lösung von 35,7 g 1,3-Dibrom-benzol in 200 ml Ether tropft man im Verlaufe von 25 Minuten bei einer Temperatur unter 0°C 93,1 ml einer 1,6-molaren Lösung von n-Butyllithium in Hexan. Man rührt 40 Minuten weiter und tropft dann bei einer Temperatur unter 10°C eine Lösung von 28,2 g frisch destilliertem 1-Benzyl-4-piperidon in 40 ml Ether zu. Man rührt noch 30 Minuten bei dieser Temperatur und schließlich 1 Stunde bei Raumtemperatur. Man versetzt mit 500 ml gesättigter Kochsalzlösung, extrahiert die wäßrige Phase noch zweimal mit Essigester, dampft die organischen Phasen im Vakuum ein und reinigt den Rückstand säulenchromatographisch über Kieselgel (Elutionsmittel: Methylenchlorid/Methanol = 100:2)
Ausbeute: 29,6 g (56 % der Theorie),
$R_f$-Wert: 0,34 (Kieselgel; Methylenchlorid/Methanol = 100:2)

Beispiel XI

3-(4-Acetamino-phenyl)-propionsäure

155 g 3-(4-Acetamino-phenyl)-propionsäure-methylester werden in 1000 ml Methanol gelöst, auf 50°C erwärmt und mit 700 ml 2N Natronlauge versetzt. Man läßt auf Raumtemperatur abkühlen und rührt noch 3 Stunden nach. Man kühlt im Eisbad ab, fügt 750 ml 2N Salzsäure zu und rührt eine Stunde bei Eiskühlung weiter. Der Niederschlag wird abfiltriert und mit wenig Eiswasser gewaschen.
Ausbeute: 112,7 g (77,6 % der Theorie),
Schmelzpunkt: 144-147°C
$R_f$-Wert: 0,25 (Kieselgel; Methylenchlorid/Essigester/ Eisessig = 8:1:0,4)

Beispiel XII

3-(4-Acetamino-phenyl)-propionsäure-methylester

138,4 g 3-(4-Amino-phenyl)-propionsäure-methylester werden in 1000 ml Methylenchlorid gelöst und 119 ml Triethylamin zugegeben. Die Mischung wird auf unter 0°C abgekühlt und tropfenweise innerhalb von 35 Minuten bei 0-10°C mit einer Lösung von 58 ml Acetylchlorid in 200 ml Methylenchlorid versetzt. Man rührt eine Stunde bei 0°C nach, versetzt mit 300 ml Wasser, extrahiert die organische Phase noch zweimal mit Wasser und dampft sie im Vakuum ein.
Ausbeute: 155,5 g (91 % der Theorie),
Schmelzpunkt: 118-120°C
$R_f$-Wert: 0,50 (Kieselgel; Cyclohexan/Essigester = 1:3)

Beispiel XIII

4-[4-[[4-[2-(Methoxycarbonyl)-ethyl]-phenyl]-iminomethyl]-phenyl]-1-trifluoracetyl-piperidin

6,9 g 4-(4-Formyl-phenyl)-1-trifluoracetyl-piperidin und 4,33 g 3-(4-Amino-phenyl)-propionsäure-methylester werden in 50 ml Toluol 5 Stunden unter Rückfluß am Wasserabscheider erhitzt. Man dampft im Vakuum ein und verwendet das Produkt ohne weitere Reinigung.
Ausbeute: 10,8 g (100 % der Theorie),
$R_f$-Wert: 0,43 (Kieselgel; Cyclohexan/Essigester = 2:1)

Beispiel XIV

4-(4-Formyl-phenyl)-1-trifluoracetyl-piperidin

Zu einer Lösung von 8,3 g 4-Phenyl-1-trifluoracetyl-piperidin in 30 ml Methylenchlorid werden bei einer Temperatur unter 0°C 7,8 ml Titantetrachlorid zugetropft. Nach 10 Minuten tropft man im Laufe von 40 Minuten 3,5 ml Dichlormethylmethylether zu, wobei die Temperatur auf unter 0°C gehalten wird. Man läßt 16 Stunden bei Raumtemperatur stehen, gießt auf Eiswasser und extrahiert die wäßrige Phase mehrfach mit Methylenchlorid. Die organischen Phasen werden im Vakuum eingedampft und der Rückstand säulenchromatographisch über Kieselgel gereinigt (Elutionsmittel: Cyclohexan/Essigester = 2:1).
Ausbeute: 6,9 g (75 % der Theorie),
Schmelzpunkt: 76-77°C
$R_f$-Wert: 0,42 (Kieselgel; Cyclohexan/Essigester = 2:1)

Beispiel XV

4-Phenyl-1-trifluoracetyl-piperidin

25 g 4-Phenyl-piperidin werden in 250 ml Methylenchlorid gelöst, mit 29,8 ml N-Ethyl-diisopropylamin versetzt und auf 0°C abgekühlt. Zu der Lösung tropft man 24,1 ml Trifluoressigsäureanhydrid so, daß die Temperatur 10°C nicht übersteigt. Man rührt noch 30 Minuten bei 0°C, läßt auf Raumtemperatur kommen, fügt 100 ml Wasser zu und wäscht die organische Phase noch zweimal mit Wasser. Die Methylenchloridphase wird eingedampft und der Rückstand ohne weitere Reinigung verwendet.
Ausbeute: 40 g (100 % der Theorie),
$R_f$-Wert: 0,90 (Kieselgel; Cyclohexan/Essigester = 1:1)

Beispiel XVI

4-(4-Amino-phenyl)-1-trifluoracetyl-piperidin

a) 4-Phenyl-1-trifluoracetyl-piperidin

Zu einer Lösung von 125 g (0,775 Mol) 4-Phenyl-piperidin und 149 ml (0,775 Mol) N,N-Diisopropylethylamin in 1300 ml Dichlormethan gibt man bei -5°C unter Rühren während 2 Stunden tropfenweise 120,5 ml (0,775 Mol) Trifluoressigsäureanhydrid. Anschließend wird eine weitere Stunde unter Eiskühlung

gerührt, über Nacht bei Raumtemperatur stehen gelassen und dann mit 400 ml Wasser verdünnt. Die Dichlormethan-Phase wird abgetrennt, 2 x mit je 400 ml Wasser gewaschen, über Natriumsulfat getrocknet und unter Vakuum zur Trockne eingeengt.
Ausbeute: 193 g (97 % der Theorie)
Gelbe Kristalle
$R_f$-Wert: 0,88 (Kieselgel; Cyclohexan/Essigester = 1:1)

b) 4-(4-Nitro-phenyl)-1-trifluoracetyl-piperidin

80 g (0,311 Mol) des unter a) gewonnenen rohen 4-Phenyl-1-trifluoracetyl-piperidins werden in einer Mischung aus 400 ml Eisessig und 200 ml Acetanhydrid gelöst. Diese Lösung wird im Eisbad auf 10 °C abgekühlt und unter Rühren mit 1,6 g Natriumnitrit und anschließend tropfenweise mit 51,9 ml (0,311 Mol) rauchender Salpetersäure versetzt. Man läßt über Nacht bei Raumtemperatur stehen und gießt dann auf 1000 ml Wasser, das 200 g Eis enthält. Mittels 8N Natronlauge wird innerhalb 4,5 Stunden unter Kühlen auf pH 8 eingestellt und zwar so, daß die Temperatur 20 °C nicht übersteigt. Man extrahiert mit insgesamt 2000 ml Dichlormethan, wäscht die vereinigten Dichlormethan-Extrakte mit 100 ml 0,1 N Natronlauge und anschließend 2 x mit Wasser, trocknet über Natriumsulfat und dampft unter Vakuum zur Trockne ein. Der Rückstand wird aus Essigester/Cyclohexan kristallisiert.
Ausbeute: 54,5 g (58 % der Theorie)
Gelbe Kristalle
Schmelzpunkt: 100-102 °C

c) 4-(4-Amino-phenyl)-1-trifluoracetyl-piperidin

Die unter b) hergestellte Verbindung wird in 700 ml Essigester gelöst und nach Zusatz von 7 g Palladium auf Kohle (10%ig) bei Raumtemperatur und mit 3 bar Wasserstoff-Druck hydriert. Der Katalysator wird abfiltriert und die Essigester-Lösung unter Vakuum zur Trockne eingeengt.
Ausbeute: 39,7 g (quantitativ)
$R_f$-Wert: 0,25 (Kieselgel; Cyclohexan: Essigester = 1:1)

## Beispiel XVII

1-tert.Butyloxycarbonyl-4-[4-[[4-[2-(methoxycarbonyl)-ethyl]-piperidino]-methyl]-phenyl]-piperidin

a) 1-tert.Butyloxycarbonyl-4-(4-hydroxymethyl-phenyl)-piperidin

Zu einer Lösung von 5 g (0,0164 Mol) 1-tert.Butyloxycarbonyl-4-(4-carboxy-phenyl)-piperidin und 1,66 g (0,0164 Mol) = 2,28 ml Triethylamin in 100 ml Tetrahydrofuran tropft man unter Rühren und bei 5 °C eine Lösung von 1,78 g (0,0164 Mol) = 1,57 ml Chlorameisensäureethylester in 10 ml Tetrahydrofuran und rührt bei dieser Temperatur eine weitere Stunde. Das ausgefallene Triethylamin-hydrochlorid wird hiernach abgesaugt und mit Tetrahydrofuran gewaschen. Die vereinigten Tetrahydrofuran-Phasen werden nun unter Rühren und bei 10 bis 15 °C in eine Lösung von 1,55 g (0,041 Mol) Natriumborhydrid eingetropft. Nach Rühren über Nacht bei Raumtemperatur wird unter Vakuum zur Trockne eingeengt. Der verbleibende Rückstand wird zwischen Essigester und 1N Natriumhydroxidlösung verteilt. Die Essigester-Phase wird mit Wasser gewaschen, über Natriumsulfat getrocknet und unter Vakuum zur Trockne eingeengt. Der verbleibende Rückstand wird aus Petrolether kristallisiert.
Ausbeute: 4,05 g (84,7 % der Theorie),
Schmelzpunkt: 78-80 °C

b) 1-tert.Butyloxycarbonyl-4-(4-chlormethyl-phenyl)-piperidin

Eine Lösung von 3,95 g (0,0136 Mol) der Verbindung a) und 2,74 g (0,0271 Mol) = 3,8 ml Triethylamin in 80 ml Dichlormethan wird langsam und unter Rühren mit 3,1 g (0,0271 Mol) = 2,1 ml Mesylchlorid versetzt. Nach beendeter Zugabe wird über Nacht stehen gelassen und anschließend die klare Losung zur Trockne eingeengt. Der verbleibende Rückstand wird über Kieselgel chromatographiert, wobei Dichlormethan als Elutionsmittel dient.
Ausbeute: 3,75 g (89 % der Theorie),
Schmelzpunkt: 56-58 °C

$R_f$-Wert: 0,47 (Kieselgel; Dichlormethan)

c) 1-tert.Butyloxycarbonyl-4-[4-[[4-[2-(methoxycarbonyl)-ethyl]-piperidino]-methyl]-phenyl]-piperidin

Eine Mischung aus 1,8 g (0,0058 Mol) der unter b) hergestellten Verbindung, 1,44 g (0,007 Mol) Piperidinopropionsäuremethylesterhydrochlorid, 1,42 g = 1,95 ml (0,014 Mol) Triethylamin und 3 g Natriumjodid in 150 ml Chloroform wird während 36 Stunden auf Rückflußtemperatur erhitzt. Danach werden die ungelösten Anteile abgesaugt. Das Filtrat wird zweimal mit Wasser gewaschen, über Natriumsulfat getrocknet und unter Vakuum zur Trockne eingeengt. Der verbleibende Rückstand wird mittels Chromatographie über Kieselgel gereinigt, wobei Methylenchlorid/Methanol (35:1) als Elutionsmittel dient.
Ausbeute: 2,05 g (79,8 % der Theorie)
Harz
$R_f$-Wert: 0,41 (Kieselgel; Dichlormethan/Methanol = 9,5:0,5)

Analog wurde folgende Verbindung hergestellt:

(1) 1-tert.Butyloxycarbonyl-4-[4-[[trans-4-[2-(methoxycarbonyl)-ethyl]-cyclohexyl]-aminomethyl]-phenyl]-piperidin

Hergestellt aus 1-tert.Butyloxycarbonyl-4-(4-chlormethylphenyl)-piperidin und trans-3-[4-Amino-cyclohexan]-propionsäuremethylester
Harz

Beispiel XVIII

1-[tert.Butyloxycarbonyl)-4-[4-[[trans-4-methoxycarbonylcyclohexyl]-aminocarbonylamino]-phenyl]-piperidin

Eine Lösung von 1,2 g (0,0044 Mol) 1-tert.Butyloxycarbonyl4-[4-amino-phenyl]-piperidin und 0,8 g (0,0044 Mol) trans-[4-Methoxy-carbonyl-cyclohexyl]-isocyanat in 20 ml Dioxan wird während 2 Stunden auf 50 °C erwärmt. Anschließend wird unter vermindertem Druck zur Trockne eingedampft und der feste Rückstand mit tert.Butylmethylether verrührt. Der verbleibende Festkörper wird abgesaugt, mit tert.Butylmethylether gewaschen und getrocknet.
$R_f$-Wert: 0,30 (Kieselgel; Cyclohexan/Essigester = 1:1)

Beispiel XIX

1-tert.Butyloxycarbonyl-[4-[[trans-4-[2-(methoxycarbonyl)-ethyl]-cyclohexyl]-aminocarbonyl]-piperidino]-piperidin

a) 1-tert.Butyloxycarbonyl-4-[(4-ethoxycarbonyl)-piperidino]-piperidin

Eine Mischung aus 24,9 g (0,1249 Mol) N-tert.Butyloxycarbonyl-4-piperidon, 19,3 ml (0,1249 Mol) Piperidino-4-carbonsäure-ethylester und 46,5 ml (0,1562 Mol) Titan(IV)-isopropylat wird während einer Stunde bei Raumtemperatur gerührt. Hiernach gibt man 170 ml wasserfreies Ethanol und dann 5,3 g (0,0837 Mol) Natriumcyanborhydrid zu und rührt weitere 20 Stunden lang. Danach versetzt man mit 34 ml Wasser, saugt den hierbei anfallenden anorganischen Festkörper ab und wäscht ihn mit Ethanol. Die vereinigten Filtrate werden zur Trockne eingeengt. Der verbleibende Rückstand wird in Essigester gelöst. Man filtriert von dem unlöslichen anorganischen Festkörper und engt das Filtrat unter Vakuum zur Trockne ein. Der Rückstand wird mittels Chromatographie über eine Kieselgelsäule gereinigt, wobei Essigester/Cyclohexan = 3:2 als Elutionsmittel verwendet wird.
Ausbeute: 32,9 g einer öligen Substanz (77,3 % der Theorie)
$R_f$-Wert: 0,36 (Kieselgel; Essigester)

b) 1-tert.Butyloxycarbonyl-4-[4-[[trans-4-[2-(methoxycarbonyl)-ethyl]-cyclohexyl]-aminocarbonyl]-piperidino]-piperidin

3,4 g (0,01 Mol) der unter a) hergestellten Verbindung werden über Nacht in 20 ml 1N Natronlauge gerührt. Nach dieser Zeit war vollständige Verseifung des Ethylesters eingetreten. Man setzt 20 ml 1N

Salzsäure zu und wäscht mit Essigester. Die wässrige Phase wird unter Vakuum zur Trockne eingeengt, der verbleibende Rückstand zweimal in Ethanol aufgenommen, jeweils unter Vakuum eingeengt und anschließend unter Vakuum bei 80 °C getrocknet. Der Rückstand wird in 100 ml Dimethylformamid gelöst. Diese Lösung versetzt man mit 2 ml (0,0102 Mol) Diphenyl-phosphorylchlorid, kühlt auf -5 °C ab, gibt unter Rühren 1,4 ml (0,0102 Mol) Triethylamin zu und rührt während einer Stunde bei -5 °C weiter. Anschließend gibt man 2,26 g (0,0102 Mol) trans-3-[4-Aminocyclohexyl]-propionsäuremethylester-hydrochlorid und 1,4 ml (0,0102 Mol) Triethylamin zu und rührt weitere 4 Stunden bei Raumtemperatur sowie anschließend eine Stunde lang bei 60 °C. Um die Umsetzung zu vervollständigen wurden erneut 1 ml Diphenyl-phosphorylchlorid und 1,4 ml Triethylamin zugegeben und über Nacht bei Raumtemperatur gerührt. Hiernach engt man unter Vakuum zur Trockne ein, nimmt den Rückstand in Essigester auf, wäscht die Essigester-Lösung zweimal mit gesättigter Natriumhydrogencarbonat-Lösung und einmal mit Wasser und trocknet über Natriumsulfat. Nach Eindampfen unter Vakuum verbleiben 4 g eines Rückstandes, welcher durch Säulenchromatographie über Kieselgel gereinigt wird, wobei Dichlormethan/Methanol = 20:1 und 10:1 als Elutionsmittel dient. Der Rückstand wird mit Ether/Petrolether verrieben, abgesaugt und getrocknet.
Ausbeute: 2,98 g (62,1 % der Theorie),
Schmelzpunkt: 182-184 °C

Beispiel XX

4-[4-[[trans-4-[2-(Methoxycarbonyl)-ethyl]-cyclohexyl]-aminocarbonyl]-phenyl]-1-benzyl-piperazin

a) 1-Benzyl-4-(4-cyano-phenyl)-Piperazin

Eine Mischung von 26 g (0,2147 Mol) 4-Fluorbenzonitril, 37,3 ml (0,2147 Mol) N-Benzylpiperazin und 36,7 ml (0,2147 Mol) N-Ethyl-diisopropylamin wird während 8 Stunden auf 140 °C erhitzt. Nach Abkühlen rührt man in Wasser ein und extrahiert mit Dichlormethan. Die vereinigten Dichlormethan-Phasen werden über Natriumsulfat getrocknet und unter Vakuum eingedampft. Der verbleibende Rückstand wird aus Ether/Petrolether kristallisiert.
Ausbeute: 29,8 g (50,1 % der Theorie),
Schmelzpunkt: 106-108 °C

b) 1-Benzyl-4-(4-carboxy-phenyl)-piperazin

29,8 g (0,1074 Mol) 1-Benzyl-4-(4-cyano-phenyl)-piperazin werden in 200 ml Ethylenglykol gelöst. Nach Zusatz von 48 g (0,8592 Mol) Kaliumhydroxid erhitzt man während 8 Stunden auf Rückfluß-Temperatur. Anschließend wird unter Vakuum das Ethylenglykol weitgehend abdestilliert und das verbleibende Öl mit Wasser verdünnt. Nach Ansäuern mit Essigsäure wird der ausgeschiedene Festkörper abgesaugt, mit Wasser und anschließend mit wenig Aceton gewaschen und getrocknet.
Ausbeute: 31,4 g (98,7 % der Theorie),
Schmelzpunkt: 225-227 °C (Zers.)

c) 4-[4-[[trans-4-[2-(Methoxycarbonyl)-ethyl]-cyclohexyl]-aminocarbonyl]-phenyl]-1-benzyl-piperazin

Zu einer Lösung von 1,5 g (0,051 Mol) 1-Benzyl-4-(4-carboxyphenyl)-piperazin in 80 ml Dimethylformamid gibt man bei -5 °C und unter Rühren 1 ml (0,0051 Mol) Diphenylphosphinsäurechlorid und 0,7 ml (0,051 Mol) Triethylamin und rührt eine weitere Stunde bei -5 °C. Hiernach gibt man 1,13 g (0,051 Mol) 3-(4-trans-Aminocyclohexyl)-propionsäuremethylester-hydrochlorid und 0,7 ml (0,051 Mol) Triethylamin zu und rührt über Nacht bei Raumtemperatur. Anschließend engt man unter Vakuum zur Trockne ein und reinigt den Rückstand durch Chromatographie über eine Kieselgelsäule, wobei Dichlormethan, das 2,5 % Methanol enthält, als Elutionsmittel dient. Der nach Abdampfen des Elutionsmittels verbleibende Rückstand wird mit Petrolether verrieben und abgesaugt.
Ausbeute: 1,04 g (44 % der Theorie),
Schmelzpunkt: 188-189 °C

Beispiel XXI

4-[2-[trans-4-(2-Methoxycarbonyl-ethyl)-cyclohexyl]-1-oxo-2,3-dihydro-isoindol-6-yl]-pyridin

a) 2-Methyl-5-trifluormethylsulfonyloxy-benzoesäure-methylester

Zu einer Lösung von 10 g (60 mMol) 3-Hydroxy-6-methyl-benzoesäure-methylester in 40 ml trockenem Pyridin tropft man unter Rühren bei -8 bis 4°C 18,2 ml (66 mMol) Trifluormethansulfonsäureanhydrid innerhalb 35 Minuten. Nach beendeter Zugabe rührt man über Nacht im Eisbad und gießt anschließend auf Wasser. Man extrahiert mit tert.Butyl-methylether, wäscht die vereinigten Extrakte mit verdünnter Salzsäure und anschließend mit Wasser, trocknet über Natriumsulfat und engt unter Vakuum zur Trockne ein. Das verbleibende orangerote Öl wird mittels Säulenchromatographie über Kieselgel, wobei Cyclohexan/Essigester = 9:1 als Elutionsmittel dient, gereinigt. Farbloses Öl.
Ausbeute: 15,3 g (85,5 % der Theorie),
$R_f$-Wert: 0,60 (Kieselgel; Essigester/Cyclohexan = 9:1)

b) 2-Brommethyl-5-trifluormethylsulfonyloxy-benzoesäuremethylester

Eine Mischung von 8,3 g (27,8 mMol) der unter a) hergestellten Verbindung, 5,3 g (30 mMol) N-Bromsuccinimid und 20 mg 2,2'-Azaisobutyronitril in 100 ml Tetrachlorkohlenstoff wird während einer Stunde mit einer 300 W UV-Tauchlampe bestrahlt. Hiernach wird abgekühlt, vom ungelösten Succinimid abfiltriert und das Filtrat unter Vakuum zur Trockne eingeengt. Das Verbleibende wird mittels Säulenchromatographie über Kieselgel, wobei Cyclohexan/Essigester = 9:1 als Elutionsmittel dient, gereinigt.
Farbloses Öl.
Ausbeute: 7,7 g (73,3 % der Theorie),
$R_f$-Wert: 0,55 (Kieselgel; Essigester/Cyclohexan = 9:1)

c) 2-[trans-4-(2-Methoxycarbonyl-ethyl)-cyclohexyl]-1-oxo-2,3-dihydro-6-trifluormethylsulfonyloxy-isoindol

Zu einer Lösung von 2,2 g (10 mMol) trans-4-Aminocyclohexylpropionsäure-methylester-hydrochlorid und 3,9 g = 5,1 ml (30 mMol) N,N-Diisopropyl-ethylamin in 100 ml trockenem Dimethylformamid gibt man unter Rühren bei Raumtemperatur eine Lösung von 3,8 g (10 mMol) der unter b) gewonnenen Verbindung in 10 ml Dimethylformamid und rührt während 16 Stunden bei Raumtemperatur weiter. Anschließend engt man unter Vakuum ein und verteilt den Rückstand zwischen Wasser und Dichlormethan. Die organische Phase wird abgetrennt, über Natriumsulfat getrocknet und unter Vakuum zur Trockne eingeengt. Der feste Rückstand wird mit Petrolether verrieben und abgesaugt.
Ausbeute: 1,9 g (42,2 % der Theorie),
Schmelzpunkt: 104-105°C

d) 4-[2-[trans-4-(2-Methoxycarbonyl-ethyl)-cyclohexyl]-1-oxo-2,3-dihydro-isoindol-6-yl]-pyridin

Eine Lösung aus 2,3 g (5,1 mMol) der unter c) gewonnenen Verbindung, 1 g (8 mMol) 4-Pyridylboronsäure, 0,58 g (0,5 mMol) Tetrakis-triphenylphosphin-palladium(o) und 3,3 ml (24 mMol) Triethylamin in 35 ml Dimethylformamid wird während 8 Stunden auf 100°C erhitzt. Nach dem Abkühlen gibt man auf 150 ml Wasser, saugt den Niederschlag ab und wäscht ihn mit Wasser. Nach dem Trocknen unter Vakuum bei 40°C reinigt man ihn mittels Säulenchromatographie über Kieselgel, wobei Dichlormethan/Methanol = 19:1 als Elutionsmittel dient.
Ausbeute: 1,2 g (62,2 % der Theorie),
Schmelzpunkt: 210-211°C

Beispiel XXII

1-Benzyl-4-[2-[trans-4-[2-(methoxycarbonyl)-ethyl]-cyclohexyl]-1-oxo-2,3-dihydro-isoindol-6-yl]-3,4-dehydro-piperidin

a) 1-Benzyl-4-[2-[trans-4-[2-(methoxycarbonyl-)ethyl]-cyclohexyl]-1-oxo-2,3-dihydro-isoindol-6-yl]-pyridinium-bromid

Eine Mischung aus 0,32 g (0,84 mMol) 4-[2-[trans-4-(2-(Methoxycarbonyl)-ethyl)-cyclohexyl]-1-oxo-2,3-dihydro-isoindolpyridin, 0,2 ml (1,68 mMol) Benzylbromid und 3 ml Acetonitril wird während einer halben Stunde auf Rückfluß-Temperatur erhitzt, wobei ein Festkörper ausfällt. Nach Abkühlen und Verdünnen mit tert.Butyl-methylether saugt man ab und trocknet den Festkörper bei 40 °C unter Vakuum.
Ausbeute: 0,4 g (86,7 % der Theorie),
Schmelzpunkt: 252-255 °C

b) 1-Benzyl-4-[2-[trans-4-[2-(methoxycarbonyl)-ethyl]-cyclohexyl]-1-oxo-2,3-dihydro-isoindol-6-yl]-3,4-dehydropiperidin

360 mg (0,66 mMol) der unter a) gewonnenen Verbindung werden in 10 ml Ethanol suspendiert. Unter Rühren gibt man bei Raumtemperatur 38 mg (1 mMol) Natriumborhydrid in 2 Portionen zu. Man rührt weitere 2 Tage bei Raumtemperatur und versetzt dann mit Wasser, extrahiert mit Essigester, trocknet die vereinigten Essigester-Extrakte über Natriumsulfat und dampft unter Vakuum zur Trockne ein. Die verbleibenden Kristalle werden bei 60 °C unter Vakuum getrocknet.
Ausbeute: 270 mg (86,6 % der Theorie),
Schmelzpunkt: 142-143 °C

Beispiel XXIII

1-tert.Butyloxycarbonyl-3-[4-[[trans-4-[2-(methoxycarbonyl)-ethyl]-cyclohexyl]-aminocarbonyl]-phenyl]-pyrrolidin

a) 4-Methoxycarbonyl-trans-zimtsäure

124,9 g (1,2 Mol) Malonsäure werden in 180 ml Pyridin gelöst. 164,2 g (1 Mol) 4-Carbomethoxy-benzaldehyd und 8,52 g (0.1 Mol) Piperidin werden zu dieser Lösung gegeben und die so erhaltene Suspension wird 1 1/2 Stunden lang auf 100 °C erhitzt. Nach dem Abkühlen auf Zimmertemperatur wird die Suspension in 800 ml Eiswasser gegossen und mit 200 ml konzentrierter Salzsäure angesäuert. Der erhaltene Niederschlag wird abgesaugt, sorgfältig mit Wasser gewaschen, getrocknet, mit Aceton verrieben, wieder abgesaugt und getrocknet.
Ausbeute: 185,0 g (89,72 der Theorie),
Schmelzpunkt: 239-241 °C

b) 4-Methoxycarbonyl-trans-zimtsäure-methylester

Eine Mischung aus 184,8 g (0,896 Mol) 4-Methoxycarbonyltrans-zimtsäure, 10 ml Schwefelsäure und 3 l Methanol wird 18 Stunden lang unter Rückfluß erhitzt. Nach dem Kühlen in einem Eisbad werden die erhaltenen Kristalle abgesaugt, mit Methanol gewaschen und getrocknet.
Ausbeute: 175.9 g (89.2% der Theorie),
Schmelzpunkt: 121-123 °C

c) 3-(4-Methoxycarbonyl-phenyl)-3-(nitro-methyl)-propionsäure-methylester

22 g (0,1 Mol) 4-Methoxycarbonyl-trans-zimtsäure-methylester werden in 59 g (0,966 Mol) = 52 ml Nitromethan suspendiert und 2,4 g (0,021 Mol) = 2,6 ml 1,1,3,3-Tetramethylguanidin hinzugegeben. Die erhaltene Suspension wird 5 Stunden lang auf 70 °C erhitzt, anschließend abgekühlt und im Vakuum zur Trockene eingeengt. Der erhaltene Rückstand wird zwischen Essigester und 2 N Salzsäure verteilt. Die erhaltene organische Phase wird mit Wasser gewaschen, getrocknet und im Vakuum zur Trockene eingeengt. Der erhaltene braune ölige Rückstand wird über Kieselgel mit Cyclohexan/Essigester (7:3 bis

3:2) chromatographiert.
Ausbeute: 20,8 g eines gelblichen Öls (74% der Theorie),
$R_f$-Wert: 0,52 (Kieselgel; Cyclohexan/Essigester = 3:2)

d) 3-(4-Methoxycarbonyl-phenyl)-pyrrolidin-5-on

20,3 g (0,72 Mol) 3-(4-Methoxycarbonyl-phenyl)-3-(nitromethyl)-propionsäure werden in 200 ml Methanol in Gegenwart von 2 g Raney-Nickel 7 Stunden lang bei einer Temperatur von 60 °C bei einem Druck von 3 bar hydriert. Anschließend wird vom Katalysator abfiltriert und das erhaltene Filtrat im Vakuum zur Trockene eingeengt. Der erhaltene gelbe Rückstand wird in Dichlormethan verrieben, der so erhaltene Niederschlag abfiltriert, mit Dichlormethan gewaschen und getrocknet.
Ausbeute: 11,2 g (70,9% der Theorie),
Schmelzpunkt: 154-156 °C

e) 3-(4-Methoxycarbonyl-phenyl)-pyrrolidin-hydrochlorid

16,5 g (274 mMol) = 15,7 ml Eisessig werden langsam zu eine Lösung von 6 g (27 mMol) 3-(4-Methoxycarbonyl-phenyl)-pyrrolidin-5-on und 10,4 g (274 mMol) Natriumborhydrid in 120 ml Dioxan unter Rühren und bei einer Temperatur von 10 bis 15 °C gegeben. Die so erhaltene Reaktionsmischung wird 7 Stunden lang unter Rückfluß erhitzt. Nach Rühren über Nacht bei Raumtemperatur werden erneut 5,2 g Natriumborhydrid und 7,9 ml Eisessig in 30 ml Dioxan hinzugegeben. Die erhaltene Lösung wird 6 Stunden lang unter Rückfluß erhitzt, anschließend auf ein kleines Volumen eingeengt, mit 500 ml Wasser versetzt und mit Dichlormethan extrahiert. Die erhaltene organische Phase wird mit Wasser gewaschen, getrocknet und auf ein kleines Volumen eingeengt. Die erhaltene Lösung wird mit Chlorwasserstoff behandelt und zur Trockene eingeengt.
Ausbeute: 6.2 g farbloses Öl (93.9% der Theorie),
$R_f$-Wert: 0,63 (Reversed Phase Plate RP8; Methanol/5% Kochsalzlösung = 6:4)

f) 1-tert.Butyloxycarbonyl-3-(4-methoxycarbonyl-phenyl)-pyrrolidin

9,0 g (41,17 mMol) di-tert.Butyldicarbonat werden zu einer Mischung aus 6,2 g (25,6 mMol) 3-(4-Methoxycarbonyl-phenyl)-pyrrolidin-hydrochlorid, gelöst in einer Mischung aus 40 ml Dioxan, 20 ml Wasser und 14,3 ml Triethylamin, unter Eiskühlung gegeben. Nach Rühren über Nacht wird die erhaltene Lösung zur Trockene eingeengt. Das erhaltene Öl wird zwischen Dichlormethan und Wasser verteilt. Die organische Phase wird mit Wasser gewaschen, getrocknet und zur Trockene eingeengt. Die so erhaltene ölige Substanz wird über Kieselgel mit Cyclohexan/Essigester (4:1) chromatographiert.
Ausbeute: 4,8 g gelbliches Öl (61,5% der Theorie)
$R_f$-Wert: 0,62 (Kieselgel; Cyclohexan/Essigester = 3:2)

g) 1-tert.Butyloxycarbonyl-3-(4-carboxy-phenyl)-pyrrolidin

Eine Mischung aus 4,7 g (15,4 mMol) 1-tert.Butyloxycarbonyl-3-(4-methoxycarbonyl-phenyl)-pyrrolidin, 15,4 ml 2 N Natronlauge, 100 ml Tetrahydrofuran und 3,3 ml Wasser wird 24 Stunden lang bei Raumtemperatur gerührt. Anschließend werden erneut 15,4 ml 2 N Natronlauge zugegeben und die Lösung 7 Stunden lang auf 50 °C erhitzt. Nach dem Abkühlen auf Raumtemperatur werden 31 ml 2 N Salzsäure und wässrige Zitronensäure bis zu einem pH-Wert von 3 zugegeben. Die erhaltene Lösung wird auf ein kleines Volumen eingeengt, die so erhaltenen Kristalle werden abgesaugt, mit Wasser gewaschen und getrocknet.
Ausbeute: 3,8 g (84,4% der Theorie),
Schmelzpunkt: 145-147 °C

h) 1-tert.Butyloxycarbonyl-3-[4-[[trans-4-[2-(methoxycarbonyl)-ethyl]-cyclohexyl]-aminocarbonyl]-phenyl]-pyrrolidin

Eine Mischung aus 3,75 g (12,87 mMol) of 1-tert.Butyloxycarbonyl-3-(4-carboxy-phenyl)-pyrrolidin, 3,1 g (13,86 mMol) trans-(4-Amino-cyclohexyl)-propionsäure-methylester, 7,9 g (77,67 mMol) = 10,8 ml Triethylamin und 4,45 g (13,86 mMol) 2-[(1H)-Benzotriazol-1-yl]-1,1,3,3-tetramethyluronium-tetrafluorboratin 100 ml absolutem Dimethylformamid wird 2 Stunden bei Raumtemperatur gerührt. Nach dem Verdünnen der

erhaltenen Suspension mit 250 ml Wasser wird der erhaltene farblose Niederschlag abfiltriert, mit Wasser gewaschen und getrocknet.
Ausbeute: 5.75 g (100% der Theorie),
Schmelzpunkt: 99-103 °C

Beispiel XXIV

1-tert.Butyloxycarbonyl-3-[4-[[trans-4-[2-(methoxycarbonyl)-ethyl]-cyclohexyl]-aminocarbonyl]-phenyl]-3-methyl-pyrrolidin

a) 2-(4-Methoxycarbonyl-phenyl)-propionsäurenitril

1,3 g Natriumhydrid (55-62%ig in Öl) werden zu einer Lösung von 5,3 g (30,25 mMol) 4-Methoxycarbonyl-phenyl-acetonitril in 50 ml Dimethylformamid unter Eiskühlung gegeben. Nach 10 Minuten werden 4,3 g (30,25 mMol) = 1,9 ml Methyliodid unter Eiskühlung tropfenweise zugegeben. Die erhaltene Suspension wird 1 1/2 Stunden lang gerührt, mit Wasser verdünnt und auf ein kleines Volumen eingeengt. Die erhaltene Lösung wird erneut mit Wasser verdünnt, mit Zitronensäure angesäuert und mit Essigester extrahiert. Die vereinigten organischen Phasen werden mit Wasser gewaschen, getrocknet und im Vakuum zur Trockene eingeengt. Die erhaltene ölige Substanz wird über Kieselgel mit Cyclohexan/Essigester (17:3) chromatographiert.
Ausbeute: 32 g gelbliches Öl (47,1% der Theorie),
$R_f$-Wert: 0,5 (Kieselgel, Cyclohexan/Essigester = 7:3)

b) 3-Cyano-3-(4-methoxycarbonyl-phenyl)-buttersäure-methylester

0,72 g Natriumhydrid (55-65%ig in Öl) werden zu einer Lösung von 3,1 g (16,38 mMol) 2-(4-Methoxycarbonyl-phenyl)-propionsäurenitril in 50 ml Dimethylformamid unter Eiskühlung gegeben. Nach 15 Minuten werden, ebenfalls unter Eiskühlung, 2,5 g (16,4 mMol) = 1,55 ml Bromessigsäure-methylester tropfenweise hinzugegeben. Die erhaltene Suspension wird 2 1/2 Stunden lang gerührt, mit Wasser verdünnt und auf ein kleines Volumen eingeengt. Die erhaltene Lösung wird erneut mit Wasser verdünnt, mit Zitronensäure angesäuert und mit Essigester extrahiert. Die vereinigten organischen Phasen werden mit Wasser gewaschen, getrocknet und im Vakuum zur Trockene eingeengt. Die erhaltene ölige Substanz wird über Kieselgel mit Cyclohexan/Essigester (7:3) chromatographiert.
Ausbeute: 3.6 g (83,7% der Theorie),
Schmelzpunkt: 75-77 °C

c) 3-(4-Methoxycarbonyl-phenyl)-3-methyl-pyrrolidin-5-on

Eine Lösung von 3,3 g (12,6 mMol) 3-Cyano-3-(4-methoxycarbonyl-phenyl)-buttersäure-methylester in 100 ml Methanol wird mit 12 ml methanolischer Salzsäure angesäuert und in Gegenwart von 1 g Palladium auf Tierkohle (10%ig) bei einem Druck von 3 bar 6 Stunden lang bei Raumtemperatur hydriert. Nach Abfiltrieren des Katalysators wird das Filtrat im Vakuum zur Trockene eingeengt. Der erhaltene feste Rückstand wird in Essigester gelöst und die organische Phase mit Wasser extrahiert. Die wässrige Phase wird durch Zugabe einer wässrigen Kaliumcarbonat-Lösung alkalisch gestellt und mit Essigester extrahiert. Die organische Phase wird getrocknet und im Vakuum zur Trockene eingeengt. Der erhaltene Rückstand wird mit Essigester verrieben, abfiltriert, mit Essigester gewaschen und getrocknet.
Ausbeute: 2,2 g (75,9% der Theorie),
Schmelzpunkt: 188-189 °C

d) 3-(4-Methoxycarbonyl-phenyl)-3-methyl-pyrrolidin-hydrochlorid

5,6 g (92,5 mMol) = 5,3 ml Eisessig werden langsam zu einer Lösung von 2,15 g (9,22 mMol) 3-(4-Methoxycarbonyl-phenyl)-3-methyl-pyrrolidin-5-on und 3,5 g (92,5 mMol) Natriumborhydrid in 60 ml Dioxan unter Rühren und bei Temperaturen zwischen 10 und 15 °C gegeben. Die so erhaltene Mischung wird 6 Stunden lang unter Rückfluß erhitzt. Nach Rühren über Nacht bei Raumtemperatur werden erneut 1,8 g Natriumborhydrid und dann 2,7 ml Eisessig in 10 ml Dioxan hinzugegeben. Nach 7 stündigem Erhitzen unter Rückfluß wird die erhaltene Lösung auf ein kleines Volumen eingeengt, mit 300 ml Wasser versetzt und mit Dichlormethan extrahiert. Die organische Phase wird mit Wasser gewaschen, getrocknet und auf ein

kleines Volumen eingeengt. Die so erhaltene Lösung wird mit Chlorwasserstoff behandelt und zur Trockene eingeengt.
Ausbeute: 1,14 g farbloses Öl (48,6% der Theorie),
$R_f$-Wert: 0,5 (Reversed Phase Plate RP8; Methanol/5%ige Kochsalzlösung = 6:7)

e) 1-tert.Butyloxycarbonyl-3-(4-methoxycarbonyl-phenyl)-3-methyl-pyrrolidin

1,6 g (7,15 mMol) di-tert.Butyldicarbonat werden zu einer Lösung von 1,14 g (4,46 mMol) 3-(4-Methoxycarbonylphenyl)-3-methyl-pyrrolidin-hydrochlorid in einer Mischung aus 8 ml Dioxan, 4 ml Wasser und 2,5 ml Triethylamin unter Eiskühlung gegeben. Nach 2-stündigem Rühren wird die Lösung zur Trockene eingeengt und der erhaltene ölige Rückstand zwischen Dichlormethan und Wasser verteilt. Die organische Phase wird mit Wasser gewaschen, getrocknet und zur Trockne eingeengt.
Ausbeute: 1,18 g farbloses Öl (83,12% der Theorie),
$R_f$-Wert: 0,62 (Kieselgel; Cyclohexan/Essigester = 3:2)

f) 1-tert.Butyloxycarbonyl-3-(4-carboxyphenyl)-3-methylpyrrolidin

Eine Mischung aus 1,1 g (3,44 mMol) 1-tert.Butylosycarbonyl-3-(4-methoxycarbonyl-phenyl)-3-methyl-pyrrolidin, 7 ml 2 N Natronlauge, 20 ml Tetrahydrofuran und 1 ml Wasser wird 3 Tage lang gerührt. Anschließend wird mit 7 ml 2 N Salzsäure und Zitronensäure auf einen pH-Wert von 2 eingestellt. Die erhaltene Lösung wird auf ein kleines Volumen eingeengt und mit Dichlormethan extrahiert. Die organische Phase wird mit Wasser gewaschen, getrocknet und zur Trockene eingeengt. Die erhaltene ölige Substanz wird über Kieselgel mit Cyclohexan/Essigester (4:1 bis 1:1) chromatographiert.
Ausbeute: 600 mg gelbliches Öl (51,1% der Theorie),
$R_f$-Wert: 0,5 (Kieselgel; Cyclohexan/Essigester = 3:2)

g) 1-tert.Butyloxycarbonyl-3-[4[[trans-4-[2-(methoxycarbonyl)-ethyl]-cyclohexyl]-aminocarbonyl]-phenyl]-1-methylpyrrolidin

Eine Mischung aus 0,57 g (1,87 mMol) 1-tert.Butyloxycarbonyl-3-(4-carboxy-phenyl)-3-methyl-pyrrolidin, 0,445 g (2,01 mMol) trans-(4-Amino-cyclohexyl)-propionsäure-methylester, 1,14 g (0,113 mMol) = 1,56 ml Triethylamin und 0,645 g (2 mMol) 2-[(1H)-Benzotriazol-1-yl]-1,1,3,3-tetramethyluronium-tetra luorborat in 15 ml absolutem Dimethylformamid wird 2 Stunden lang bei Raumtemperatur gerührt. Die erhaltene Suspension wird mit Wasser verdünnt und zur Trockene eingeengt. Der erhaltene Rückstand wird zwischen Essigester und einer verdünnten Kaliumcarbonat-Lösung verteilt. Die erhaltene organische Phase wird mit Wasser gewaschen, getrocknet und zur Trockene eingeengt. Das verbleibende Öl wird über Kieselgel mit Cyclohexan/Essigester (4:1) chromatographiert.
Ausbeute: 300 mg farbloses Öl (37,5% der Theorie),
$R_f$-Wert: 0,31 (Kieselgel; Cyclohexan/Essigester = 3:2)

Herstellung der Endverbindungen:

Beispiel 1

1-tert.Butyloxycarbonyl-4-[4-[[trans-4-[2-(methoxycarbonyl)-ethyl]-cyclohexyl]-aminocarbonyl]-phenyl]-piperidin

Eine Lösung von 0,76 g 1-tert.Butyloxycarbonyl-4-(4-carboxyphenyl)-piperidin, 0,90 g 2-[(1H)-Benzotriazol-1-yl]-1,1,3,3-tetramethyluronium-tetrafluorborat, 0,55 g 3-(trans-4-Aminocyclohexyl)-propionsäure-methylesterhydrochlorid, 0,38 g 1-Hydroxy-(1H)-benzotriazol und 0,67 g N-Methyl-morpholin in 50 ml Dimethylformamid wird 16 Stunden bei Raumtemperatur gerührt. Das Lösungsmittel wird unter vermindertem Druck entfernt. Zum Rückstand gibt man 200 ml Wasser und extrahiert die wäßrige Phase mit Essigester. Die organische Phase wird über Natriumsulfat getrocknet, filtriert und das Lösungsmittel unter vermindertem Druck entfernt. Man erhält 1,8 g eines Feststoffs, welcher mit Methylenchlorid/Methanol (9:1) über Kieselgel chromatographiert wird.
Ausbeute: 1,15 g (98 % der Theorie),
Schmelzpunkt: Sinterung ab 169 °C
$R_f$-Wert: 0,60 (Kieselgel; Methylenchlorid/Methanol = 9:1)

Analog werden folgende Verbindungen erhalten:

(1) 1-tert.Butyloxycarbonyl-4-[4-[[4-[2-(Methoxycarbonyl)-ethyl]-phenyl]-aminocarbonyl]phenyl]piperidin
Schmelzpunkt: 157-162 ° C
$R_f$-Wert: 0,53 (Kieselgel; Cyclohexan/Essigester = 1:1)
(2) 4-[4-[[4-[2-(n-Butansulfonylamino)-2-(methoxycarbonyl)-ethyl]-phenyl]-aminocarbonyl]-phenyl]-1-tert.butyloxycarbonylpiperidin
$R_f$-Wert: 0,41 (Kieselgel; Cyclohexan/Essigester = 1:1)
Massenspektrum: $(M+Na)^+$ = 624
(3) 4-[4-[[trans-4-[2-(Methoxycarbonyl)-ethyl]-cyclohexyl]-aminocarbonyl]-phenyl]-chinuclidin
(4) 4-[4-[[trans-4-[2-(Methoxycarbonyl)-ethyl]-cyclohexyl]-aminocarbonyl]-phenyl]-pyridin
(5) 4-[4-[[4-[2-(Methoxycarbonyl)-ethyl]-phenyl]-aminocarbonyl]-piperidino]-pyridin
(6) 4-[4-[[trans-4-[2-(Methoxycarbonyl)-ethyl]-cyclohexyl]-aminocarbonyl]-piperidino]-pyridin
(7) 4-[4-[[4-[2-(n-Butansulfonylamino)-2-(methoxycarbonyl)-ethyl]-phenyl]-aminocarbonyl]-piperidino]-pyridin
(8) 4-[4-[[4-[2-(n-Hexanoylamino)-2-(methoxycarbonyl)-ethyl]-phenyl]-aminocarbonyl]-piperidino]-pyridin
(9) 4-[4-[[trans-4-[2-(n-Butansulfonylamino)-2-(methoxycarbonyl)-ethyl]-cyclohexyl]-aminocarbonyl]-piperidino]-pyridin
(10) 4-[4-[[trans-4-[2-(n-Hexanoylamino)-2-(methoxycarbonyl)-ethyl]-cyclohexyl]-aminocarbonyl]-piperidino]-pyridin
(11) 1-tert.Butyloxycarbonyl-4-[4-[[trans-4-(methoxycarbonyl)-cyclohexyl]-aminocarbonyl]-phenyl]-piperidin
Schmelzpunkt: 197-200 ° C
$R_f$-Wert: 0,20 (Kieselgel; Methylenchlorid/Methanol = 20:1)
(12) 4-[4-[[4-(tert.Butyloxycarbonyl-methyloxy)-phenyl]-carbonylamino]-phenyl]-1-trifluoracetyl-piperidin
Hergestellt aus 4-[4-Amino-phenyl]-1-trifluoracetyl-piperidin und 4-[tert.Butylosycarbonyl-methyloxy]-benzoesäure
Schmelzpunkt: 177-178 ° C

## Beispiel 2

### 4-[4-[[trans-4-[2-(Methoxycarbonyl)-ethyl]-cyclohexyl]-aminocarbonyl]-phenyl]-piperidin-hydrochlorid

Ein Gemisch aus 1,1 g 1-tert.Butyloxycarbonyl-4-[4-[[trans-4-[2-(methoxycarbonyl)-ethyl]-cyclohexyl]-aminocarbonyl]-pheny l]-piperidin, 40 ml Dioxan und 40 ml etherischer Salzsäure wird 5 Stunden bei Raumtemperatur gerührt, wobei sich ein Öl abscheidet. Das Lösungsmittelgemisch wird abdekantiert und das verbleibende Öl mit Ether verrieben. Der entstandene Feststoff wird abgenutscht und getrocknet.
Ausbeute: 0,87 g (91 % der Theorie),
Schmelzpunkt: 225-232 ° C
$R_f$-Wert: 0,53 (Kieselgel; Methylenchlorid/Methanol/konz. Ammoniak = 4:1:0,25)
Massenspektrum: $M^+$ = 372

Analog werden folgende Verbindungen erhalten:

(1) 4-[4-[[4-[2-(Methoxycarbonyl)-ethyl]-phenyl]-aminocarbonyl]-phenyl]-piperidin-hydrochlorid
Schmelzpunkt: 222-225 ° C
$R_f$-Wert: 0,54 (Kieselgel; Methylenchlorid/Methanol/konz. Ammoniak = 4:1:0,25)
Massenspektrum: $M^+$ = 366
(2) 4-[4-[[4-[2-(n-Butansulfonylamino)-2-(methoxycarbonyl)-ethyl]-phenyl]-aminocarbonyl]-phenyl]-piperidin-hydrochlorid
Schmelzpunkt: sintert ab 70 ° C
$R_f$-Wert: 0,63 (Kieselgel; Methylenchlorid/Methanol/konz. Ammoniak = 4:1:0,25)
Massenspektrum: $(M+H)^+$ = 502
(3) 4-[4-[[trans-4-[2-Acetamino-2-(methoxycarbonyl)-ethyl]-cyclohexyl]-aminocarbonyl]-phenyl]-piperidin-hydrochlorid
(4) 4-[4-[[trans-4-[2-(Methoxycarbonyl)-ethyl]-cyclohexyl]-oxymethyl]-phenyl]-piperidin-hydrochlorid
(5) 4-[4-[[trans-4-[2-(Methoxycarbonyl)ethyl]-cyclohexyl]-methyloxy]-phenyl]-piperidin-hydrochlorid

(6) 4-[4-[[trans-4-[2-(n-Butansulfonylamino)-2(methoxycarbonyl)-ethyl]-cyclohexyl]-aminocarbonyl]-phenyl]-piperidin hydrochlorid
(7) 4-[4-[[trans-4-[2-(Methansulfonylamino]-2-(methoxycarbonyl)-ethyl]-cyclohexyl]-aminocarbonyl]-phenyl]-piperidinhydrochlorid
(8) 4-[4-[[trans-4-[2-(n-Hexanoylamino)-2-(methoxycarbonyl)-ethyl]-cyclohexyl]-aminocarbonyl]-phenyl]-piperidinhydrochlorid
(9) 4-[4-[[trans-4-[2-(Methoxycarbonyl)-propyl]-cyclohexyl]-aminocarbonyl]-phenyl]-piperidin-hydrochlorid
(10) 4-[4-[N-[trans-4-[2-(Methoxycarbonyl)-ethyl]-cyclohexyl]-N-methyl-aminocarbonyl]-phenyl]-piperidin-hydrochlorid
(11) 4-[4-[[trans-4-(2-(Methoxycarbonyl)-ethyl]-cyclohexyl]-carbonylamino]-phenyl]-piperidin-hydrochlorid
(12) 4-[4-[[trans-4-[3-(Methoxycarbonyl)-propyl]-cyclohexyl]-aminocarbonyl]-phenyl]-piperidin-hydrochlorid
(13) 4-[4-[[trans-4-(Methoxycarbonylmethyl)-cyclohexyl]-aminocarbonyl]-phenyl]-piperidin-hydrochlorid
Schmelzpunkt: >250 ° C
$R_f$-Wert: 0,4 (Reversed Phase Platte RP8; Methanol/5%ige Natriumchloridlösung = 6:4)
(14) 4-[4-[[4-[3-(Methoxycarbonyl)-propyl]-piperidino]-carbonyl]-phenyl]-piperidin-hydrochlorid
(15) 4-[4-[[4-[4-(Methoxycarbonyl)-butyl]-piperidino]-carbonyl]-phenyl]-piperidin-hydrochlorid
(16) 4-[4-[[4-[3-(Methoxycarbonyl)-propyl]-piperazino]-carbonyl]-phenyl]-piperidin-dihydrochlorid
(17) 4-[4-[[1-[2-(Methoxycarbonyl)-ethyl]-4-piperidinyl]-aminocarbonyl]-phenyl]-piperidin-dihydrochlorid
(18) 4-[4-[[4-[3-(Methoxycarbonyl)-propyl]-3-oxo-piperazino]-carbonyl]-phenyl]-piperidin-hydrochlorid
(19) 1-[4-[[trans-4-[2-(Methoxycarbonyl)-ethyl]-cyclohexyl]-aminocarbonyl]-phenyl]-piperazin-hydrochlorid
Schmelzpunkt: 190-192 ° C
(20) 4-[4-[[trans-4-[2-(Methoxycarbonyl)-ethyl]-cyclohexyl]-aminocarbonyl]-phenyl]-3,4-dehydro-piperidin-hydrochlorid
(21) 4-[4-[[trans-4-[2-(Methoxycarbonyl)-n-octyl]-cyclohexyl]-aminocarbonyl]-phenyl]-piperidin-hydrochlorid
(22) 4-[4-[[trans-4-[2-(Benzylsulfonylamino)-2-(methoxycarbonyl)-ethyl]-cyclohexyl]-aminocarbonyl]-phenyl]-piperidinhydrochlorid
(23) 4-[4-[[trans-4-[2-(Methoxycarbonyl)-ethyl]-cyclohexyl]-aminocarbonyl]-phenyl]-4-methyl-piperidin-hydrochlorid
(24) 4-Cyano-4-[4-[[trans-4-[2-(methoxycarbonyl)-ethyl]-cyclohexyl]-aminocarbonyl]-phenyl]-piperidin-hydrochlorid
(25) 4-Aminocarbonyl-4-[4-[[trans-4-[2-(methoxycarbonyl)-ethyl]-cyclohexyl]-aminocarbonyl]-phenyl]-piperidin-hydrochlorid
(26) 4-(Methoxycarbonyl)-4-[4-[[trans-4-[2-(methoxycarbonyl)-ethyl]-cyclohexyl]-aminocarbonyl]-phenyl]-piperidin-hydrochlorid
(27) 3-[4-[[trans-4-[2-(Methoxycarbonyl)-ethyl]-cyclohexyl]-aminocarbonyl]-phenyl]-n-propylamin-hydrochlorid
(28) 5-[4-[[trans-4-[2-(Methoxycarbonyl)-ethyl]-cyclohexyl]-aminocarbonyl]-phenyl]-n-pentylamin-hydrochlorid
(29) 5-[4-[[trans-4-[2-(n-Butansulfonylamino)-2-(methoxycarbonyl)-ethyl]-cyclohexyl]-aminocarbonyl]-phenyl]-n-pentylamin-hydrochlorid
(30) 5-[4-[[trans-4-[2-(n-Hexanoylamino)-2-(methoxycarbonyl)-ethyl]-cyclohexyl]-aminocarbonyl]-phenyl]-n-pentylamin-hydrochlorid
(31) 1-[5-[[trans-4-[2-(Methoxycarbonyl)-ethyl]-cyclohexyl]-aminocarbonyl]-pyridin-2-yl]-piperazin-dihydrochlorid
(32) 4-[4-[[trans-4-[2-(Methoxycarbonyl)-ethyl]-cyclohexyl]-aminocarbonyl]-3-methyl-phenyl]-piperidin-hydrochlorid
(33) 4-[3-Methoxy-4-[[trans-4-[2-(methoxycarbonyl)-ethyl]-cyclohexyl]-aminocarbonyl]-phenyl]-piperidin-hydrochlorid
(34) 4-[4-[[trans-4-[2-Amino-2-(methoxycarbonyl)-ethyl]-cyclohexyl]-aminocarbonyl]-phenyl]-piperidin-dihydrochlorid
(35) 4-[4-[[cis-4-[2-(Methoxycarbonyl)-ethyl]-cyclohexyl]-aminocarbonyl]-phenyl]-piperidin-hydrochlorid
(36) 4-[4-[[cis-4-[2-(n-Butansulfonylamino)-2-(methoxycarbonyl)-ethyl]-cyclohexyl]-aminocarbonyl]-phenyl]-piperidinhydrochlorid
(37) 4-[4-[[4-[2-(n-Hexanoylamino)-2-(methoxycarbonyl)-ethyl]-phenyl]-aminocarbonyl]-phenyl]-piperidin-hydrochlorid
(38) 4-[4-[[trans-4-(Methoxycarbonyl-methoxy)-cyclohexyl]-aminocarbonyl]-phenyl]-piperidin-hydrochlorid

(39) 4-[3-[[4-[2-(Benzylsulfonylamino)-2-(methoxycarbonyl)-ethyl]-phenyl]-aminocarbonyl]-phenyl]-piperidin-hydrochlorid
(40) 4-[3-[[4-[2-(n-Hexanoylamino)-2-(methoxycarbonyl)-ethyl)-phenyl]-aminocarbonyl]-phenyl]-piperidin-hydrochlorid
(41) 4-[4-[[4-[2-(Benzylsulfonylamino)-2-(methoxycarbonyl)-ethyl]-phenyl]-aminocarbonyl]-phenyl]-piperidin-hydrochlorid
(42) 4-(3-Fluor-4-[[trans-4-[2-(methoxycarbonyl)-ethyl]-cyclohexyl]-aminocarbonyl]-phenyl]-piperidin-hydrochlorid
(43) 4-(3-Ethoxy-4-[[trans-4-[2-(methoxycarbonyl)-ethyl]-cyclohexyl]-aminocarbonyl]-phenyl]-piperidin-hydrochlorid
(44) 1-(2-Brom-4-[[trans-4-[2-(methoxycarbonyl)-ethyl]-cyclohexyl]-aminocarbonyl]-phenyl]-piperazin-hydrochlorid
(45) 4-[3-Chlor-4-[[trans-4-[2-(methoxycarbonyl)-ethyl]-cyclohexyl]-aminocarbonyl]-phenyl]-piperidin-hydrochlorid
(46) 4-[4-[[2-Brom-4-[2-(methoxycarbonyl)-ethyl]-phenyl]-aminocarbonyl]-phenyl]-piperidin-hydrochlorid
(47) 4-[4-[[2-Methoxy-4-[2-(methoxycarbonyl)-ethyl]-phenyl]-aminocarbonyl]-phenyl]-piperidin-hydrochlorid
(48) 4-[4-[[trans-4-[2-(Methoxycarbonyl)-ethyl]-cyclohexyl]-aminocarbonyl]-2-methylthio-phenyl]-piperazin-hydrochlorid
(49) 1-[4-[[trans-4-[2-(Methoxycarbonyl)-ethyl]-cyclohexyl]-aminocarbonyl]-2-methylsulfonyl-phenyl]-piperazin-hydrochlorid
(50) 4-[4-[[4-[2-(Methoxycarbonyl)-ethyl]-2-methyl-phenyl]-aminocarbonyl]-phenyl]-piperidin-hydrochlorid
(51) 4-[4-[[4-[2-(Methoxycarbonyl)-ethyl]-phenyl]-sulfonyl-amino]-phenyl]-piperidin-hydrochlorid
(52) 4-[4-[[4-[2-(Methoxycarbonyl)-ethenyl]-phenyl]-aminocarbonyl]-phenyl]-piperidin-hydrochlorid
(53) 4-[4-[[trans-4-(Methoxycarbonyl)-cyclohexyl]-aminocarbonyl]-phenyl]-piperidin-hydrochlorid
Schmelzpunkt: 260-265 ° C
$R_f$-Wert: 0,39 (Kieselgel; Methylenchlorid/Methanol/konz. Ammoniak = 4:1:0,25)
(54) 4-[4-[[4-[2-(Methoxycarbonyl)-ethyl]-piperidino]-methyl]phenyl]-piperidin-dihydrochlorid
Ausbeute: 0,44 g (88 % der Theorie), Harz;
$R_f$-Wert: 0,65 (Reversed Phase Platte RP8; Methanol/5%ige Natriumchlorid-Lösung = 6:4)
$^1$H-NMR-Spektrum (200 MHz, DMSO-$d_6$); Signale bei ppm: 1,2-1,6 (m, 4H); 1,6-2,05 (m, 6+1H); 2,25-2,4 (t, 2H); 2,8-3,1 (m, 4 +1H); 3,3-3,55 (t, 4H); 3,6 (s, 3H); 4,2 (d, 2H); 7,3 (d, 2H); 7,5 (d, 2H); 8,4-8,9 (m, 2H); 9,9 (s, 1H)
(55) 1-[4-[[4-(Methoxycarbonyl)-methoxy]-phenyl]-carbonylamino]-phenyl]-piperazin-hydrochlorid
(56) 4-[4-[[4-[2-(Methoxycarbonyl)-ethenyl]-phenyl]-carbonylamino]-phenyl]-piperazin-hydrochlorid
(57) 4-[4-[[4-[2-(Methoxycarbonyl)-ethyl]-phenyl]-carbonylamino]-phenyl]-piperidin-hydrochlorid
(58) 4-[4-[[trans-4-(Methoxycarbonyl)-cyclohexyl]-methylaminocarbonyl]-phenyl]-piperidin-hydrochlorid
Schmelzpunkt: >250 ° C
$R_f$-Wert: 0,43 (Reversed Phase Platte RP8; Methanol/5%ige Natriumchlorid-Lösung = 6:4)
(59) 4-[4-[[trans-4-(Methoxycarbonyl)-cyclohexyl]-methylaminocarbonyl]-phenyl]-piperidin-hydrochlorid
$R_f$-Wert: 0,43 (Reversed Phase Platte RP8; Methanol/5%ige Natriumchloridlösung = 6:4)
(60) 4-[4-[[trans-4-(Methoxycarbonyl)-cyclohexyl]-aminocarbonylamino]-phenyl]-piperidin-hydrochlorid
(61) 4-[4-[[trans-4-(Methoxycarbonyl-methoxy)-cyclohexyl]-aminocarbonyl]-phenyl]-piperidin-hydrochlorid
$R_f$-Wert: 0,6 (Reversed Phase Platte RP8; Methanol/5%ige Natriumchloridlösung = 6:4)
(62) 4-[4-[[4-[2-(Methoxycarbonyl)-ethyl]-piperidinyl]-carbonyl]-phenyl]-piperidin-hydrochlorid Anstelle von Salzsäure wurde Trifluoressigsäure verwendet.
Schmelzpunkt: 177-179 ° C (Zers.)
(63) 4-[4-[[trans-4-[2-(Methoxycarbonyl)-ethyl]-cyclohexyl]-aminocarbonyl]-piperidino]-piperidin-dihydrochlorid Anstelle von Salzsäure wurde Trifluoressigsäure verwendet.
Ausbeute: 1,98 g (75,4 % der Theorie),
Schmelzpunkt: 302-303 ° C (Zers.)

Beispiel 3

4-[4-[[trans-4-(2-Carboxy-ethyl)-cyclohexyl]-aminocarbonyl]-phenyl]-piperidin-hydrochlorid

0,41 g 4-[4-[[trans-4-[2-(Methoxycarbonyl)-ethyl]-cyclohexyl]-aminocarbonyl]-phenyl]-piperidin-hydrochlorid werden mit 80 ml 6N Salzsäure 4 Stunden bei Raumtemperatur gerührt. Der gebildete Niederschlag

wird abfiltriert und mit Wasser gewaschen.
Ausbeute: 0,33 g (84 % der Theorie),
Schmelzpunkt: 280-285 ° C
$R_f$-Wert: 0,07 (Kieselgel; Methylenchlorid/Methanol/konz. Ammoniak = 4:1:0,25)
Massenspektrum: $M^+$ = 358

Analog werden folgende Verbindungen erhalten:

(1) 4-[4-[[4-(2-Carboxy-ethyl)-phenyl]-aminocarbonyl]-phenyl]-piperidin-hydrochlorid
Schmelzpunkt: 290-293 ° C
$R_f$-Wert: 0,11 (Kieselgel; Methylenchlorid/Methanol/konz. Ammoniak = 4:1:0,25)
Massenspektrum: $M^+$ = 352
(2) 4-[4-[[4-(2-Carboxy-ethyl)-phenyl]-aminocarbonyl]-phenyl]-1-methyl-piperidin-hydrochlorid
Man verseift mit Lithiumhydroxid in einem 5:4-Gemisch aus Tetrahydrofuran und Wasser, versetzt mit 1N Salzsäure und dampft das Tetrahydrofuran im Vakuum ab.
Schmelzpunkt: über 300 ° C
$R_f$-Wert: 0,13 (Kieselgel; Methylenchlorid/Methanol/konz. Ammoniak = 4:1:0,25)
Massenspektrum: $M^+$ = 366
(3) 4-[4-[[4-[2-(n-Butansulfonyl-amino)-2-carboxy-ethyl]-phenyl]-aminocarbonyl]-phenyl]-piperidin-hydrochlorid
Schmelzpunkt: 130 ° C (sintert ab 120 ° C)
$R_f$-Wert: 0,15 (Kieselgel; Methylenchlorid/Methanol/konz. Ammoniak = 4:1:0,25)
Massenspektrum: (M-H) = 486
(4) 4-[3-[[trans-4-(2-Carboxy-ethyl)-cyclohexyl]-aminocarbonyl]-phenyl]-piperidin-hydrochlorid
Man verwendet ein 1:1:0,5-Gemisch aus Wasser, konz. Salzsäure und Eisessig.
$R_f$-Wert: 0,60 (Reversed Phase Platte RP8; Methanol/5%ige Natriumchloridlösung = 6:4)
Massenspektrum: $M^+$ = 358
(5) 4-[3-[[4-(2-Carboxy-ethyl)-phenyl]-aminocarbonyl]-phenyl]-piperidin-hydrochlorid
Man verwendet ein 1:1:1-Gemisch aus Wasser, konz. Salzsäure und Eisessig.
$R_f$-Wert: 0,43 (Reversed Phase Platte RP8; Methanol/5%ige Natriumchloridlösung = 6:4)

| | | | | | | |
|---|---|---|---|---|---|---|
| Ber. x HCl x 1,3 $H_2O$<br>Gef. | C | 60,47<br>60,25 | H | 6,67<br>6,77 | N | 6,71<br>6,65 |

(6) 4-[3-[[4-[2-(n-Butansulfonylamino)-2-carboxy-ethyl]-phenyl]-aminocarbonyl]-phenyl]-piperidin-hydrochlorid
Man verfährt wie unter (4).
Schmelzpunkt: über 200 ° C
$R_f$-Wert: 0,60 (Reversed Phase Platte RP8; Methanol/5%ige Natriumchloridlösung = 6:4)
Massenspektrum: $(M+H)^+$ = 488
(7) 4-[4-[[trans-4-(2-Acetamino-2-carboxy-ethyl)-cyclohexyl]-aminocarbonyl]-phenyl]-piperidin-hydrochlorid
(8) 4-[4-[[trans-4-(2-Carboxy-ethyl)-cyclohexyl]-aminocarbonyl]-phenyl]-1-isopropyl-piperidin-hydrochlorid
(9) 4-[4-[[trans-4-(2-Carboxy-ethyl)-cyclohexyl]-oxymethyl]-phenyl]-piperidin-hydrochlorid
(10) 4-[4-[[trans-4-(2-Carboxy-ethyl)-cyclohexyl]-methyloxy]-phenyl]-piperidin-hydrochlorid
(11) 4-[4-[[trans-4-[2-(n-Butansulfonylamino)-2-carboxyethyl]-cyclohexyl]-aminocarbonyl]-phenyl]-piperidin-hydrochlorid
(12) 4-[4-[[trans-4-[2-Carboxy-2-(methansulfonyl-amino)-ethyl]-cyclohexyl]-aminocarbonyl]-phenyl]-piperidin-hydrochlorid
(13) 4-[4-[[trans-4-[2-Carboxy-2-(n-hexanoylamino)-ethyl]-cyclohexyl]-aminocarbonyl]-phenyl]-piperidin-hydrochlorid
(14) 4-[4-[[trans-4-(2-Carboxy-propyl)-cyclohexyl]-aminocarbonyl]-phenyl]-piperidin-hydrochlorid
(15) 4-[4-[N-[trans-4-(2-Carboxy-ethyl)-cyclohexyl]-N-methylaminocarbonyl]-phenyl]-piperidin-hydrochlorid Schmelzpunkt: 245-247 ° C
(16) 4-[4-[[trans-4-(2-Carboxy-ethyl)-cyclohexyl]-carbonylamino]-phenyl]-piperidin-hydrochlorid
(17) 4-[4-[[trans-4-(3-Carboxy-propyl)-cyclohexyl]-aminocarbonyl]-phenyl]-piperidin-hydrochlorid

(18) 4-[4-[(trans-4-Carboxymethyl-cyclohexyl)-aminocarbonyl]-phenyl]-piperidin-hydrochlorid
Schmelzpunkt: >250 °C
$R_f$-Wert: 0,5 (Reversed Phase Platte RP8; Methanol/5%ige Natriumchloridlösung = 6:4)
(19) 4-[4-[[4-(3-Carboxy-propyl)-piperidino]-carbonyl]-phenyl]-piperidin-hydrochlorid
(20) 4-[4-[[4-(4-Carboxy-butyl)-piperidino]-carbonyl]-phenyl]-piperidin-hydrochlorid
(21) 4-[4-[[4-(3-Carboxy-propyl)-piperazino]-carbonyl]-phenyl]-piperidin-dihydrochlorid
(22) 4-[4-[[1-(2-Carboxy-ethyl)-4-piperidinyl]-aminocarbonyl]-phenyl]-piperidin-dihydrochlorid
(23) 4-[4-[[4-(3-Carboxy-propyl)-3-oxo-piperazino]-carbonyl]-phenyl]-piperidin-hydrochlorid
(24) 1-[4-[[trans-4-(2-Carboxy-ethyl)-cyclohexyl]-aminocarbonyl]-phenyl]-piperazin-hydrochlorid
Schmelzpunkt: 300-302 °C (Zers.)
(25) 4-[4-[[trans-4-(2-Carboxy-ethyl)-cyclohexyl]-aminocarbonyl]-phenyl]-chinuclidin-hydrochlorid
(26) 4-[4-[[trans-4-(2-Carboxy-ethyl)-cyclohexyl]-aminocarbonyl]-phenyl]-pyridin-hydrochlorid
(27) 4-[4-[[trans-4-(2-Carboxy-ethyl)-cyclohexyl]-aminocarbonyl]-phenyl]-3,4-dehydro-piperidin-hydrochlorid
(28) 4-[4-[[trans-4-(2-Carboxy-n-octyl)-cyclohexyl]-aminocarbonyl]-phenyl]-piperidin-hydrochlorid
(29) 4-[4-[[trans-4-(2-Benzylsulfonylamino)-2-carboxyethyl]-cyclohexyl]-aminocarbonyl]-phenyl]-piperidin-hydrochlorid
(30) 4-[4-[[trans-4-(2-Carboxy-ethyl)-cyclohexyl]-aminocarbonyl]-phenyl]-4-methyl-piperidin-hydrochlorid
(31) 4-[4-[[trans-4-(2-Carboxy-ethyl)-cyclohexyl]-aminocarbonyl]-phenyl]-4-cyano-piperidin-hydrochlorid
(32) 4-Aminocarbonyl-4-[4-[[trans-4-(2-carboxy-ethyl)-cyclohexyl]-aminocarbonyl]-phenyl]-piperidin-hydrochlorid
(33) 4-Carboxy-4-[4-[[trans-4-(2-carboxy-ethyl)-cyclohexyl]-aminocarbonyl]-phenyl]-piperidin-hydrochlorid
(34) 3-[4-[[trans-4-(2-Carboxy-ethyl)-cyclohexyl]-aminocarbonyl]-phenyl]-n-propylamin-hydrochlorid
(35) 5-[4-[[trans-4-(2-Carboxy-ethyl)-cyclohexyl]-aminocarbonyl]-phenyl]-n-pentylamin-hydrochlorid
(36) 5-[4-[[trans-4-[2-(n-Butansulfonylamino)-2-carboxyethyl]-cyclohexyl]-aminocarbonyl]-phenyl]-n-pentylamin-hydrochlorid
(37) 5-[4-[[trans-4-[2-Carboxy-2-(n-hexanoylamino)-ethyl]-cyclohexyl]-aminocarbonyl]-phenyl]-n-pentylamin-hydrochlorid
(38) 1-[5-[[trans-4-(2-Carboxy-ethyl)-cyclohexyl]-aminocarbonyl]-pyridin-2-yl]-piperazin-dihydrochlorid
(39) 4-[4-[[trans-4-(2-Carboxy-ethyl)-cyclohexyl]-aminocarbonyl]-3-methyl-phenyl]-piperidin-hydrochlorid
(40) 4-[4-[[trans-4-(2-Carboxy-ethyl)-cyclohexyl]-aminocarbonyl]-3-methoxy-phenyl]-piperidin-hydrochlorid
Man verfährt wie unter (2)
(41) 4-[4-[[trans-4-(2-Amino-2-carboxy-ethyl)-cyclohexyl]-aminocarbonyl]-phenyl]-piperidin-dihydrochlorid
(42) 4-[4-[[cis-4-(2-Carboxy-ethyl)-cyclohexyl]-aminocarbonyl]-phenyl]-piperidin-hydrochlorid
(43) 4-[4-[[cis-4-[2-(n-Butansulfonylamino)-2-carboxy-ethyl]-cyclohexyl]-aminocarbonyl]-phenyl]-piperidin-hydrochlorid
(44) 4-[4-[[4-[2-Carboxy-2-(hexanoylamino)-ethyl]-phenyl]-aminocarbonyl]-phenyl]-piperidin-hydrochlorid
(45) 4-[4-[[trans-4-(Carboxy-methoxy)-cyclohexyl]-aminocarbonyl]-phenyl]-piperidin-hydrochlorid
(46) 4-[4-[[4-(2-Carboxy-ethyl)-phenyl]-aminocarbonyl]-piperidino]-pyridin-hydrochlorid
(47) 4-[4-[[trans-4-(2-Carboxy-ethyl)-cyclohexyl]-aminocarbonyl]-piperidino]-pyridin-hydrochlorid
(48) 4-[4-[[4-[2-(n-Butansulfonylamino)-2-carboxy-ethyl]-phenyl]-aminocarbonyl]-piperidino]-pyridin-hydrochlorid
(49) 4-[4-[[4-[2-Carboxy-2-(n-hexanoylamino)-ethyl]-phenyl]-aminocarbonyl]-piperidino]-pyridin-hydrochlorid
(50) 4-[4-[[trans-4-[2-(n-Butansulfonylamino)-2-carboxyethyl]-cyclohexyl]-aminocarbonyl]-piperidino]-pyridin-hydrochlorid
(51) 4-[4-[[trans-4-[2-Carboxy-2-(n-hexanoylamino)-ethyl]-cyclohexyl]-aminocarbonyl]-piperidino]-pyridin-hydrochlorid
(52) 4-[3-[[4-[2-(Benzylsulfonylamino)-2-carboxy-ethyl]-phenyl]-aminocarbonyl]-phenyl]-piperidin-hydrochlorid
(53) 4-[3-[[4-[2-Carboxy-2-(n-hexanoylamino)-ethyl]-phenyl]-aminocarbonyl]-phenyl]-piperidin-hydrochlorid
(54) 4-[4-[[4-[2-(Benzylsulfonylamino)-2-carboxy-ethyl]-phenyl]-aminocarbonyl]-phenyl]-piperidin-hydrochlorid
(55) 4-[4-[[trans-4-(2-Carboxy-ethyl)-cyclohexyl]-aminocarbonyl]-3-fluor-phenyl]-piperidin-hydrochlorid
(56) 4-[4-[[trans-4-(2-Carboxy-ethyl)-cyclohexyl]-aminocarbonyl]-3-ethoxy-phenyl]-piperidin-hydrochlorid
Man verfährt wie unter (2)
(57) 1-[2-Brom-4-[[trans-4-(2-carboxy-ethyl)-cyclohexyl]-aminocarbonyl]-phenyl]-piperazin-hydrochlorid

(58) 4-[4-[[trans-4-(2-Carboxy-ethyl)-cyclohexyl]-aminocarbonyl]-3-chlor-phenyl]-piperidin-hydrochlorid
(59) 4-[4-[[2-Brom-4-(2-carboxy-ethyl)-phenyl]-aminocarbonyl]-phenyl]-piperidin-hydrochlorid
(60) 4-[4-[[4-(2-Carboxy-ethyl)-2-methoxy-phenyl]-aminocarbonyl]-phenyl]-piperidin-hydrochlorid
(61) 1-[4-[[trans-4-(2-Carboxy-ethyl)-cyclohexyl]-aminocarbonyl]-2-methylthio-phenyl]-piperazin-hydrochlorid
(62) 1-[4-[[trans-4-(2-Carboxy-ethyl)-cyclohexyl]-aminocarbonyl]-2-methylsulfonyl-phenyl]-piperazin-hydrochlorid
(63) 4-[4-[[4-(2-Carboxy-ethyl)-2-methyl-phenyl]-aminocarbonyl]-phenyl]-piperidin-hydrochlorid
(64) 4-[4-[[4-(2-Carboxy-ethyl)-phenyl]-sulfonylamino]-phenyl]-piperidin-hydrochlorid
(65) 4-[4-[[4-(2-Carboxy-ethenyl)-phenyl]-aminocarbonyl]-phenyl]-piperidin-hydrochlorid
(66) 4-[4-[[trans-4-(2-Carboxy-ethyl)-cyclohexyl]-aminocarbonyl]-phenyl]-1-methyl-piperidin-hydrochlorid
Schmelzpunkt: >300 ° C
Massenspektrum: $M^+$ = 372
(67) 4-[1-[[trans-4-(2-Carboxy-ethyl)-cyclohexyl]-aminocarbonyl]-piperidino]-piperidin-hydrochlorid
(68) 4-[4-[[trans-4-(2-Carboxy-ethyl)-cyclohexyl]-aminocarbonyl]-piperidino]-piperidin-dihydrochlorid
Ausbeute: 0,77 g (79,5 % der Theorie),
Schmelzpunkt: 297-300 ° C (Zers.)
(69) 1-[4-[[4-(Carboxy-methoxy)-phenyl]-carbonylamino]-phenyl]-piperazin-hydrochlorid
(70) 4-[4-[[4-(2-Carboxy-ethenyl)-phenyl]-carbonylamino]-phenyl]-piperidin-hydrochlorid
(71) 4-[4-[[4-(2-Carboxy-ethyl)-phenyl]-carbonylamino]-phenyl]-piperidin-hydrochlorid
(72) 4-[4-[[trans-4-(Carboxy)-cyclohexyl]-methyl-aminocarbonyl]-phenyl]-piperidin-hydrochlorid
Schmelzpunkt: 109-110 ° C
(73) 4-[2-[4-(2-carboxy-ethyl)-phenyl]-1-ozo-2,3-dihydroisoindol-6-yl]-piperidin-hydrochlorid
Schmelzpunkt: 291-293 ° C
$R_f$-Wert: 0,52 (Reversed Phase Platte RP8; Methanol/5%ige Natriumchloridlösung = 6:4)
(74) 2-[4-[[trans-4-(2-Carboxy-ethyl)-cyclohexyl]-aminocarbonyl]-phenyl]-ethylamin-hydrochlorid
Schmelzpunkt: >250 ° C
$R_f$-Wert: 0,65 (Reversed Phase Platte RP8; Methanol/5%ige Natriumchloridlösung = 6:4)
(75) 4-[4-[[trans-4-(Carboxy-methoxy)-cyclohexyl]-aminocarbonyl]-phenyl]-piperidin-hydrochlorid
Schmelzpunkt: >250 ° C
$R_f$-Wert: 0,65 (Reversed Phase Platte RP8; Methanol/5%ige Natriumchloridlösung = 6:4)
(76) 4-[4-[[trans-4-Carboxy-cyclohexyl]-aminocarbonyaminol]-phenyl]-piperidin-hydrochlorid
Schmelzpunkt: >200 ° C
$R_f$-Wert: 0,70 (Reversed Phase Platte RP8; Methanol/5%ige Natriumchloridlösung = 6:4)
(77) 4-[4-[[4-(2-Carboxy-ethyl)-phenyl]-aminomethyl]-phenyl]-piperidin-hydrochlorid
(78) 4-[4-[[4-(2-Carboxy-ethyl)-piperidinyl]-carbonyl]-phenyl]-piperidin-hydrochlorid
Schmelzpunkt: 207-208 ° C (Zers.)
(79) 4-[2-[trans-4-(2-Carboxy-ethyl)-cyclohexyl]-1-oxo-2,3-dihydro-isoindol-6-yl]-piperidin-hydrochlorid
$R_f$-Wert: 0,50 (Reversed Phase Platte RP8; Methanol/5%ige Natriumchloridlösung = 6:4)
(80) 4-[2-[trans-4-(2-Carboxy-ethyl)-cyclohexyl]-1-oxo-2,3-dihydro-isoindol-5-yl]-piperidin-hydrochlorid
$R_f$-Wert: 0,50 (Reversed Phase Platte RP8; Methanol/5%ige Natriumchloridlösung = 6:4)
Schmelzpunkt: >300 ° C
(81) 4-[2-[4-(2-Carboxy-ethyl)-phenyl]-1-oxo-2,3-dihydro-isoindol-5-yl]-piperidin-hydrochlorid
Schmelzpunkt: 303-306 ° C
(82) 4-[4-[[trans-4-(2-Carboxy-ethyl)-cyclohexyl]-aminocarbonyl]-phenyl]-1-aza-bicyclo[2.2.1]heptan-hydrochlorid
(83) 4-[4-[[trans-4-(Carboxy)-cyclohexyl]-methylaminocarbonyl]-piperidin-hydrochlorid
$R_f$-Wert: 0,56 (Reversed Phase Platte RP8; Methanol/5%ige Natriumchloridlösung = 6:4)

Beispiel 4

4-[4-[[4-[2-(Methoxycarbonyl)-ethyl]-phenyl]-aminocarbonyl]-phenyl]-1-methyl-piperidin-hydrochlorid

0,8 g 4-[4-[[4-[2-(Methoxycarbonyl)-ethyl]-phenyl]-aminocarbonyl]-phenyl]-piperidin-hydrochlorid werden in 35 ml Methanol gelost, mit 0,42 g Paraformaldehyd und 0,45 g Natriumcyanborhydrid versetzt und 3 Stunden bei Raumtemperatur gerührt, wobei man den pH-Wert durch Zusatz von 1N Salzsäure zwischen 3 und 6 hält. Danach werden weitere 0,5 g Paraformaldehyd und 0,5 g Natriumcyanborhydrid zugesetzt und 16 Stunden bei Raumtemperatur unter Beibehaltung der oben erwähnten Acidität der Lösung gerührt. Man

stellt mit 10N Natronlauge alkalisch, extrahiert mit Essigester, dampft die organische Phase ein und reinigt durch Chromatographie an Kieselgel (Elutionsmittel: Methylenchlorid/Methanol = 9:1).
Ausbeute: 0,58 g (76 % der Theorie),
Schmelzpunkt: 161-164 °C
$R_f$-Wert: 0,17 (Kieselgel; Methylenchlorid/Methanol = 9:1) Massenspektrum: $M^+$ = 380

Analog wird folgende Verbindung erhalten:

(1) 1-Isopropyl-4-[4-[[trans-4-[2-(methoxycarbonyl)-ethyl]-cyclohexyl]-aminocarbonyl]-phenyl]-piperidin
(2) 4-[4-[[trans-4-[2-(Methoxycarbonyl)-ethyl]-cyclohexyl]-aminocarbonyl]-phenyl]-1-methyl-piperidin
Schmelzpunkt: 202-204 °C
(3) 4-[2-[trans-4-(2-Carboxy-ethyl)-cyclohexyl]-1-oxo-2,3-dihydro-isoindol-5-yl]-1-methyl-piperidin-hydrochlorid
Schmelzpunkt: 301-303 °C
Hergestellt aus 4-[2-[trans-4-(2-Carboxy-ethyl)-cyclohexyl]-1-oxo-2,3-dihydro-isoindol-5-yl]-piperidin mit Paraformaldehyd und Ameisensäure.
(4) 3-[4-[[trans-4-(2-Carboxy-ethyl)-cyclohexyl]-aminocarbonyl]-phenyl]-1-methyl-pyrrolidin-hydrochlorid
Schmelzpunkt: 246-250 °C
Hergestellt aus 3-[4-[[trans-4-(2-Carboxy-ethyl)-cyclohexyl]-aminocarbonyl]-phenyl]-pyrrolidin-hydrochlorid mit Paraformaldehyd und Ameisensäure.

Beispiel 5

4-[3-[[trans-4-[2-(Methoxycarbonyl)-ethyl]-cyclohexyl]-aminocarbonyl]-phenyl]-piperidin-hydrochlorid

1,7 g 1-Benzyl-4-[3-[[trans-4-[2-(methoxycarbonyl)-ethyl]-cyclohexyl]-aminocarbonyl]-phenyl]-3,4-dehydro-piperidin werden in 40 ml Methanol gelöst, mit 2 ml methanolischer Salzsäure und 0,5 g Palladiumhydroxid auf Kohle versetzt und bei 50 °C mit Wasserstoff von 5 bar Druck hydriert. Anschließend filtriert man vom Katalysator ab und dampft im Vakuum ein.
Ausbeute: 1,30 g (87 % der Theorie),
$R_f$-Wert: 0,25 (Reversed Phase Platte RP8; Methanol/5%ige Natriumchloridlösung = 6:4)
Massenspektrum: $M^+$ = 372

Analog werden folgende Verbindungen erhalten:

(1) 4-[3-[[4-[2-(Methoxycarbonyl)-ethyl]-phenyl]-aminocarbonyl]-phenyl]-piperidin
Man arbeitet ohne Salzsäurezusatz mit 10%iger Palladiumkohle.
$R_f$-Wert: 0,27 (Reversed Phase Platte RP8; Methanol/5%ige Natriumchloridlösung = 6:4)
(2) 4-[3-[[4-[2-(n-Butansulfonylamino)-2-(methoxycarbonyl)-ethyl]-phenyl]-aminocarbonyl]-phenyl]-piperidin-hydrochlorid
Man verfährt wie unter (1) aber bei 60 °C.
$R_f$-Wert: 0,28 (Reversed Phase Platte RP8; Methanol/5%ige Natriumchloridlösung = 6:4)
Massenspektrum: $(M+H)^+$ = 502
(3) 1-[4-[[trans-4-[2-(Methoxycarbonyl)-ethyl]-cyclohexyl]-aminocarbonyl]-phenyl]-piperazin-hydrochlorid
Hergestellt durch Debenzylierung von 4-[4-[[trans-4-[2-(Methoxycarbonyl)-ethyl]-cyclohexyl]-aminocarbonyl]-phenyl]-1-benzyl-piperazin in Gegenwart von 10%iger Palladiumkohle.
Schmelzpunkt: 190-192 °C
(4) 4-[2-[4-[2-(Methoxycarbonyl)-ethyl]-phenyl]-1-oxo-2,3-dihydro-isoindol-6-yl]-piperidin-hydrochlorid
(5) 4-[2-[trans-4-[2-(Methoxycarbonyl)-ethyl]-cyclohexyl]-1-oxo-2,3-dihydro-isoindol-6-yl]-piperidin-hydrochlorid
Schmelzpunkt: 175-178 °C
Hergestellt aus 1-Benzyl-4-[2-[trans-4-[2-(methoxycarbonyl)-ethyl]-cyclohexyl]-1-oxo-2,3-dihydro-isoindol-6-yl]-3,4-dehydro-piperidinund Palladiumhydroxid auf Kohle als Katalysator.
(6) 4-[2-[trans-4-[2-(Methoxycarbonyl)-ethyl]-cyclohexyl]-1-oxo-2,3-dihydro-isoindol-5-yl]-piperidin-hydrochlorid
Schmelzpunkt: 260-262 °C
(7) 4-[2-[4-[2-(Methoxycarbonyl)-ethyl]-phenyl]-1-oxo-2,3-dihydro-isoindol-5-yl]-piperidin-hydrochlorid
Schmelzpunkt: 232-235 °C

Beispiel 6

1-Benzyl-4-[3-[[trans-4-[2-(methoxycarbonyl)-ethyl]-cyclohexyl]-aminocarbonyl]-phenyl]-3,4-dehydro-piperidin

2 g 1-Benzyl-4-(3-carboxy-phenyl)-3,4-dehydro-piperidin-hydrochlorid werden in 3,5 ml Thionylchlorid 45 Minuten zum Rückfluß erhitzt und anschließend im Vakuum zur Trockne eingedampft. Das so erhaltene Säurechlorid wird portionsweise zu einer auf 0°C abgekühlten Lösung von 1,3 g 3-(trans-4-Amino-cyclohexyl)-propionsäure-methylester-hydrochlorid und 3,3 ml N-Ethyl-diisopropylamin in 50 ml Methylenchlorid gegeben. Nach 2 Stunden fügt man 300 ml Methylenchlorid und 15 ml Methanol zu, wäscht nacheinander mit Wasser, 0,1N Natronlauge, Wasser, 0,1N Salzsäure und Wasser und dampft die organische Phase ein. Das Produkt wird säulenchromatographisch über Kieselgel gereinigt (Elutionsmittel: Methylenchlorid/Methanol = 9:1)
Ausbeute: 1,8 g (64 % der Theorie),
$R_f$-Wert: 0,42 (Kieselgel; Methylenchlorid/Methanol = 9:1)

Analog werden folgende Verbindungen erhalten:

(1) 1-Benzyl-4-[3-[[4-[2-(methoxycarbonyl)-ethyl]-phenyl]-aminocarbonyl]-phenyl]-3,4-dehydro-piperidin
Schmelzpunkt: 194-196°C
$R_f$-Wert: 0,66 (Kieselgel; Methylenchlorid/Methanol = 9:1)
(2) 1-Benzyl-4-[3-[[4-[2-(n-butansulfonylamino)-2-(methoxycarbonyl)-ethyl]-phenyl]-aminocarbonyl]-phenyl]-3,4-dehydropiperidin-hydrochlorid
$R_f$-Wert: 0,25 (Kieselgel; Methylenchlorid/Methanol = 20:1)

Beispiel 7

4-[4-[[4-(2-Carboxy-ethyl)-phenyl]-aminomethyl]-phenyl]-piperidin-hydrochlorid

0,7 g 4-[4-[[4-[2-(Methoxycarbonyl)-ethyl]-phenyl]-aminomethyl]-phenyl]-1-trifluoracetyl-piperidin, 10 ml Eisessig und 1 ml konz. Salzsäure werden 4 Stunden bei 80°C und 16 Stunden bei Raumtemperatur gerührt. Man dampft im Vakuum zur Trockne ein, dampft mehrmals mit Toluol nach und verrührt den Rückstand mit 50 ml Aceton.
Ausbeute: 0,39 g (66 % der Theorie),
$R_f$-Wert: 0,76 (Reversed Phase Platte RP8; Methanol/5%ige Natriumchloridlösung = 9:1)
Massenspektrum: $M^+$ = 338

Analog werden folgende Verbindungen erhalten:

(1) 4-[2-[trans-4-(2-Carboxy-ethyl)-cyclohexyl]-1-oxo-2,3-dihydro-isoindol-6-yl]-piperidin-hydrochlorid
(2) 4-[2-[4-[2-(n-Butansulfonylamino)-2-carboxy-ethyl]-phenyl]-1-oxo-2,3-dihydro-isoindol-6-yl]-piperidin-hydrochlorid
(3) 4-[4-[[trans-4-(2-Carboxy-ethyl)-cyclohexyl]-aminomethyl]-phenyl]-piperidin-dihydrochlorid
Schmelzpunkt: 308-311°C (Zers.)
(4) 4-[4-[N-[trans-4-(2-Carboxy-ethyl)-cyclohexyl]-N-methylaminomethyl]-phenyl]-piperidin-dihydrochlorid
(5) 4-[4-[N-[trans-4-(2-Carboxy-ethyl)-cyclohexyl]-N-(2-phenyl-ethyl)-aminomethyl]-phenyl]-piperidin-dihydrochlorid
(6) 4-[4-[[trans-4-(2-Carboxy-ethyl)-cyclohexyl]-aminosulfonyl]-phenyl]-piperidin-hydrochlorid
(7) 4-[4-[2-[4-(2-Carboxy-ethyl)-phenyl]-ethenyl]-phenyl]-piperidin-hydrochlorid
(8) 4-[2-[trans-4-(2-Carboxy-ethyl)-cyclohexyl]-1-oxo-2,3-dihydro-isoindol-5-yl]-piperidin-hydrochlorid
(9) 4-[2-[4-(2-Carboxy-ethyl)-phenyl]-1-oxo-2,3-dihydro-isoindol-5-yl]-piperidin-hydrochlorid
(10) 4-[2-[4-[2-(n-Butansulfonylamino)-2-carboxy-ethyl]-phenyl]-1-oxo-2,3-dihydro-isoindol-5-yl]-piperidin-hydrochlorid
(11) 4-[2-[trans-4-(2-Carboxy-ethyl)-cyclohexyl]-1-oxo-3,4-dihydro-isochinolin-7-yl]-piperidin-hydrochlorid
(12) 4-[2-[4-(2-Carboxy-ethyl)-phenyl]-1-oxo-3,4-dihydro-isochinolin-7-yl]-piperidin-hydrochlorid
(13) 4-[2-[4-(2-Carboxy-ethyl)-phenyl]-1-oxo-2,3-dihydro-isoindol-6-yl]-piperidin-hydrochlorid
(14) 4-[4-[2-[4-(2-Carboxy-ethyl)-phenyl]-ethyl]-phenyl]-piperidin-hydrochlorid
(15) 4-[4-[[4-(2-Carboxy-ethyl)-phenyl]-methylamino]-phenyl]-piperidin-hydrochlorid

(16) 4-[4-[(trans-4-Carboxy-cyclohexyl)-aminocarbonyl]-phenyl]-piperidin-hydrochlorid
Schmelzpunkt: über 310 °C
$R_f$-Wert: 0,13 (Kieselgel; Methylenchlorid/Methanol/konz. Ammoniak = 4:1:0,25)
(17) 4-[4-[[4-(Carboxy-methyloxy)-phenyl]-carbonylamino]-phenyl]-piperidin-hydrochlorid
Hergestellt aus 4-[4-[[4-(tert.Butyloxycarbonyl-methyloxy)-phenyl]-carbonylamino]-phenyl]-1-trifluoracetyl-piperidin
$R_f$-Wert: 0,10 (Kieselgel; Methylenchlorid/Methanol = 9:1) Massenspektrum: $(M+H)^+$ = 355

Beispiel 8

4-[4-[[4-[2-(Methoxycarbonyl)-ethyl]-phenyl]-aminomethyl]-phenyl]-1-trifluoracetyl-piperidin

8,6 g 4-[4-[[4-[2-(Methoxycarbonyl)-ethyl]-phenyl]-iminomethyl]-phenyl]-1-trifluoracetyl-piperidin werden in 100 ml Essigester gelöst und in Gegenwart von 0,5 g Platindioxid zunächst 1,5 Stunden bei Raumtemperatur mit Wasserstoff von 5 bar Druck hydriert. Man fügt weitere 0,5 g Platindioxid zu und steigert die Temperatur auf 50 °C. Nach 3 Stunden werden nochmals 0,5 g Platindioxid zugesetzt und 4 Stunden weiter hydriert. Nach Abfiltrieren des Katalysators wird das Lösungsmittel entfernt und der Rückstand säulenchromatographisch über Kieselgel gereinigt (Elutionsmittel: Cyclohexan/Essigester = 3:1).
Ausbeute: 3,6 g (42 % der Theorie),
$R_f$-Wert: 0,50 (Kieselgel; Cyclohexan/Essigester = 2:1)

Beispiel 9

4-[4-[[trans-4-[2-(Isopropyloxycarbonyl)-ethyl]-cyclohexyl]-aminocarbonyl]-phenyl]-piperidin-hydrochlorid

0,15 g 4-[4-[[trans-4-(2-Carboxy-ethyl)-cyclohexyl]-aminocarbonyl]-phenyl]-piperidin-hydrochlorid werden in einem Gemisch aus 100 ml Isopropanol und 50 ml etherischer Salzsäure suspendiert und 16 Stunden bei Raumtemperatur gerührt. Das Produkt wird durch Eindampfen der Lösung im Vakuum erhalten.
Ausbeute: 0,135 g (81 % der Theorie),
Schmelzpunkt: 255-260 °C
$R_f$-Wert: 0,31 (Kieselgel; Methylenchlorid/Methanol/konz. Ammoniak = 4:1:0,25)
Massenspektrum: $M^+$ = 400

Analog werden folgende Verbindungen erhalten:

(1) 4-[4-[[trans-4-[2-(sec.Butyloxycarbonyl)-ethyl]-cyclohexyl]-aminocarbonyl]-phenyl]-piperidin-hydrochlorid
(2) 4-[4-[[trans-4-[2-(Cyclohexyloxycarbonyl)-ethyl]-cyclohexyl]-aminocarbonyl]-phenyl]-piperidin-hydrochlorid
Schmelzpunkt: 238-240 °C
(3) 4-[4-[[trans-4-[2-(Isobutyloxycarbonyl)-ethyl]-cyclohexyl]-aminocarbonyl]-phenyl]-piperidin-hydrochlorid
Schmelzpunkt: 238-240 °C
(4) 4-[4-[[trans-4-[2-[(2-Phenyl-ethyl)-oxycarbonyl]-ethyl]-cyclohexyl]-aminocarbonyl]-phenyl]-piperidin-hydrochlorid
(5) 4-[2-[4-[2-(Methoxycarbonyl)-ethyl]-phenyl]-1-oxo-2,3-dihydro-isoindol-6-yl]-piperidin-hydrochlorid
(6) 4-[2-[trans-4-[2-(Methoxycarbonyl)-ethyl]-cyclohexyl]-1-oxo-2,3-dihydro-isoindol-6-yl]-piperidin-hydrochlorid
(7) 4-[2-[4-[2-(n-Butansulfonylamino)-2-(methoxycarbonyl)-ethyl]-phenyl]-1-oxo-2,3-dihydro-isoindol-6-yl]-piperidinhydrochlorid
(8) 4-[4-[[trans-4-[2-(Methoxycarbonyl)-ethyl]-cyclohexyl]-aminomethyl]-phenyl]-piperidin-ditrifluoracetat
Schmelzpunkt: 77-79 °C (Zers.)
(9) 4-[4-[N-[trans-4-[2-(Methoxycarbonyl)-ethyl]-cyclohexyl]-N-methyl-aminomethyl]-phenyl]-piperidin-dihydrochlorid
(10) 4-[4-[N-[trans-4-[2-(Methoxycarbonyl)-ethyl]-cyclohexyl]-N-(2-phenyl-ethyl)-aminomethyl]-phenyl]-piperidin-dihydrochlorid
(11) 4-[4-[[trans-4-[2-(Methoxycarbonyl)-ethyl]-cyclohexyl]-aminosulfonyl]-phenyl]-piperidin-hydrochlorid
(12) 4-[4-[2-[4-[2-(Methoxycarbonyl)-ethyl]-phenyl]-ethenyl]-phenyl]-piperidin-hydrochlorid

(13) 4-[2-[trans-4-[2-(Methoxycarbonyl)-ethyl]-cyclohexyl]-1-oxo-2,3-dihydro-isoindol-5-yl]-piperidin-hydrochlorid
(14) 4-[2-[4-[2-(Methoxycarbonyl)-ethyl]-phenyl]-1-oxo-2,3-dihydro-isoindol-5-yl]-piperidin-hydrochlorid
(15) 4-[2-[4-[2-(n-Butansulfonylamino)-2-(methoxycarbonyl)-ethyl]-phenyl]-1-oxo-2,3-dihydro-isoindol-5-yl]-piperidinhydrochlorid
(16) 4-[2-[trans-4-[2-(Methoxycarbonyl)-ethyl]-cyclohexyl]-1-oxo-3,4-dihydro-isochinolin-7-yl]-piperidin-hydrochlorid
(17) 4-[2-[4-[2-(Methoxycarbonyl)-ethyl]-phenyl]-1-oxo-3,4-dihydro-isochinolin-7-yl]-piperidin-hydrochlorid
(18) 4-[4-[2-[4-[2-(Methoxycarbonyl)-ethyl]-phenyl]-ethyl]-phenyl]-piperidin-hydrochlorid
(19) 4-[4-[[4-[2-(Methoxycarbonyl)-ethyl]-phenyl]-methylamino]-phenyl]-piperidin-hydrochlorid
(20) 4-[4-[[4-[2-(Methoxycarbonyl)-ethyl]-phenyl]-aminomethyl]-phenyl]-piperidin-hydrochlorid
(21) 4-[4-[[trans-4-(Isopropyloxycarbonyl)-cyclohexyl]-aminocarbonyl]-phenyl]-piperidin-hydrochlorid
Schmelzpunkt: 270 °C (sintert ab 250 °C)
$R_f$-Wert: 0,38 (Kieselgel; Methylenchlorid/Methanol/konz. Ammoniak = 4:1:0,25)
(22) 4-[4-[[trans-4-[2-(Ethoxycarbonyl)-ethyl]-cyclohexyl]-aminocarbonyl]-phenyl]-piperidin-hydrochlorid
Schmelzpunkt: 246-248 °C
(23) 4-[4-[[trans-4-[Ethoxycarbonyl-methyloxy)-cyclohexyl]-aminocarbonyl]-phenyl]-piperidin-hydrochlorid
$R_f$-Wert: 0,60 (Reversed Phase Platte RP8; Methanol/5%ige Natriumchloridlösung = 6:4)
(24) 4-[4-[[4-(Methoxycarbonyl-methyloxy)-phenyl]-carbonylamino]-phenyl]-piperidin
(25) 4-[2-[trans-4-[2-(Ethoxycarbonyl)-ethyl]-cyclohexyl]-1-oxo-2,3-dihydro-isoindol-5-yl]-piperidin-hydrochlorid Schmelzpunkt: 260-264 °C

Beispiel 10

1-(Methoxycarbonyl)-4-[4-[[trans-4-[2-(methoxycarbonyl)-ethyl]-cyclohexyl]-aminocarbonyl]-phenyl]-piperidin

Herstellung aus 4-[4-[[trans-4-[2-(Methoxycarbonyl)-ethyl]-cyclohexyl]-aminocarbonyl]-phenyl]-piperidin und Chlorameisensäure-methylester in Methylenchlorid in Gegenwart von 0,2N Natronlauge.

Analog wird folgende Verbindung erhalten:

(1) 1-(Acetoxy-methoxycarbonyl)-4-[4-[[trans-4-[2-(methoxycarbonyl)-ethyl]-cyclohexyl]-aminocarbonyl]-phenyl]-piperidin

Beispiel 11

2-[4-[[trans-4-[2-(Methoxycarbonyl)-ethyl]-cyclohexyl]-aminocarbonyl]-phenyl]-ethylamin-hydrochlorid

Eine Lösung von 1,6 g (4,87 mMol) 2-[4-[[trans-4-[2-(Methoxycarbonyl)-ethyl]-cyclohexyl]-aminocarbonyl]-phenyl]-acetonitril in 400 ml Methanol wird nach Ansäuern mit methanolischer Salzsäure in Gegenwart von 0,5 g Palladium auf Kohle (10%ig) bei Raumtemperatur und einem Druck von 3 bar bis zur beendeten Wasserstoff-Aufnahme hydriert. Anschließend saugt man vom Katalysator ab und dampft unter vermindertem Druck zur Trockne ein. Der Rückstand wird mit einer 1:1-Mischung aus tert.Butylmethylether und Essigester verrieben, erhitzt, wieder auf Raumtemperatur abgekühlt und abgesaugt. Nach Auskochen mit Aceton und erneutem Absaugen erhält man 1,2 g = 66,7 % einer gelblichen kristallinen Verbindung.
Schmelzpunkt: >250 °C
$R_f$-Wert: 0,45 (Kieselgel; Dichlormethan/Methanol = 4:1)

Beispiel 12

4-[1-[[trans-4-[2-(Methoxycarbonyl)-ethyl]-cyclohexyl]-aminocarbonyl]-piperidino-piperidin

Zu einer äquimolaren Lösung von trans-4-[2-(Methoxycarbonyl)-ethyl]-cyclohexylamin und Chlorameisensäure-p-nitrophenylester in trockenem Tetrahydrofuran tropft man unter Rühren und bei 0 °C eine Lösung von 2 Äquivalenten Triethylamin in Tetrahydrofuran und rührt weitere 2,5 Stunden bei 0 °C. Anschließend tropft man unter weiterem Rühren ein Äquivalent 4,4'-Bipiperidyl zu, rührt anschließend während 16 Stunden bei Raumtemperatur und während weiterer 4 Stunden bei 50 °C. Hiernach wird unter vermindertem Druck zur Trockne eingedampft, der Rückstand in Essigester aufgenommen und diese

Essigester-Lösung mit 1N-Natriumhydroxidlösung und anschließend mit Wasser gewaschen, über Natriumsulfat getrocknet und unter vermindertem Druck zur Trockne eingedampft. Das verbleibende Rohprodukt wird über Kieselgel chromatographiert, wobei Essigester als Elutionsmittel dient.

Beispiel 13

4-[4-[[4-(2-Carboxy-ethyl)-piperidino]-methyl]-phenyl]-piperidin-dihydrochlorid

1,3 g (0,0029 Mol) 1-tert.Butyloxycarbonyl-4-[4-[[2-(methoxycarbonyl-ethyl)-piperidino]-methyl]-phenyl]-piperidin werden in 80 ml halbkonzentrierter Salzsäure 15 Minuten lang bei Raumtemperatur gerührt. Danach engt man unter Vakuum zur Trockne ein und digeriert den verbleibenden Rückstand dreimal mit Aceton und trocknet den verbleibenden harzigen Rückstand unter Vakuum.
Ausbeute: 0,82 g (77,1 % der Theorie)
$R_f$-Wert: 0,54 (Reversed Phase Platte RP8; Methanol/5%ige Natriumchloridlösung = 6:4)
$^1$H-NMR-Spektrum (200 MHz, DMSO-$d_6$); Signale bei ppm: 1,3-1,7 (m, 5H); 1,7-2,0 (m, 6H); 2,15-2,3 (t, 2H); 2,7-3,1 (m, 4+1H); 3,2-3,55 (m, 4H); 4,15-4,2 (d, 2H); 7,3 (d, 2H); 7,6 (d, 2H); 9,1 (s, 2H); 10,7 (s, 1H); 12,05 (s, 1H)

Analog werden folgende Verbindungen hergestellt:

(1) 4-[4-[[trans-4-(2-Carboxy-ethyl)-cyclohexyl]-aminomethyl]-phenyl]-piperidin-dihydrochlorid
Schmelzpunkt: 308-311 °C (Zers.)
Hergestellt aus 1-tert.Butylosycarbonyl-4-[4-[[trans-4-(2-methoxycarbonyl-ethyl)-cyclohexyl]-aminomethyl]-phenyl]-piperidin und halbkonzentrierter Salzsäure.
(2) 3-[4-[[trans-4-(2-Carboxy-ethyl)-cyclohexyl]-aminocarbonyl]-phenyl]-pyrrolidin-hydrochlorid
(3) 3-[4-[[trans-4-(2-Carboxy-ethyl)-cyclohexyl]-aminocarbonyl]-phenyl]-3-methyl-pyrrolidin-hydrochlorid
(4) 4-[2-[trans-4-(2-Carboxy-ethyl)-cyclohexyl]-1-oxo-2,3-dihydro-isoindol-6-yl]-pyridin
$R_f$-Wert: 0,39 (Reversed Phase Platte RP8; Methanol/5%ige Natriumchloridlösung = 6:4)
(5) 1-[4-[[trans-4-(2-Carboxy-ethyl)-cyclohexyl]-aminomethyl]-phenyl]-piperazin-dihydrochlorid
Schmelzpunkt: 245-248 °C (Zers.)
Hergestellt aus 1-tert.Butyloxycarbonyl-4-[4-[[trans-4-(2-methoxycarbonyl-ethyl)-cyclohexyl]-aminomethyl]-phenyl]-piperazin und halbkonzentrierter Salzsäure.
(6) 3-[4-[[trans-4-(2-Carboxy-ethyl)-cyclohexyl]-aminocarbonyl]-phenyl]-pyrrolidin-hydrochlorid
Schmelzpunkt: > 250 °C
$R_f$-Wert: 0,55 (Reversed Phase Plate RP8; Methanol/5%ige Kochsalzlösung = 6/4)
Hergestellt aus 1-tert.Butyloxycarboxyl-3-[4-[[trans-4-[2-(methoxycarbonyl)-ethyl]-cyclohexyl]-aminocarbonyl]-phenyl]-pyrrolidin und halbkonzentrierter Salzsäure.
(7) 3-[4-[[trans-4-(2-Carboxy-ethyl)-cyclohexyl]-aminocarbonyl]-phenyl]-3-methyl-pyrrolidin-hydrochlorid
Schmelzpunkt: >250 °C
$R_f$-Wert: 0,5 (Reversed Phase Plate RP8; Methanol/5%ige Kochsalzlösung = 6/4)
Hergestellt aus 1-tert.Butyloxycarbonyl-3-[4-[[trans-4-[2-(methoxycarbonyl-ethyl]-cyclohexyl]-aminocarbonyl]-phenyl]-3-methyl-pyrrolidin und halbkonzentrierter Salzsäure

Beispiel 14

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 15

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 16

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 17

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 18

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 19

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4.0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Carbonsäurederivate der allgemeinen Formel

$$A - B - C - D - E - COOR_a \quad ,(I)$$

in der

$R_a$ ein Wasserstoffatom, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkylgruppe mit 5 bis 7 Kohlenstoffatomen oder eine $R_1$-CO-O-($R_2$CH)-Gruppe, in der

$R_1$ eine Alkylgruppe mit 1 bis 5 Kohlenstoffatomen, eine Cycloalkylgruppe mit 5 bis 7 Kohlenstoffatomen, eine Phenylalkylgruppe mit 1 bis 3 Kohlenstoffatomen im Alkylteil, eine Alkoxygruppe mit 1 bis 5 Kohlenstoffatomen, eine Cycloalkoxygruppe mit 5 bis 7 Kohlenstoffatomen oder eine Phenylgruppe und

$R_2$ ein Wasserstoffatom, eine Alkylgruppe mit 1 bis 4 Kohlenstoffatomen, eine Cycloalkylgruppe mit 5 bis 7 Kohlenstoffatomen oder eine Phenylgruppe darstellen,

A eine über ein Kohlenstoffatom des Restes A mit dem Rest B verknüpfte Aminoalkylgruppe mit 1 bis 5 Kohlenstoffatomen oder eine 5-, 6- oder 7-gliedrige Azacycloalkylgruppe, wobei die Verknüpfung der Azacycloalkylgruppe mit dem Rest B über ein Kohlenstoffatom der Azacycloalkylgruppe erfolgt sowie ein Wasserstoffatom der Aminogruppe in den vorstehend erwähnten Aminoalkylgruppen und das Wasserstoffatom am Stickstoffatom in den vorstehend erwähnten Azacycloalkylgruppen durch den Rest $R_b$ ersetzt sein kann und zusätzlich die Kohlenstoffatome der Azacycloalkylgruppe durch ein bis drei Alkylgruppen mit jeweils 1 bis 3 Kohlenstoffatomen, durch eine Aminocarbonyl-, Cyano-, $R_3$O- oder $R_3$O-CO-Gruppe substituiert sein können, wobei

$R_b$ eine Alkylgruppe mit 1 bis 3 Kohlenstoffatomen, eine Phenylalkylgruppe mit 1 bis 3 Kohlenstoffatomen im Alkylteil, eine Alkanoylgruppe mit 1 bis 3 Kohlenstoffatomen im Alkylteil, eine Trifluoracetylgruppe, eine Alkoxycarbonylgruppe mit insgesamt 2 bis 6 Kohlenstoffatomen, eine Phenylalkoxycarbonylgruppe mit 1 bis 3 Kohlenstoffatomen im Alkoxyteil, eine Alkenyloxycarbonylgruppe mit insgesamt 4 bis 6 Kohlenstoffatomen oder eine $R_1$-CO-O-($R_2$CH)-O-CO-Gruppe, in der $R_1$ und $R_2$ wie vorstehend erwähnt definiert sind, und

$R_3$ ein Wasserstoffatom, eine Alkyl- oder Phenylalkylgruppe mit jeweils 1 bis 3 Kohlenstoffatomen im Alkylteil darstellen,

sowie in einer so gebildeten 6- oder 7-gliedrigen Azacycloalkylgruppe eine >CH- Einheit in 4-Stellung, bezogen auf das Ringstickstoffatom, durch ein Stickstoffatom oder in einer so gebildeten 5- bis 7-gliedrigen Azacycloalkylgruppe eine $-CH_2-CH<$ Einheit durch eine $-CH=C<$ Einheit und in einem so gebildeten Piperazinyl- oder Homopiperazinylring eine Methylengruppe, die benachbart zu dem Stickstoffatom in 4-Stellung steht, durch ein Carbonylgruppe ersetzt sein kann, eine Chinuclidinyl- oder Pyridylgruppe,

B eine gegebenenfalls durch ein Fluor-, Chlor- oder Bromatom, durch eine Trifluormethylgruppe, durch eine Alkyl-, Alkoxy-, Alkylsulfenyl-, Alkylsulfinyl- oder Alkylsulfonylgruppe mit jeweils 1 bis 3 Kohlenstoffatomen im Alkyl- und Alkoxyteil mono- oder disubstituierte Phenylengruppe, wobei die Substituenten gleich oder verschieden sein und in der zusätzlich 1 oder 2 unsubstituierte Methingruppen jeweils durch ein N-Atom ersetzt sein können, oder eine Piperidinylengruppe,

C eine Carbonyl-, $-CH_2CH_2-$, $-CH=CH-$, $-CH_2-$, $-CH_2O-$, $-OCH_2-$, $-CONR_4-$, $-CONR_4-CH_2-$, $-NR_4CO-$, $-NR_4CO-NR_4-$, $-CH_2NR_4-$, $-NR_4CH_2-$, $-SO_2NR_4-$, $-SO_2NR_4-CH_2-$ oder $-NR_4SO_2$-Gruppe, in denen

$R_4$ ein Wasserstoffatom, eine Alkyl- oder Phenylalkylgruppe mit jeweils 1 bis 3 Kohlenstoffatomen im Alkylteil oder auch, wenn C eine über die Carbonylgruppe an den Rest B gebundene $-CONR_4$-Gruppe darstellt, $R_4$ eine Methylen- oder 1,2-Ethylengruppe darstellt, die jeweils an das zur Anknüpfungsstelle des $-CONR_4$-Restes orthoständige Kohlenstoffatom des Restes B gebunden sind,

D eine gegebenenfalls durch ein Fluor-, Chlor- oder Bromatom, durch eine Trifluormethylgruppe, durch eine Alkyl-, Alkoxy-, Alkylsulfenyl-, Alkylsulfinyl- oder Alkylsulfonylgruppe mit jeweils 1 bis 3 Kohlenstoffatomen im Alkyl- und Alkoxyteil mono- oder disubstituierte Phenylengruppe, wobei die Substituenten gleich oder verschieden sein und in der zusätzlich 1 oder 2 unsubstituierte Methingruppen jeweils durch ein N-Atom ersetzt sein können, eine Cycloalkylengruppe mit 5 bis 7 Kohlenstoffatomen, in der eine oder zwei >CH- Einheiten, durch ein N-Atom ersetzt sein können, wobei zusätzlich in einem so gebildeten Aza- oder Diazacyclohexylenring eine zu einem Stickstoffatom benachbarte Methylengruppe durch eine Carbonylgruppe ersetzt sein kann, und die Bindung an die Reste C und E auch über ein Stickstoffatom des Aza- oder Diazacyclohexylenringes erfolgen kann, und

E eine Bindung, eine Alkylenoxygruppe mit 1 bis 3 Kohlenstoffatomen im Alkylenteil, wobei das Sauerstoffatom an den Rest D gebunden ist, eine geradkettige Alkylengruppe mit 1 bis 5 Kohlenstoffatomen, die durch 1 oder 2 Alkylgruppen, durch eine Hydroxy-, Alkoxy-, $R_5$NH-, $R_5$N(Alkyl)- oder $R_5$N-(Phenylalkyl)-Gruppe substituiert sein kann, oder eine Alkenylengruppe mit 2 bis 5 Kohlenstoffatomen, die durch ein oder zwei Alkylgruppen substituiert sein kann, wobei der Alkylteil der Phenylalkylgruppe 1 bis 3 Kohlenstoffatome und die übrigen Alkyl-, Alkylen- und Alkoxyteile jeweils 1 bis 8 Kohlenstoffatome enthalten können, wobei

$R_5$ ein Wasserstoffatom, eine Alkylgruppe mit 1 bis 8 Kohlenstoffatomen, eine Alkoxycarbonylgruppe mit insgesamt 2 bis 5 Kohlenstoffatomen, eine Phenylalkoxycarbonylgruppe oder eine durch eine Alkylgruppe mit 1 bis 8 Kohlenstoffatomen, durch eine Cycloalkyl-, Phenylalkyl- oder Phenylgruppe substituierte Carbonyl- oder Sulfonylgruppe darstellt, in denen der Alkyl- und Alkoxyteil in einer vorstehend erwähnten Phenylalkyl und Phenylalkoxygruppe jeweils 1 bis 3 Kohlenstoffatome und der Cycloalkylteil 3 bis 7 Kohlenstoffatome enthalten kann, wobei die bei der Definition des Restes $R_5$ vorstehend erwähnten Phenylkerne jeweils zusätzlich durch ein Fluor-, Chlor- oder Bromatom, durch eine Trifluormethylgruppe, durch eine Alkyl-, Alkoxy-, Alkylsulfenyl, Alkylsulfinyl- oder Alkylsulfonylgruppe mit jeweils 1 bis 3 Kohlenstoffatomen im Alkyl- und Alkoxyteil mono- oder disubstituiert und die Substituenten gleich oder verschieden sein können, bedeuten,

wobei, wenn

(i) die A-B-Gruppe eine in 4-Stellung durch eine $R_b$-NH-$CH_2$-Gruppe substituierte Phenylgruppe bedeutet, in der $R_b$ eine Benzyloxycarbonylgruppe darstellt, die $R_a$OOC-E-D-C-Gruppe keine 3-Carboxy-phenylaminocarbonylgruppe darstellen kann (siehe EP-A-0,372,486 und J. Med. Chem. 35, 4393-4407 (1992)),

oder, wenn

(ii) die A-B-Gruppe eine in 4-Stellung durch eine $R_b$-NH-$CH_2$-Gruppe substituierte Phenylgruppe bedeutet, in der $R_b$ ein Wasserstoffatom oder eine Acetylgruppe darstellt, die $R_a$OOC-E-D-C-Gruppe keine in 4-Stellung durch eine Carboxymethyl-, Methoxycarbonylmethyl-, 2-Carboxy-ethyl-, 2-Methoxycarbonyl-ethyl- oder 2-Ethoxycarbonylethylgruppe substituierte Phenylcarbonylgruppe darstellen kann (siehe EP-A-0,044,541, JP-A-5813553, JP-A-5939866, JP-A-5846051 und Chem. Pharm. Bull. 33, 5059-5061 (1985)),

oder, wenn

(iii) die A-B-Gruppe eine in 4-Stellung durch eine $NH_2$-$CH_2$-$CH_2$-Gruppe substituierte Phenylgruppe bedeutet, die $R_a$OOC-E-D-C-Gruppe keine 4-Ethoxycarbonyl-phenylcarbonylgruppe darstellen kann (siehe DE-A-3,718,638),

oder, wenn

(iv) die A-B-Gruppe eine 4-Aminomethyl-phenyl, 3-Aminomethyl-phenyl-, 4-Aminomethyl-3-methoxyphenyl oder 3-Aminomethyl-4-methoxyphenylgruppebedeutet, die $R_a$OOC-E-D-C-Gruppe keine 4-Ethoxycarbonylmethoxy-2,3-dichlor-phenylcarbonylgruppe darstellt (siehe J. Med. Chem. 27, 1579-1587 (1984) und Diuretics: Chem. Pharmacol., Clin. Appl., Proc. Int. Conf. Diuretics, 1st, 21-29 (1984),

oder, wenn

(v) die A-B-Gruppe eine in 4-Stellung durch eine $NH_2$-$CH_2$- oder $(CH_3)_3$CO-CO-NH-$CH_2$-Gruppe substituierte Phenylgruppe darstellt, die $R_a$OOC-E-D-C-Gruppe keine 4-Carboxyphenylmethoxygruppe darstellen kann (siehe Int. J. Pept. Protein Res. 18, 451-458 (1981)),

oder, wenn

(vi) die A-B-Gruppe eine in 4-Stellung durch eine $NH_2$-$CH_2$-Gruppe substituierte Phenylgruppe darstellt, die $R_a$OOC-E-D-C-Gruppe keine 4-Carboxy-phenylaminosulfonylgruppe darstellen kann (siehe Chem. Pharm. Bull (Tokio) 7, 734-739 (1959) und J. Chem. Phys. 32, 691-697 (1960)),

oder, wenn

(vii) die A-B-Gruppe eine 4-(2-Pyridyl)-phenyl- oder 4-(3-Pyridyl)-phenylgruppe darstellt, die $R_a$OOC-E-D-C-Gruppe keine 4-Carboxy-phenylcarbonylamino-, 4-Benzyloxycarbonyl-phenylcarbo- nylamino- oder 2-(4-Carboxy-phenyl)-ethylgruppe darstellen kann (siehe J. Med. Chem. 11, 295 (1968) und

US-A-2,837,522),
oder, wenn
(viii) die A-B-Gruppe eine 3-(4-Pyridyl)-phenylgruppe darstellt, die $R_a$OOC-E-D-C-Gruppe keine 2-(Carboxymethyl)-phenylcarbonylaminogruppe darstellen kann (siehe Farmaco 44, 721-729 (1989)),
deren Tautomere, deren Stereoisomere einschließlich deren Gemische und deren Salze.

**2.** Carbonsäurederivate der allgemeinen Formel I gemäß Anspruch 1, in der
$R_a$ ein Wasserstoffatom, eine Alkylgruppe mit 1 bis 5 Kohlenstoffatomen, eine Phenylalkylgruppe mit 1 bis 3 Kohlenstoffatomen im Alkylteil oder eine Cycloalkylgruppe mit 5 bis 7 Kohlenstoffatomen,
A eine über ein Kohlenstoffatom des Restes A mit dem Rest B verknüpfte Aminoalkylgruppe mit 2 bis 5 Kohlenstoffatomen oder eine Piperidinylgruppe, wobei die Verknüpfung der Piperidinylgruppe mit dem Rest B über ein Kohlenstoffatom der Piperidinylgruppe erfolgt sowie ein Wasserstoffatom der Aminogruppe in den vorstehend erwähnten Aminoalkylgruppen und das Wasserstoffatom am Stickstoffatom in den vorstehend erwähnten Piperidinylgruppen durch den Rest $R_b$ ersetzt sein kann und zusätzlich die Kohlenstoffatome der Piperidinylgruppe durch eine Methyl-, Cyano-, Carboxy-, Methoxycarbonyl- oder Aminocarbonylgruppe substituiert sein können, wobei
$R_b$ eine Alkylgruppe mit 1 bis 3 Kohlenstoffatomen, eine Benzylgruppe, eine Alkoxycarbonylgruppe mit insgesamt 2 bis 6 Kohlenstoffatomen, eine Benzyloxycarbonylgruppe oder eine $CH_3$-CO-O-($CH_2$)-O-CO-Gruppe darstellt,
sowie in einer so gebildeten Piperidinylgruppe eine >CH- Einheit in 4-Stellung durch ein Stickstoffatom oder in einer so gebildeten Piperidinylgruppe eine -$CH_2$-CH< Einheit durch eine -CH=C< Einheit ersetzt sein kann, eine Chinuclidinyl- oder Pyridylgruppe,
B eine gegebenenfalls durch ein Fluor-, Chlor- oder Bromatom, durch eine Alkyl-, Alkoxy-, Alkylsulfenyl-, Alkylsulfinyl- oder Alkylsulfonylgruppe mit jeweils 1 oder 2 Kohlenstoffatomen im Alkyl- und Alkoxyteil substituierte Phenylengruppe, eine Pyridinylen- oder Piperidinylengruppe,
C eine -CO-, -$CH_2CH_2$-, -CH=CH-, -$CH_2$-, -$CH_2$O-, -O$CH_2$-, -CON$R_4$-, -N$R_4$CO-, -N$R_4$CO-N$R_4$-, -$CH_2$N$R_4$-, -N$R_4$$CH_2$-, -$SO_2$N$R_4$- oder -$NH_4SO_2$-Gruppe, in denen
$R_4$ ein Wasserstoffatom, eine Alkyl- oder Phenylalkylgruppe mit jeweils 1 oder 2 Kohlenstoffatomen im Alkylteil oder auch, wenn C eine über die Carbonylgruppe an den Rest B gebundene -CON$R_4$-Gruppe darstellt, $R_4$ eine Methylen- oder 1,2-Ethylengruppe darstellt, die jeweils an das zur Anknüpfungsstelle des -CON$R_4$-Restes orthoständige Kohlenstoffatom des Restes B gebunden sind,
D eine gegebenenfalls durch ein Chlor- oder Bromatom, durch eine Alkyl- oder Alkoxygruppe mit 1 oder 2 Kohlenstoffatomen substituierte Phenylengruppe oder eine Cyclohexylengruppe, wobei in der Cyclohexylengruppe eine oder zwei >CH- Einheiten, durch ein N-Atom ersetzt sein können, wobei zusätzlich in einem so gebildeten Piperidinylen- oder Piperazinylenring eine zu einem Stickstoffatom benachbarte Methylengruppe durch eine Carbonylgruppe ersetzt sein kann, und die Bindung an die Reste C und E auch über ein Stickstoffatom des Piperidinylen- oder Piperazinylenringes erfolgen kann, und
E eine Bindung, eine Methylenoxygruppe, wobei das Sauerstoffatom an den Rest D gebunden ist, eine 1,2-Ethenylengruppe oder eine geradkettige Alkylengruppe mit 1 bis 5 Kohlenstoffatomen, die durch eine Alkylgruppe mit 1 bis 7 Kohlenstoffatomen oder durch eine $R_5$NH-Gruppe substituiert sein kann, wobei
$R_5$ ein Wasserstoffatom oder eine durch eine Alkylgruppe mit 1 bis 7 Kohlenstoffatomen oder durch eine Benzylgruppe substituierte Carbonylgruppe oder eine durch eine Alkylgruppe mit 1 bis 5 Kohlenstoffatomen oder eine Benzylgruppe substituierte Sulfonylgruppe darstellt, bedeuten,
wobei, wenn
(iii) die A-B-Gruppe eine in 4-Stellung durch eine $NH_2$-$CH_2$-$CH_2$-Gruppe substituierte Phenylgruppe bedeutet, die $R_a$OOC-E-D-C-Gruppe keine 4-Ethoxycarbonyl-phenylcarbonylgruppe darstellen kann,
oder, wenn
(vii) die A-B-Gruppe eine 4-(2-Pyridyl)-phenyl- oder 4-(3-Pyridyl)-phenylgruppe darstellt, die $R_a$OOC-E-D-C-Gruppe keine 4-Carboxy-phenylcarbonylamino, 4-Benzyloxycarbonyl-phenylcarbonylamino- oder 2-(4-Carboxy-phenyl)-ethylgruppe darstellen kann,
oder, wenn
(viii) die A-B-Gruppe eine 3-(4-Pyridyl)-phenylgruppe darstellt, die $R_a$OOC-E-D-C-Gruppe keine 2-(Carboxymethyl)-phenylcarbonylaminogruppe darstellen kann,
deren Tautomere, deren Stereoisomere einschließlich deren Gemische und deren Salze.

**3.** Carbonsäurederivate der allgemeinen Formel I gemäß Anspruch 1, in der
$R_a$ ein Wasserstoffatom, eine Alkylgruppe mit 1 bis 4 Kohlenstoffatomen, eine 2-Phenylethyl- oder Cyclohexylgruppe,
A eine über ein Kohlenstoffatom des Restes A mit dem Rest B verknüpfte Aminoalkylgruppe mit 3 bis 5 Kohlenstoffatomen oder eine Piperidinylgruppe, wobei die Verknüpfung der Piperidinylgruppe mit dem Rest B über ein Kohlenstoffatom der Piperidinylgruppe erfolgt sowie ein Wasserstoffatom der Aminogruppe in den vorstehend erwähnten Aminoalkylgruppen und das Wasserstoffatom am Stickstoffatom in den vorstehend erwähnten Piperidinylgruppen durch den Rest $R_b$ ersetzt sein kann und zusätzlich die Kohlenstoffatome der Piperidinylgruppe durch eine Methyl-, Cyano-, Carboxy-, Methoxycarbonyl- oder Aminocarbonylgruppe substituiert sein können, wobei
$R_b$ eine Alkylgruppe mit 1 bis 3 Kohlenstoffatomen, eine Benzylgruppe, eine Alkoxycarbonylgruppe mit insgesamt 2 bis 5 Kohlenstoffatomen oder eine $CH_3$-CO-O-($CH_2$)-O-CO-Gruppe darstellt,
sowie in einer so gebildeten Piperidinylgruppe eine >CH- Einheit in 4-Stellung durch ein Stickstoffatom oder in einer so gebildeten Piperidinylgruppe eine -$CH_2$-CH< Einheit durch eine -CH=C< Einheit ersetzt sein kann, eine Chinuclidinyl- oder 4-Pyridylgruppe,
B eine gegebenenfalls durch eine Methyl- oder Methoxygruppe substituierte Phenylengruppe, eine 2,5-Pyridinylen- oder 1,4-Piperidinylengruppe,
C eine -CO-, -$CH_2CH_2$-, -CH=CH-, -$CH_2$-, -$CH_2$O-, -O$CH_2$-, -CON$R_4$-, -N$R_4$CO-, -N$R_4$CO-N$R_4$- oder -$CH_2$N$R_4$-Gruppe oder eine -$SO_2$N$R_4$-Gruppe, wobei die $SO_2$-Gruppe mit dem Rest B verknüpft ist und in denen
$R_4$ ein Wasserstoffatom, eine Methyl- oder 2-Phenylethylgruppe oder auch, wenn C eine über die Carbonylgruppe an den Rest B gebundene -CON$R_4$-Gruppe darstellt, $R_4$ eine Methylen- oder 1,2-Ethylengruppe darstellt, die jeweils an das zur Anknüpfungsstelle des -CON$R_4$-Restes ortho-ständige Kohlenstoffatom des Restes B gebunden sind,
D eine Phenylen- oder Cyclohexylengruppe, wobei in der Cyclohexylengruppe eine oder zwei >CH-Einheiten, durch ein N-Atom ersetzt sein können, wobei die Bindung an die Reste C und E auch über ein Stickstoffatom des Piperidinylen- oder Piperazinylenringes erfolgen kann, und
E eine Bindung, eine Methylenoxygruppe, wobei das Sauerstoff an den Rest D gebunden ist, eine geradkettige Alkylengruppe mit 1 bis 5 Kohlenstoffatomen, die durch $R_5$NH-Gruppe substituiert sein kann, wobei
$R_5$ ein Wasserstoffatom, eine durch eine Alkylgruppe mit 1 bis 5 Kohlenstoffatomen substituierte Carbonylgruppe oder eine durch eine Alkylgruppe mit 1 bis 4 Kohlenstoffatomen oder eine Benzylgruppe substituierte Sulfonylgruppe darstellt, bedeuten,
deren Tautomere, deren Stereoisomere einschließlich deren Gemische und deren Salze.

**4.** Carbonsäurederivate der allgemeinen Formel I gemäß Anspruch 1, in der
$R_a$ ein Wasserstoffatom, eine Alkylgruppe mit 1 bis 4 Kohlenstoffatomen oder eine Cyclohexylgruppe,
A eine über die 4-Stellung mit dem Rest B verknüpfte Piperidinyl- oder 3,4-Dehydro-piperidinylgruppe, wobei jeweils das Wasserstoffatom am Stickstoffatom durch den Rest $R_b$ ersetzt sein kann und
$R_b$ eine Alkoxycarbonylgruppe mit insgesamt 2 bis 5 Kohlenstoffatomen darstellt,
eine gegebenenfalls in 1-Stellung durch den Rest $R_b$ substituierte 4-Piperazinylgruppe, wobei $R_b$ wie vorstehend erwähnt definiert ist, oder eine Chinuclidinylgruppe,
B eine Phenylengruppe,
C eine -CON$R_4$-Gruppe, wobei
$R_4$ ein Wasserstoffatom oder eine Methylgruppe oder auch, wenn C eine über die Carbonylgruppe an den Rest B gebundene -CON$R_4$-Gruppe darstellt, $R_4$ eine Methylen- oder 1,2-Ethylengruppe darstellt, die jeweils an das zur Anknüpfungsstelle des -CON$R_4$-Restes ortho-ständige Kohlenstoffatom des Restes B gebunden sind,
D eine 1,4-Phenylen- oder 1,4-Cyclohexylengruppe, und
E eine Bindung, eine 1,2-Ethylengruppe, die durch eine $R_5$NH-Gruppe substituiert sein kann, wobei
$R_5$ eine durch eine Alkylgruppe mit 1 bis 5 Kohlenstoffatomen substituierte Carbonylgruppe oder eine durch eine Alkylgruppe mit 1 bis 4 Kohlenstoffatomen substituierte Sulfonylgruppe darstellt, bedeuten,
deren Tautomere, deren Stereoisomere einschließlich deren Gemische und deren Salze.

**5.** Folgende Verbindungen der allgemeinen Formel I gemäß Anspruch 1:
4-[4-[[trans-4-[2-(Methoxycarbonyl)-ethyl]-cyclohexyl]-aminocarbonyl]-phenyl]piperidin,
4-[4-[[4-[2-(n-Butansulfonylamino)-2-(methoxycarbonyl)-ethyl]-phenyl]-aminocarbonyl]-phenyl]-piperidin,

4-[4-[[trans-4-(2-Carboxy-ethyl)-cyclohexyl]-aminocarbonyl]-phenyl]-piperidin,
4-[4-[[4-[2-(n-Butansulfonyl-amino)-2-carboxy-ethyl]-phenyl]-aminocarbonyl]-phenyl]-piperidin,
4-[3-[[4-[2-(n-Butansulfonylamino)-2-carboxy-ethyl]-phenyl]-aminocarbonyl]-phenyl]-piperidin und
4-[3-[[4-[2-(n-Butansulfonylamino)-2-(methoxycarbonyl)-ethyl]-phenyl]-aminocarbonyl]-phenyl]-piperidin,
deren Tautomere, deren Stereoisomere einschließlich deren Gemische und deren Salze.

**6.** Physiologisch verträgliche Additionssalze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Additionssalz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Carbonsäurederivate gemäß den Ansprüchen 1 bis 6, dadurch gekennzeichnet, daß

a) zur Herstellung von Verbindungen der allgemeinen Formel I, in der $R_a$ ein Wasserstoffatom darstellt, eine Verbindung der allgemeinen Formel

$$A - B - C - D - E - COOR_a' \quad ,(II)$$

in der
A bis E wie in den Ansprüchen 1 bis 5 definiert sind und $R_a'$ mit Ausnahme des Wasserstoffatoms die in den Ansprüchen 1 bis 5 für $R_a$ erwähnten Bedeutungen besitzt, mittels Hydrolyse, Pyrolyse oder Hydrogenolyse in eine Verbindung der allgemeinen Formel I, in der $R_a$ ein Wasserstoffatom darstellt, übergeführt wird oder

b) zur Herstellung von Verbindungen der allgemeinen Formel I, in der A durch den Rest $R_b$ substituiert ist, eine Verbindung der allgemeinen Formel

$$A_1 - B - C - D - E - COOR_a \quad ,(III)$$

in der
B bis E und $R_a$ wie in den Ansprüchen 1 bis 5 definiert sind und
$A_1$ eine über ein Kohlenstoffatom mit dem Rest B verknüpfte Aminoalkylgruppe mit 1 bis 5 Kohlenstoffatomen oder eine über ein Kohlenstoffatom mit dem Rest B verknüpfte 5-, 6-, oder 7-gliedrige Azacycloalkylgruppe darstellt, wobei die Kohlenstoffatome der Azacycloalkylgruppe durch ein bis drei Alkylgruppen mit jeweils 1 bis 3 Kohlenstoffatomen, durch eine Aminocarbonyl, Cyano-, $R_3O$- oder $R_3O$-CO-Gruppe substituiert sein können, wobei $R_3$ wie in den Ansprüchen 1 bis 5 erwähnt definiert ist, mit einer Verbindung der allgemeinen Formel

$$R_b - Z_1 \quad ,(IV)$$

in der
$R_b$ wie in den Ansprüchen 1 bis 5 definiert ist und $Z_1$ eine nukleophile Austrittsgruppe
oder auch, wenn $Z_1$ an eine Carbonylgruppe gebunden ist, eine Hydroxy-, Alkanoyloxy- oder Alkoxycarbonyloxygruppe darstellt,
oder auch in Gegenwart eines Reduktionsmittels, wenn $Z_1$ zusammen mit einem benachbarten Wasserstoffatom des Restes $R_b$ ein Sauerstoffatom bedeuten, umgesetzt wird oder

c) zur Herstellung von Verbindungen der allgemeinen Formel I, in der $R_a$ mit Ausnahme des Wasserstoffatoms wie in den Ansprüchen 1 bis 5 definiert ist, eine Verbindung der allgemeinen Formel

A - B - C - D - E - COOH ,(V)

in der
A bis E wie in den Ansprüchen 1 bis 5 definiert sind, mit einer Verbindung der allgemeinen Formel

$Z_2$ - $R_a$' ,(VI)

in der
$R_a$' mit Ausnahme des Wasserstoffatoms die für $R_a$ in den Ansprüchen 1 bis 5 erwähnten Bedeutungen besitzt und $Z_2$ eine nukleophile Austrittsgruppe darstellt, umgesetzt wird oder
d) zur Herstellung von Verbindungen der allgemeien Formel I, in der C eine $-CH_2$-NH-Gruppe darstellt, eine Verbindung der allgemeinen Formel

A - B - CH=N - D - E - $COOR_a$ ,(VII)

in der
A, B, D, E und $R_a$ wie in den Ansprüchen 1 bis 5 definiert sind, reduziert wird oder
e) zur Herstellung von Verbindungen der allgemeinen Formel I, in der C eine $-CO-NR_4$-Gruppe darstellt, eine Verbindung der allgemeinen Formel

$A_2$ - B - COOH ,(VIII)

in der
B wie in den Ansprüchen 1 bis 5 definiert ist und
$A_2$ eine am Stickstoffatom durch einen Alkyl-, Aralkyl-, Alkanoyl-, Trifluoracetyl- oder Alkoxycarbonylrest substituierte Gruppe A darstellt, mit einem Amin der allgemeinen Formel

$R_4$ - NH - D - E - $COOR_{a'}$ ,(IX)

in der
D, E und $R_4$ wie in den Ansprüchen 1 bis 5 definiert sind und
$R_a$' mit Ausnahme des Wasserstoffatoms die für $R_a$ in den Ansprüchen 1 bis 5 erwähnten Bedeutungen besitzt, umgesetzt wird oder
f) zur Herstellung von Verbindungen der allgemeinen Formel I, in der C eine $-NR_4$-CO-Gruppe darstellt, eine Verbindung der allgemeinen Formel

$A_2$ - B - NH-$R_4$ ,(X)

in der
$R_4$ und B wie in den Ansprüchen 1 bis 5 definiert sind und $A_2$ eine am Stickstoffatom durch einen Alkyl-, Aralkyl-, Alkanoyl-, Trifluoracetyl- oder Alkoxycarbonylrest substituierte Gruppe A darstellt, mit einer Carbonsäure der allgemeinen Formel

HOOC - D - E - $COOR_a$' ,(XI)

in der
D und E wie in den Ansprüchen 1 bis 5 definiert sind und $R_a$' mit Ausnahme des Wasserstoffatoms die für $R_a$ in den Ansprüchen 1 bis 5 erwähnten Bedeutungen besitzt, umgesetzt wird oder
g) zur Herstellung von Verbindungen der allgemeinen Formel I, in der A eine über ein Kohlenstoffatom mit dem Rest B verknüpfte Aminoalkylgruppe mit 1 bis 5 Kohlenstoffatomen darstellt, eine Verbindung der allgemeinen Formel

$A_3$ - B - C - D - E - $COOR_a$ ,(XII)

in der
B bis E und $R_a$ wie in den Ansprüchen 1 bis 5 definiert sind und
$A_3$ eine Cyanogruppe oder eine Cyanoalkylgruppe mit 1 bis 4 Kohlenstoffatomen im Alkylteil

darstellt, reduziert wird und
erforderlichenfalls ein während den Umsetzungen zum Schutze von reaktiven Gruppen verwendeter Schutzrest abgespalten wird und/oder
gewünschtenfalls eine so erhaltene Verbindung der allgemeinen Formel I, in der $R_4$ ein Wasserstoffatom darstellt, mittels Alkylierung in eine entsprechende Verbindung der allgemeinen Formel I, in der $R_4$ eine gegebenenfalls durch eine Phenylgruppe substituierte Alkylgruppe mit 1 oder 2 Kohlenstoffatomen darstellt, übergeführt wird und/oder
eine so erhaltene Verbindung der allgemeinen Formel I in ihre Enantiomeren und/oder Diastereomeren aufgetrennt wird und/oder
eine so erhaltene Verbindung der Formel I in ihre Salze, insbesondere für die pharmazeutische Anwendung in ihre physiologisch verträglichen Salze mit anorganischen oder organischen Säuren, übergeführt wird und/oder
eine so erhaltene Verbindung der allgemeinen Formel I, falls diese eine Carboxylgruppe enthält, gewünschtenfalls anschließend in ihre Additionssalze mit anorganischen oder organischen Basen, insbesondere für die pharmazeutische Anwendung in ihre physiologisch verträglichen Additionssalze, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 93 11 9786

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.5) |
|---|---|---|---|
| D,A | EP-A-0 372 486 (HOFFMANN-LA ROCHE) 13. Juni 1990<br>* das ganze Dokument *<br>--- | 1-10 | C07D211/34<br>C07D295/14<br>C07D211/70<br>C07D207/08<br>C07D401/04<br>A61K31/445<br>A61K31/40 |
| D,X | JOURNAL OF MEDICINAL CHEMISTRY.<br>Bd. 35 , 1992 , WASHINGTON US<br>Seiten 4393 - 4407<br>T. WELLER ET. AL. 'Low Molecular Weight, Non-Peptide Fibrinogen Receptor Antagonist.'<br>* Beispiel 22b *<br>--- | 1-3,6-9 | |
| A | EP-A-0 496 378 (THOMAE) 29. Juli 1992<br>* das ganze Dokument *<br>--- | 1-10 | |
| A | EP-A-0 478 362 (MERCK) 1. April 1992<br>* das ganze Dokument *<br>--- | 1-10 | |
| A | EP-A-0 483 667 (THOMAE) 6. Mai 1992<br>* das ganze Dokument *<br>--- | 1-10 | RECHERCHIERTE SACHGEBIETE (Int.Cl.5) |
| P,X | EP-A-0 528 369 (THOMAE) 24. Februar 1993<br>* Anspruch 1 *<br>---<br>-/-- | 1-3,6-9 | C07D |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 24. März 1994 | Kissler, B |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 93 11 9786

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich der maßgeblichen Teile | Betrifft Anspruch | |
| P,A | EP-A-0 567 966 (THOMAE) 3. November 1993<br>* das ganze Dokument *<br>--- | 1-10 | |
| P,A | EP-A-0 567 968 (THOMAE) 3. November 1993<br>* das ganze Dokument *<br>--- | 1-10 | |
| D,X | EP-A-0 044 541 (TEIJIN) 27. Januar 1982<br>* siehe Formel I-a-2 auf Seite 9 und dazugehörige Beispiele auf den Seiten 11-15 *<br>* Anspruch 1 *<br>--- | 1-3,6-9 | |
| D,X | DE-A-37 18 638 (THOMAE) 22. Dezember 1988<br>* Zwischenprodukte der Formel III, Seite 7 *<br>--- | 1-3,6-9 | RECHERCHIERTE SACHGEBIETE (Int.Cl.5) |
| D,X | JOURNAL OF MEDICINAL CHEMISTRY.<br>Bd. 11 , März 1968 , WASHINGTON US<br>Seiten 295 - 300<br>G. J. ATWELL, B. F. CAIN 'Potential Antitumor Agents.'<br>* Tabelle II *<br>--- | 1-3,6-9 | |
| D,X | JOURNAL OF MEDICINAL CHEMISTRY.<br>Bd. 27 , 1984 , WASHINGTON US<br>Seiten 1579 - 1587<br>C. LEE ET. AL.<br>'[(Aminomethyl)aryloxy]acetic Acid Esters. A New Class of High-Ceiling Diuretics.'<br>* Tabellen I,II *<br>---<br>-/-- | 1-3,6-9 | |

EPO FORM 1503 03.82 (P04C12)

Europäisches Patentamt

# EUROPÄISCHER TEILRECHERCHENBERICHT

Nummer der Anmeldung
EP 93 11 9786

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich der maßgeblichen Teile | Betrifft Anspruch | |
| X | JOURNAL OF MEDICINAL CHEMISTRY.<br>Bd. 27, Nr. 12 , 1984 , WASHINGTON US<br>Seiten 1587 - 1596<br>J. J. PLATTNER ET. AL.<br>'[(Aminomethyl)aryloxy]acetic Acid Esters. A New Clas of High-Ceiling Diuretics.'<br>* Tabelle I *<br>* Beispiele 44,53,57 *<br>--- | 1-3,6-9 | |
| D,X | FARMACO<br>Bd. 44 , 1989<br>Seiten 721 - 729<br>T. MODENA ET. AL.<br>'2-[(arylamino)carbonyl]benzeneacetic acids affecting root gravitropism.'<br>* Beispiel VII; Tabelle 1 *<br>----- | 1-3 | RECHERCHIERTE SACHGEBIETE (Int.Cl.5) |

EPO FORM 1503 03.82 (P04C12)

EP 93 11 9786

- C -

UNVOLLSTÄNDIGE RECHERCHE

Vollständig recherchierte Patentansprüche : 4-5
Unvollständig recherchierte Patentansprüche : 1-3,6-10

Die folgende(n) Definition(en)

"Azacycloalkylgruppe" (Anspruch 1 und abhängige Ansprüche): Diese Definition umfasst im allgemeinen Reste wie Pyrrolidin- oder Piperidin etc. und gilt nicht für Reste wie Pyridin oder Chinolin.
"Phenylengruppe,.... in der zusätzlich 1 oder 2 unsubstituierte Methingruppen jeweils durch ein N-Atom ersetzt sein können" (Anspruch 1 und abhängige Ansprüche)
"eine Cycloalkylengruppe mit 5 bis 7 Kohlenstoffatomen, in der eine oder zwei CH-Einheiten, durch ein N-Atom ersetzt sein können..." (Anspruch 1 und abhängige Ansprüche).

sind ungenau und daher nicht zuläsig.

Die Definition des/der folgenden Substituenten ist zu allgemein und/oder umfasst einen zu grossen Bereich von chemisch grundverschiedenen Resten und ist nur teilweise durch Beispiele in der Beschreibung gestützt:

A,B,C,D,E zusammen mit den nicht klar definierten Verknüpfungspunkten.

Die grosse Zahl der sich aus der Kombination aller beanspruchten Substituenten der obigen Liste ergebenden, theoretisch denkbaren Verbindungen schliesst eine umfassende Recherche aus. In Anlehnung an den Geist und das erfinderische Konzept der vorliegenden Anmeldung ist die Recherche auf den/die folgenden Fall/fälle beschränkt worden:

Definition der Variablen A,B,C,D,E wie in Anspruch 4

(Vgl. Art. 83, 84 EPC, Regel 45 EPC und Richtlinien zur Durchführung Teil B, Kapitel III, 3.6, 3.7)